# EUROPEAN PATENT APPLICATION

(11) **EP 2 537 880 A2**
(43) Date of publication of application: **26.12.2012**
(21) Application number: 11719842.4
(22) Date of filing: 17.02.2011
(51) Int. Cl.: C08G 83/00

(54) **CARBOSILANE DENDRIMERS AND THE USE THEREOF AS ANTIVIRAL AGENTS**

(30) Priority: 25.03.2010 ES 201030450; 18.02.2010 ES 201030233
(71) Applicant: Universidad De Alcalá De Henares (UAH), 28801 Alcalá de Henares (ES); Hospital General Universitario Gregorio Marañon (HUGUGM), 28007 Madrid (ES)
(72) Inventor: DE LA MATA DE LA MATA, Fco. Javier, E-28801 Alcalá de Henares (ES); GÓMEZ RAMÍREZ, Rafael, E-28801 Alcalá de Henares (ES); MUÑOZ FERNÁNDEZ, Mª Ángeles, E-28007 Madrid (ES); SÁNCHEZ-NIEVES FERNÁNDEZ, Javier, E-28801 Alcalá de Henares (ES); ORTEGA LÓPEZ, Paula, E-28801 Alcalá de Henares (ES); CHONCO JÍMENEZ, Louis, E-28007 Madrid (ES); RASINES MORENO, Beatriz, E-28801 Alcalá de Henares (ES); ARNAIZ GARRIDO, Eduardo, E-28801 Alcalá de Henares (ES); SERRAMÍA LOBERA, Mª Jesús, E-28007 Madrid (ES)
(74) Representative: HOFFMANN EITLE
(86) International application number: PCT/ES2011/070104
(87) International publication number: WO 2011/101520

(57) **Abstract**

Highly branched macromolecules synthesized from a polyfunctional core, preferably silicon, or polyphenolic, with a carbosilane structure at its periphery functionalized with groups, preferably anionic, giving to the macromolecule a net negative charge. Furthermore, the invention relates to the procedures for their synthesis and their uses as antiviral agents, antibacterial and antifungal agents.

## Description

The present invention relates to highly branched macromolecules synthesized from a polyfunctional core, called dendrimers, of carbosilane structure and its periphery functionalized with anionic groups that give the macromolecule a net negative charge. Furthermore, the invention relates to a process for their synthesis and their biomedical applications.

### STATE OF THE ART

There are several types of dendrimers or dendronized polymers with net negative charge on the surface used as antiviral agents, because many types of viruses such as HIV or HSV, bind to polyanionic compounds. Thus, sulfated polysaccharides are typical effective inhibitors of viral inhibition. Within this type of polyanionic dendrimers several examples have been described, for instance polylysine dendrimers with carboxylate and sulfonate end groups that are able to effectively block HSV infection (see Gong, Y. et al. Antiviral Res, 2002, 55 (2), 319-329). The same authors conducted studies in mice showed that these dendrimers protected the animals against vaginal infection and the sulfonate derivatives were able to interfere in the final stages of virus replication in infected cells.

Similar dendrimers with a polylysine backbone and terminal carboxylate or sulfonate groups have also shown to be effective in inhibiting HIV *in vitro* (cf. McCarthy, TD et al. Mol Pharm. 2005 Jul-Aug; 2 (4): 312-8). Polyanionic dendrimers inhibit the entry of virus into the cell and the activity of two viral enzymes, reverse transcriptase and integrase, indicating that these polyanionic derivatives may act on different replicative cycles of HIV depending on how they are integrated and placed in the cell. More recent studies show that dendrimers with sulfonate groups protect female macaques against vaginal infection with simian immunodeficiency virus (SIV).

In this section, we highlight the negatively charged dendrimer formulated as a gel and called SPL7013. This dendrimer has been developed by an Australian pharmaceutical company and studies *in vitro* and *in vivo* have shown that it is the best candidate to enter the market, becoming the first commercial dendrimer with a defined biomedical application. The SPL7013 is a lysine-based dendrimer with naphthalene disulfonic acid groups on its surface and is the active ingredient of a vaginal microbicide gel called VivaGel^{®}. It is a potent inhibitor *in vitro* against many strains of HIV-1. It is used in the prevention of vaginal transmission of HIV and genital herpes. Thus, a study by Dezutti and colleagues has shown that a galenic formulation of this dendrimer (5%) have very low toxicity in epithelial cells and is effective in preventing infection by HIV-1 in peripheral blood mononuclear cells (PBMC), macrophages and in the transfer of HIV-1 from epithelial cells to PBMCs. Subsequent studies have shown that daily application of SPL7013 gel in proportions of 1-3% were well tolerated. Similar results were obtained for the prevention of infection by HIV-1 in rectal application of this gel. Also, studies using the gel SPL7013 to the vaginal level in macaques have been done and have shown that it produces no sign of irritation of the vaginal mucosa and is capable of protecting totally (100%) the SIV infection of macaques when applied at 5%. Other similar dendrimers to SPL7013 have also been prepared using skeletons of type PAMAM or PPI, which had similar biological activities but were discarded for commercial use due to difficulties in the synthetic process.

Other anionic dendrimers described as antiviral agents are: the SRI2923 that is a dendrimer-like polyamidoamine (PAMAM) grown from a core of ammonia and surface functionalized with 24 naphthalene disulfonic acid groups. The BRI6195, which is also a PAMAM dendrimer type, grown in this case from an ethylenediamine core and is functionalized with 32 phenyldicarboxilate groups. These dendrimers are able to inhibit the replication of different strains of HIV in the cell line MT-4 (cf. Jiang YH, et al., AIDS Res Hum. Retriviruses, 2005, 21, 207).

The synthesis of dendrimers based on a skeleton containing phosphorus atoms has been also published and these dendrimers are functionalized on its surface with anionic phosphonate groups (cf. L. Griffe, et al., Angew. Chem Int Ed, 2007, 46, 2523). These dendrimers show high activity in the activation and proliferation of natural killer cells (NK). These data are promising as they open the door to the use of antiviral or anticancer immunotherapies, where large numbers of NK cells might be needed.

### DESCRIPTION OF THE INVENTION

The present invention provides highly branched macromolecules synthesized from a polyfunctional core, called dendrimers, of carbosilane structure and functionalized at their periphery with carboxylic, sulfonic or phosphonic groups, preferably anionic ones which give a net negative charge to the macromolecule. Furthermore, the invention provides a process for their preparation and their use in biomedicine. A first aspect of the present invention relates to a carbosilane dendrimer comprising: a multifunctional nucleus (Nu) and an outer layer, which consists, wholly or partly in the same or different units of the group of formula (I): 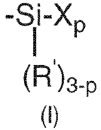
where: R'is an alkyl group (C1-G4),
p is an integer number and varies from 1 to 3, preferably p is 1,
and X is the following group of formula (II):

   -E-N[-(CH₂)_{z}-R¹]₂ (II)

   where E is a linking group located between the silicon of the general formula (I) and the nitrogen of the amine group,
   z is an integer and varies between 1 and 4, R¹ is selected from a group of the list comprising -COOR²,-SO₃R² or -PO₃(R²)₂ and R² is hydrogen or an alkyl group (C1-C4).

For "carbosilane dendrimer" refers herein to a highly branched macromolecule with spherical shape, where the core of dendrimer growth is multifunctional, the units, branches or ramifications of growth have a carbosilane skeleton and the outer layer, surface or periphery of dendrimer incorporates functional groups. This surface or periphery would be the corresponding to the extremities of the ramifications.

The term "polyfunctional core" means herein a versatile element or compound covalently bound to the at least two branches, i.e. at least should be divalent. In a preferred embodiment the core is tetravalent and more preferably the core is silicon (i.e., a silyl group). In another preferred embodiment the core may be a polyphenol, and the term "polyphenol" meaning a molecule of benzene substituted by at least two hydroxyl groups in any of their positions, for example 1,4-dihydroxybenzene, 1,2-dihydroxybenzene or 1,3-dihydroxybenzene, most preferably hydroquinone (1,4-dihydroxybenzene), but may have three, four, five or six hydroxyl groups. More preferably, the polyphenol is trisubstituted, and more preferably 1,3,5-trihydroxybenzene.

For linking group means herein any group that joins the branches, which in turn are attached to the nucleus, with functional groups of the outer layer of the dendrimer. E-bonding group may be selected preferably from alkyl (C1-C10) or a group - (CH₂)ₓ-R³, where R³ can be a triazole or phenoxide group and x is an integer ranging between 1 and 6.

The term "alkyl" refers herein to aliphatic chains, linear or branched. In the case of R' and R², these branches are of 1 to 4 carbon atoms, for example, methyl, ethyl, n-propyl, i-propyl, n-butyl, tert-butyl or sec-butyl, preferably the group alkyl is a methyl. In the case of a bonding group (E), these chains are of 1 to 10 carbon atoms, more preferably a chain alkyl group having 1 to 4 carbon atoms and more preferably the linking group (E) is a propyl group.

In another preferred embodiment, the binding group (E) is the group - (CH₂) x-R³ and R³ is triazole, i.e., of formula: 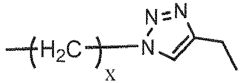 where x is described above and more preferably x is 4.

In another preferred embodiment, the binding group (E) is the group - (CH₂)x-R³ and R³ is phenoxide, i.e. the formula: 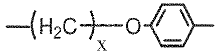 where x is described above and more preferably x is 1.
When R¹ is COOR² group or the group-SO₃R², preferably R² is hydrogen or methyl and more preferably z is 2. When the group R¹ is -PO₃(R²)₂, preferably R² is hydrogen or methyl and more preferably z is 1.

The dendrimer of the present invention can also be anionic, forming the carboxylate, phosphonate or sulfonate groups.

Therefore, the present invention includes not only the dendrimers themselves, but any of their salts, e.g. alkali metal or alkaline earth metal, which can be selected from sodium, potassium or calcium salts, preferably are sodium salts.

In another preferred embodiment, the generation dendrimer can be zero and can be represented by the following formula (III): 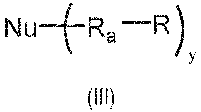 where: Nu represents a polyfunctional core as defined above, Ra is an alkyl group (C1-C6) and y is an integer ranging from 2 to 6, preferably is 4 when the core is silicon, and R is the terminal group of general formula (I) as described above. If the core is silicon or polyphenol, dendrimer of formula (III) may be of formula IIIa or IIIb, respectively: 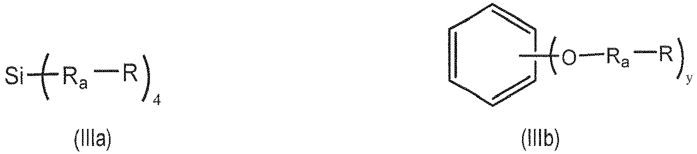 where: R, Rₐ, and y are defined above. In the case of IIIb, y is preferably 3.

The term "generation" refers to the iterative number of branches that are necessary for the preparation of the dendrimer.

In another preferred embodiment, the dendrimer is at least of first generation and can be represented by the following general formula (IV): 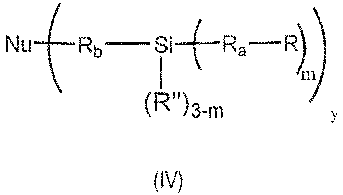 where: Nu represents a polyfunctional core as defined above, Rₐ R_{b} and R^{II} are the same or different and represent an alkyl group (C1-C6), m is an integer ranging between 1 and 3, preferably m is 2; y is an integer ranging between 2 and 6, and R is the terminal group of general formula (I) as described above. If the core is silicon or polyphenol, dendrimer of formula (IV) may be of formula IVa or IVb, respectively: 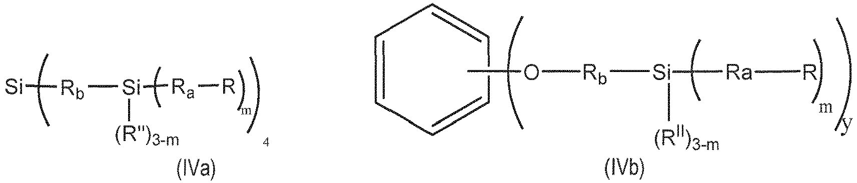 where: R, Rₐ R_{b}, R^{II}, m and y are defined above. In the case of IVb, y is preferably 3.

In another preferred embodiment, the dendrimer is at least of second generation and can be represented by the following formula (V): 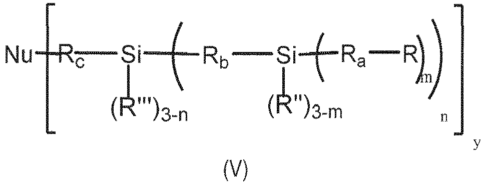 where: Nu represents a polyfunctional core as defined above, Rₐ, R_{b}, R_{c}, R^{II} and R^{III}, are the same or different and represent an alkyl group (C1-C6), m and n are the same or different and is an integer ranging between 1 and 3; preferably m and/or n is 2, and y is an integer ranging between 2 and 6, and R is the terminal group of general formula (I) as described previously. If the core is silicon or polyphenol, dendrimer of formula (V) may be of formula Va or Vb, respectively: 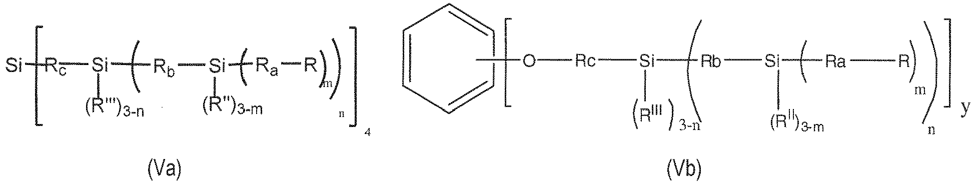 where: R, Rₐ, R_{b}, R_{c}, R^{II}, R^{III}, m, n and y are defined above. In the case of Vb, y is preferably 3.

In another preferred embodiment, the dendrimer is at least of third generation and can be represented by the following formula (VI): 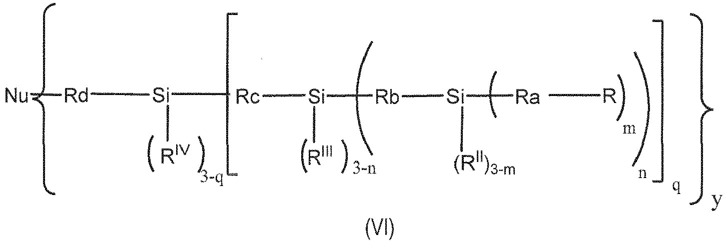 Rₐ, R_{b}, R_{c}, R_{d}, R_{II}, R_{III} and R_{IV} are the or and an alkyl (C1-C6), m, n and q are the same or different and is an integer ranging between 1 and 3; preferably m, n and q is 2; y is an integer ranging between 2 and 6, and R is the terminal group of general formula (I) as described previously above. If the core is silicon or polyphenol, dendrimer of formula (VI) may be of formula VIa or VIb, respectively: 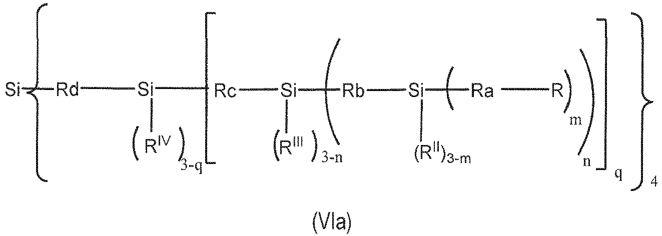 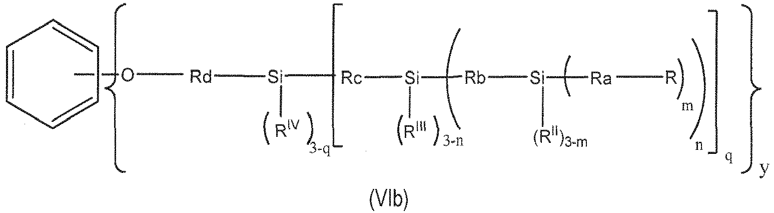 where: R, Rₐ, R_{b}, R_{c}, R_{d}, R^{II}, R^{III}, R^{IV}, m, n, q and y have been previously described. In the case of Vb, y is preferably 3.

In these dendrimers of formula (III) (IV), (V) or (VI) radicals Rₐ, R_{b}, R_{c} or R_{d} may be the same or different and preferably represent an alkyl (C2-C4). In another preferred embodiment, the radical R^{II}, R^{III} and R^{IV} are independent of each other, and represent an alkyl group (C1-C4), more preferably are a methyl group.

These organosilane dendrimers of the invention can be prepared from different generations with high yields using well-known reactions, through divergent methods (growth from the inside out) from a polyvalent core, preferably of tetraallylsilane and subsequent surface functionalization with different functional groups, preferably anionics. Carboxylate and sulfonate dendrimers are stable and soluble in water. Depending on the pH, dendrimers can be obtained as sodium salts, acids or amino acids, providing greater versatility in their applications. Their synthesis is relatively simple, making easier to obtain them in large quantities. In addition, the carbosilane dendrimers have a high chemical inertness, which can be very useful for the study of any biomedical application.

The synthesis of dendritic wedges or dendrons of carbosilane nature with a functional group at the focal point, allow the coupling of several of these dendrons on a polyvalent core. Proper functionalization of the periphery of the dendrimers obtained, permits obtaining anionic dendrimer with end-groups such as carboxylate, sulfonate or phosphonate. That is, the preparation of these anionic derivatives can be done by following a synthetic sequence involving 3 steps:
- Synthesis of dendrons or dendritic wedges with a focal point and an appropriately functionalized periphery.
- Coupling of dendrons or dendritic wedges synthesized in the previous section on a nucleus.
- Functionalization of the periphery of the dendrimers obtained in the previous section with anionic groups of different nature, such as carboxylate, sulfonate or phosphonate.

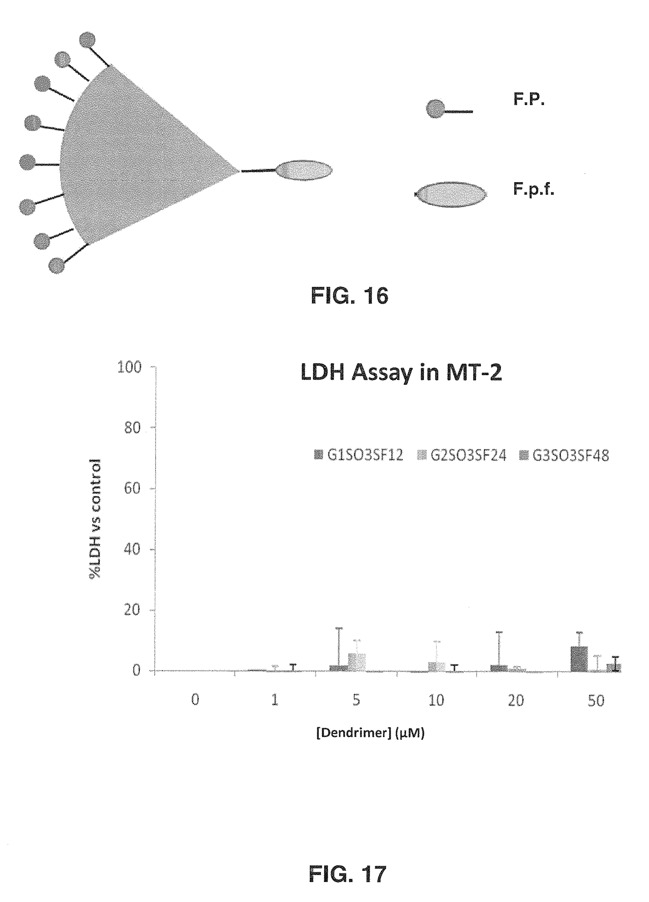

For the synthesis of these dendritic wedges one can start from a haloalkene, more preferably bromoalkene such as allyl bromide or 4-bromobutene using a divergent growth strategy. Depending on the type of functionalization that is achieved in the periphery of these dendrons, dendritic, wedges are available that allow a great versatility of preparation procedures for obtaining anionic carbosilane dendrimers. In this way, one can prepare dendritic wedges having at its focal point a bromo-alkyl group BrCH₂-Dend and exhibiting in its surface different groups such as: allyl, Si-H or Si-Cl, ester terminal groups, etc. A general outline of these dendritic wedges is shown in FIG. 16.

The dendrons can have in its focal point a functionality -CH₂Br, so it can be anchored onto a core, giving rise to dendrimers with different functional groups on the surface. This coupling reaction of dendritic wedges with C-Br bonds at the focal point of various derivatives, such as polyphenols, may be performed generally in a solvent such as acetone, tetrahydrofuran (THF) or dimethylformamide (DMF), heated to temperatures between 60 and 100° C for several hours in the presence of a base such as potassium carbonate (K₂CO₃) or cesium carbonate (Cs₂CO₃) and adding crown ether, for example 18-C-6, to favor the solubility of the inorganic salt in the organic solvent used.

Once synthesized the dendrimers described above, the formation of dendrimers with carboxylic, phosphonic or sulphonic groups, and more preferably their anionic groups on the surface, can be approached in different ways depending primarily on the features present in the starting dendrimer and secondly on the basis on the nature of the anionic groups that are desired in the final dendrimer:
Thus, to obtain carboxylate derivatives two different methods can be used, the first is the hydrosilylation reaction of allylamine with Si-H terminated dendrimers using a platinum catalyst, followed by Michael addition of methyl acrylate on the amino groups, -NH₂ terminated dendrimer and subsequent conversion of the ester groups resulting in carboxylate groups by treatment with a base such as NaOH. The second method is to prepare an initial ester allyl terminal amine groups through Michael type addition of methylacrylate and subsequent hydrosilylation of the functionalized allylamine with Si-H -terminated dendrimers. In this case, also the final step would be the transformation of the terminal ester groups to carboxylate groups by adding a base.

On the other hand, dendrimers with sulfonate groups can be initially prepared through the hydrosilylation of allylamine with Si-H -terminated dendrimers and subsequent Michael type addition of sodium vinyl sulfonate on NH₂-terminater dendrimers resulting in the first stage.

In the case of phosphonate derivatives, they can be prepared initially by synthesizing NH₂-terminated dendrimers and later introducing the corresponding groups by adding methyl phosphite, HPO(OMe)₂. Again the formation of the anionic derivatives with terminal phosphonate groups can be achieved by adding base, e.g. NaOH, on the previous dendrimers.

Therefore, another aspect of the present invention relates to a method for obtaining dendrimers of the invention, including the Michael type addition of a dendrimer containing a multivalent-core and terminal NH₂ groups to an alkene containing carboxylic or sulfonate groups, for example, methyl acrylate or vinyl sulfonate (for the functionalization of the dendrimer surface with carboxylate or sulfonate groups) or two-step reaction of these dendrimers with formaldehyde and dimethyl phosphonate (for the functionalization of the dendrimer surface with phosphonate groups). A scheme could be as follow: 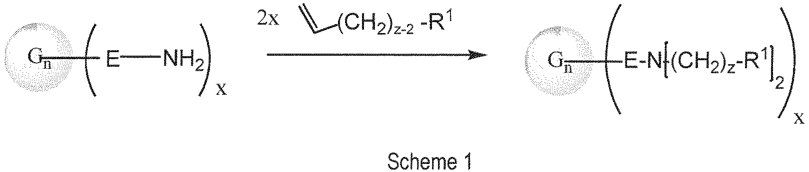 Where: E, z R¹ have been described above, n represents the number of generations (G) and X the number of terminal groups, which will depend on the number of generations.
By "alkene" refers herein to liniar or branched unsaturated hydrocarbon containing at least one terminal double bond, are chains that have 2 to 4 carbon atoms and further comprise a carboxylic or sulfonate group.
In a preferred embodiment of the procedure for obtaining dendrimers of the invention, the NH₂-ending dendrimers can be obtained by hydrosilylation of allylamine with carbosilane dendrimers of different generations with terminal Si-H bonds.
In another preferred embodiment, the dendrimer containing an NH₂ group is obtained by:
a) the synthesis of dendrons with a haloalkyl at the focal point and functionalized with allyl groups from an haloalkene;
b) coupling of the dendrons obtained in step (a) to a multivalent-core, and
c) the functionalization of the dendrimer obtained in (b) with terminal-NH₂ groups.

Another aspect of the present invention relates to a method for obtaining dendrimers of the invention, comprising the hydrosilylation of a dendrimer containing a Si-H groups with a terminal allyl amine group functionalized with carboxylic, phosphonic or sulfonic groups.

In another preferred embodiment, the multivalent dendrimer containing polyvalent nuclei and Si-H terminal groups is given by:
a) the synthesis of dendrons with a haloalkyl at the focal point and functionalized by Si-H groups from an haloalkene, and
b) coupling of the dendrons obtained in step (a) to a multivalent core, or alternatively
a') the synthesis of dendrons with an haloalkyl at the focal point and functionalized with allyl groups from an haloalkene
b') coupling of the dendrons obtained in step (a') to a nucleus, and c') the functionalization of the dendrimer obtained in (b') with Si-H terminal groups.

By "haloalkene" means in the present invention to an alkene (C1-C6) substituted by a halogen atom e.g. bromine, iodine or chlorine, more preferably is a bromoalkene (C1-C6), even more preferably is a bromoalkene (C2-C4), such as 4-bromobuteno. In the process of obtaining the dendrimers of the invention, the NH₂-terminated dendrimers can be obtained using the "click chemistry" methodology for the union of carbosilane dendrimer containing propargilamine on different generations of carbosilane dendrimers functionalized with terminal azide groups. But, as described above, the functionalization of the amino terminus with carboxylic, sulfonic or phosphoric groups may also be pre-binding to the surfaces of the dendrimer skeleton.
Therefore, another aspect of the present invention relates to a method for obtaining dendrimers of the invention, including the initial functionalization of organic amines with carboxylic groups, sulfonic or phosphoric or their group precursors, and subsequent binding to carbosilane dendrimers of different generations with terminal Si-H bonds or functionalized with terminal azide groups.
In another aspect, the present invention relates to a compound of formula (VII):

Et₃-R (VII)

where Et is an ethyl group and R is the terminal group of formula (I) as described above, a group -Si (R')₃₋ₚ[(CH₂)₄-N₃]ₚ, or the group -Si(R')₃₋ₚ[E-NH₂]ₚ wherein R' is an alkyl group (C1-C4), preferably R 'is methyl, p varies between 1 and 3, preferably p is 1 and E is a bonding group as described above.

Therefore, other reaction intermediates can be carbosilane dendrimers functionalized with azide or amine terminal groups comprising:
- a multifunctional core defined above, preferably tetraallylsilane or polyphenol and
- an outer layer, which consists, wholly or partly in the same or different units of the group -Si(R')₃₋ₚ[(CH₂)₄-N₃]ₚ or the group -Si(R')₃₋ₚ[E-NH₂]ₚ where: R', p and E have been described above, preferably E is the group - (CH₂)ₓ-R³ and R³ is triazole, x is defined in claim 6, preferably x is 4.

Dendrimers of the invention can be applied in different fields of biomedicine, among which include its use as therapeutic agents, antiviral, antibacterial or antiprionics. In addition to its antimicrobial activity, also have anti-inflammatory activity, improving its prophylactic properties as the probability of infection from HIV increases significantly in the presence of inflammatory processes. It is clear that a good vaginal topical microbicide should prevent infection by HIV and maintain the integrity of vaginal epithelial barrier. In this sense a process of vaginal inflammation may increase HIV infection by activation of the dendritic cells and recruiting T cells (targets of HIV) to the site of inflammation, increasing the risk of HIV infection and activating the transcription process via cytokines, regulating the transcription factor NF-kappaB, which in turn binds to and activates the HIV. The transcription factor NF-kappaB is a major drug targets for anti-inflammatory compounds. The anti-inflammatory property of dendrimers of the invention is an additional advantage over other dendrimers with antiviral, antibacterial or antiprionics activity known in the state of art.

Therefore, another aspect of the present invention relates to dendrimers as a drug, beeing this drug of choice for the prevention and / or treatment of diseases caused by viruses, bacteria or fungi, and more preferably when the disease is caused by strains of HIV. More preferably, these dendrimers are anionic.
As an antiviral, the dendrimer of the invention of nanoscopic dimensions, prevents the proper process of adherenting to the target cell and the infection of this and the related production of new viral particles.
In addition to the prophylactic application, this nanoparticie could prevent infection of cells not yet infected due to the results of the experiments, also the dendrimers of the invention have therapeutic effect, since in infected cells this nanoparticle reduces HIV viral replication, but mostly have therapeutic effect for sexually transmitted diseases (STDs) (antiviral, antibacterial or antifungal). Therefore, in a preferred embodiment diseases are sexually transmitted diseases by acting the dendrimers of the invention as antiviral, antibacterial or antifungal systems.
The term "sexually transmitted disease" means in the present invention to infections that are acquired by having sex with someone who is infected and the infection may be caused by bacteria, fungi, protozoa and viruses, such as infection caused by HIV. The vast majority of HIV infections occur because of the lack of protection when having sex with an infected person.
Another aspect of the present invention relates to a pharmaceutical composition comprising at least one dendrimer as described above and a pharmaceutically acceptable carrier. In addition, this pharmaceutical composition can comprise another active product.

The "pharmaceutically acceptable carriers " that can be used in such compositions are vehicles known to one expert in the state of art.

Examples of pharmaceutical composition includes any solid (tablets, pills, capsules, granules, etc.) or liquid (gels, solutions, suspensions or emulsions) for oral, nasal, topical or parenteral administration, preferably the administration will be topical and still more preferably in the form of gel.
Another aspect of the present invention relates to a method of treating or preventing diseases caused by viruses, bacteria or fungi in a mammal, preferably a human, comprising the administration of a therapeutically effective amount of a composition comprising at least one dendrimer of the invention. Preferably the administration of the composition can be done orally, nasal, topical or parenteral.
As used herein, the term "therapeutically effective amount" refers to the quantity of composition calculated to produce the desired effect and generally will be determined, *inter* alia, by the characteristics of the composition, age, condition and history of the patient, the severity of the disease, and the route and frequency of administration. It is also possible to use the dendrimers of the invention as vehicles for transporting molecules, preferably molecules with pharmacological activity (active), and more preferably positively charged molecules when negatively charged dendrimers are used.
Throughout the description and claims, the word "comprise "and its variations are not intended to exclude other technical features, additives, components or steps. For those experts in the state of art, other objects, advantages and features of the invention will emerge from the description and part of the practice of the invention. The following examples and drawings are provided as examples and not intending to be limiting of the present invention.

### DESCRIPTION OF THE FIGURES

**FIG. 1****.** Shows the cytotoxicity of the three generations of anionic dendrimers with terminal groups carboxylate (G1COO8, G2COO16 and G3COO32) and sulfonate (G1SF8, G2SF16 and G3SF32) at a concentration of 10 µM in HEC-1A (human endometrial epithelial cells). FIG. 1A, LDH assay (release of lactate dehydrogenase). The Tx-100 (triton x-100) 1% represents 100% of toxicity. FIG. 1B Bromide MTT assay. DMSO 20% represents 100% of toxicity.
Dx (Dextran) is used as a safe control molecule, DxS (Dextran sulfate) and Suramin are molecules with anionic groups used as positive control for inhibition of HIV.
**FIG. 2****.** Shows the viability of the PBMC in the presence of different concentrations of G2COO16 and G2SF16. FIG. 2A, LDH assay and FIG. 2B, MTT assay.
**FIG. 3****.** Shows cytotoxicity of G2SF16 dendrimer on human blood erythrocytes (release of hemoglobin into the supernatant).
**FIG. 4****.** Represents a 4 days lymphoproliferative assay in PBMC. PBMC were stimulated with 2 µg/mL of PHA (purified phytohemagglutinin). FIG. 4A shows that the dendrimer G2SF16 has no mitogenic effect. FIG. 4B shows that the dendrimer G2SF16 inhibits the mitogenic effect of PHA in the PBMC.
**FIG. 5****.** Represents a test of adsorption of viral particles to the monolayer of epithelial cells in the presence of a pretreatment with dendrimers, G2COO16 G2SF16 and at different times. Assesses the amount of HIV by an enzyme immunoassay for the quantification of Agp24 in the culture supernatant (p24 ELISA). After 24 hours of infection (Fig. 5A) and after 72 hours of infection (Fig. 5B).
**FIG. 6****.** Shows a text of internalization of viral particles through the cell line HEG-1A. Cells were pretreated for 1 h with the dendrimers before infecting them with X4 HIV NL4.3 for 3h. After infection HEC-1A cells were lysed with TX-100 at 0.2% to quantify the viral particles within the cell.
**FIG. 7****.** Shows the mechanism of interaction between the dendrimers and the viral particles. Strains X4 HIV NL4.3 and R5 HIV BaI were pretreated with dendrimers before be exposed to the PBMC. The viral supernatant was collected after 4 days.
**FIG. 8****.** Shows inhibition of transcytosis in HEC-1A. We evaluated the amount of Agp24 in the basolateral of the transwell devices used to simulate a single layer of the vaginal mucosa.

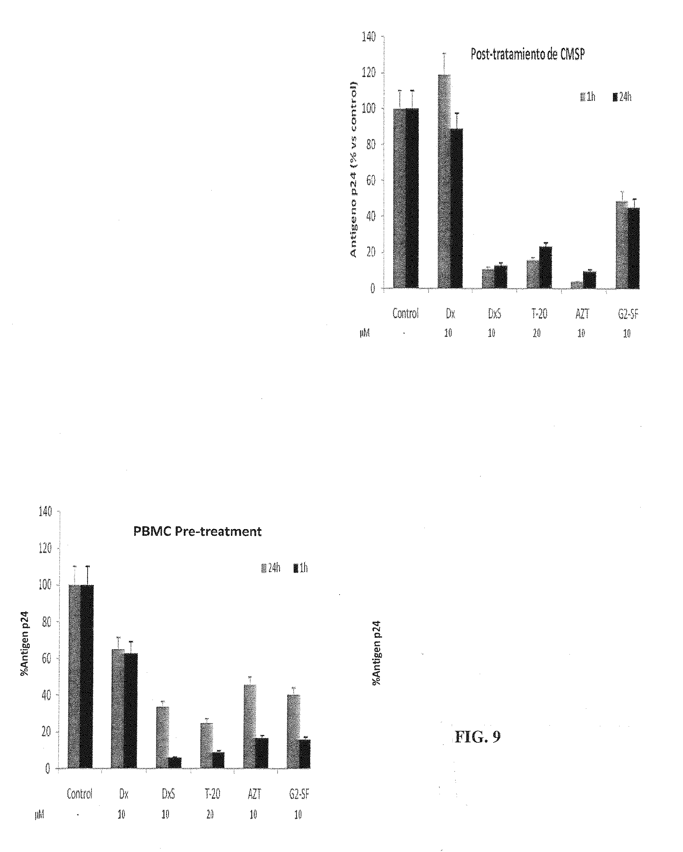

**FIG. 9****.** Shows the evaluation of prophylactic and therapeutic effect of the dendrimer G2SF16 in PBMC. PBMC were treated at different times before and after being infected by HIV to analyze the activity of the dendrimer G2SF16. T-20 is an inhibitor of viral membrane fusion with the cell membrane via the gp41 protein, inhibits virus entry into the cell and AZT is a reverse transcriptase inhibitor. In summary both are inhibitors of viral replication and are used as controls for inhibiting virus replication. FIG. 9A is pretreatment, first dendrimer is added and then infection, the FIG. 9B is post-treatment, that is first infected and then add the dendrimer.
**FIG. 10****.** Shows the inhibition of HIV entry into dendritic cells and macrophages. Cells were pretreated for 1 h with the dendrimer G2SF16. Cells were infected with strains X4 HIV NL4.3 (black columns) and R5 HIV BaI (gray columns).
**FIG. 11****.** Shows the bactericidal effect of anionic carbosilane dendrimers. Representation of the concentration-dependent effect of ampicillin and dendrimers G2SF16 and G2COO16 at 24h post-treatment of S. *aureus.* The slope of the triangle represents the decrease of the concentration of antibiotic (*2*, *1, 0.5, 0.25*, *1.12, 0.6, 0.3, 0.15 □G*/*mL)* or dendrimer (*160, 80, 40, 20, 10,* 5, *2.5, 1.25 □*M)*.* The gray bar represents the dendrimer at 10 µM.
**FIG. 12****.** Shows the bacteriostatic effect of anionic carbosilane dendrimers. Representation of the concentration-dependent effect of ampicillin and dendrimers G2SF16 and G2COO16 at 24h post-treatment of S. *aureus.* The slope of the triangle represents the decrease of the concentration of antibiotic *(2, 1,* 0.5, *0.25, 1.12,* 0.6, *0.3, 0.15* µg/mL) ordendrimer*(160, 80, 40, 20, 10,* 5, *2.5, 1.25 □*M). The gray bar represents the dendrimer at 10 µM.
**FIG. 13****.** Shows the study of the biocompatibility and inhibitory effect on HIV entry of dendrimers obtained via click chemistry. FIG. 13A represents a curve of toxicity of the dendrimers in HEC-1A cells by MTT assay. FIG. 13B shows inhibition of HIV entry into cells HEC-1A after pretreated with dendrimers G2CKC0016 and G2CKP32 1h before infection.
**FIG. 14****.** Shows the inhibition of TNF-alpha (TNF) in T lymphocytes. Transfection of Jurkat cells with TNF-luc plasmid in the presence of G2SF16. aTNF is anti-TNF-alpha.
**FIG. 15****.** Shows Jurkat cell transfection with plasmid pNF-kB-luc in the presence of G2SF16.
**FIG. 16****.** Schematically represents a carbosilane dendritic wedge (dendron) with dual functionality, in the focal point (F.p.f) and in the periphery (FP)
**FIG. 17****.** Shows a study of the release of LDH after 24 hours of contact between the three generations of dendrimer and MT-2 cells.
FIG. 18. Shows a study of the release of LDH after 24 hours of contact between the three generations of dendrimer and the PBMC.
FIG. 19. Shows a study of mitochondrial activity in the PBMC in contact with different concentrations of dendrimer at 24h.
FIG. 20. Represents the inhibition of HIV transcytosis in a monolayer of HEC-1A cells. This monolayer was treated with 70 ng of Bal virus at the apical part 1h after pretreatment with the dendrimer and basolateral supernatant was collected after 48h.
FIG. 21. Represents the inhibition of infection of PBMC pretreated 1 h with G203SF24. All reagents were added to a final concentration in the well of 10 µM.
FIG. 22. Shows a test of inhibition of infection in macrophages (M), immature dendritic cells (Di) and mature dendritic cells (Dm) after 4 days. Cells were pretreated 1 h before infected with NL4.3 (3ng).
**FIG. 23****.** Representation of bactericidal effect depending on the concentration of ampicillin (commercial antibiotic) and G203SF24 at 24h post-treatment of S. *aureus.* The slope of the triangle represents the decrease of the concentration of antibiotic (*2*, *1*, *0.5, 0.25, 1.12, 0.6, 0.3, 0.15 □*g/mL) or dendrimer (*160, 80, 40, 20, 10, 5, 2.5, 1.25* □M). The gray bar represents the dendrimer at 10 µM.
**FIG. 24****.-** Representation of bacteriostatic effect depending on the concentration of ampicillin (commercial antibiotic) and G203SF24 at 24h post-treatment of S. *aureus.* The slope of the triangle represents the decrease of the concentration of antibiotic (*2; 1; 0.5; 0.25; 1.12; 0.6; 0.3; 0.15 µg*/*mL)* or dendrimer (*160; 80; 40; 20; 10; 5; 2.5; 1.25 µM).* The gray bar represents the dendrimer at 10 µM.
**FIG. 25****.-** Representation of fungicide effect depending on the concentration of amphotericin B (commercial fungicide) and G203SF24 at 24h post-treatment of *C.albicans.* The slope of the triangle represents the decrease of the concentration of amphotericin B *(2; 1; 0.5; 0.25; 1.12; 0.6; 0.3; 0.15 µg*/*mL)* or dendrimer *(160; 80; 40; 20; 10; 5; 2.5; 1.25 µM).* The gray bar represents the dendrimer at 10 µM.
**FIG. 26****.-** Representation of fungistatic effect depending on the concentration of amphotericin B (commercial fungicide) and G203SF24 at 24h post-treatment of *C.albicans.* The slope of the triangle represents the decrease of the concentration of amphotericin B *(2; 1; 0.5; 0.25; 1.12; 0.6; 0.3; 0.15 µg*/*mL)* or dendrimer *(160; 80*; *40; 20; 10; 5; 2.5; 1.25 µM*). The gray bar represents the dendrimer at 10 µM.

### EXAMPLES

The following illustrate the invention by a test conducted by the inventors, highlighting the effectiveness of carbosilane dendrimers and their synthetic methods.

### EXAMPLE 1: Synthesis of the invention dendrimers grown from a core of Silicon.

The synthesis was carried out following two methods listed below:

### METHOD 1: Synthesis of carbosilane dendrimers from a tetrallylsilane core with terminal amino groups.

The synthesis of carbosilane dendrimers with terminal amino groups was carried out by two strategies: 1) hydrosilylation of allyl amines with different generation of carbosilane dendrimers containing terminal Si-H bonds, or 2) using the click chemistry methodology to bond propargylamine to different generation carbosilane dendrimers containing terminal azides.

### METHOD 1 (a): synthesis of anionic dendrimers from carbosilane dendrimers with terminal amino groups obtained by hydrosilylation of allylamine.

In general, to simplify the way of naming the dendrimers as described in these examples can be represented as **Gn-(Fp)x,** where:
**n,** indicates the number of generation **G.**
**F,** indicates the nature of the functional groups located on the periphery of the dendrimer
**p** indicates the number of functional groups in each branch.
***x*** denotes the number of terminals units present on the dendrimer

When the dendrimer is zero generation, G0, n=0, or when it is first generation G1, n=1, or second-generation, G2, i.e., n = 2, the general structures would be as follows:

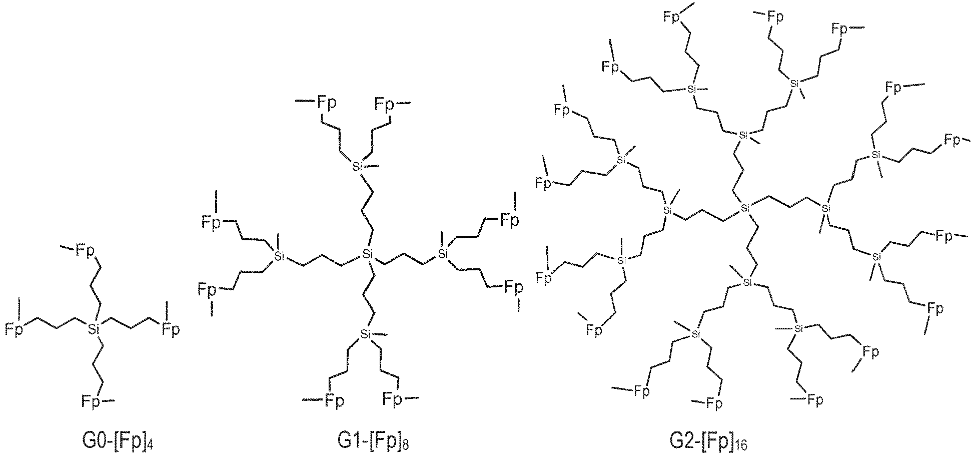

The corresponding mononuclear compound would be: Et₃-[Fp], where Et is ethyl group.

The anionic dendrimers were prepared starting from carbosilane dendrimer with amino terminal groups or their mononuclear counterpart triethylsilyl substitute (Et₃-[Si(CH_{2]3}NH₂] (1), where F is Si(CH₂)₃NH₂ group and the formulae would be Gn-[Si(CH₂)₃NH₂]ₓ,(n = 0, x = 4 (2); n = 1, x = 8 (3); n = 2, x = 16 (4)). These dendrimers of different generations would be prepared following reactions described in the literature through divergent procedures (M. Angeles Muñoz-Fernández et al. "Water-Soluble Carbosilane Dendrimers: Synthesis, Biocompatibility and Complexation with Oligonucleotides; evaluation for Medical Applications" Chem. Eur. J.. 2007, 13, 483-495). From these compounds, the anionic dendrimers with carboxylate, sulfonate or phosphonate groups finally could be obtain following the procedures described below:

### METHOD 1 (a) carboxylate dendrimers

The synthesis of anionic carboxylate dendrimers of different generation was carried out by Michel addition of methyl acrylate to dendrimers with terminal amino groups -NH₂, **1-4,** described above. The reaction conditions are described in the scheme 2 and thus obtained the dendrimers Gn-[Si(CH₂)₃N(CH₂CH₂COOMe)₂]ₓ: (triethylsilil: Et₃-[Si(CH₂)₃N(CH₂CH₂COOMe)₂] **(5);** G0-[Si(CH₂)₃N(CH₂CH₂COOMe)₂]₄ n = 0 and x = 4 **(6);** G1-[Si(CH₂)₃N(CH₂CH₂COOMe)₂]₈, n = 1 and x = 8 (7); G2-[Si(CH₂)₃N(CH₂CH₂COOMe)₂]₁₆ n = 2 and x = 16 **(8))** as colorless oils soluble in common organic solvents, but insoluble in water. To illustrate this, the structures of triethylsilyl intermediate (5) and dendrimers (6), (7) and (8) are represented bellow.

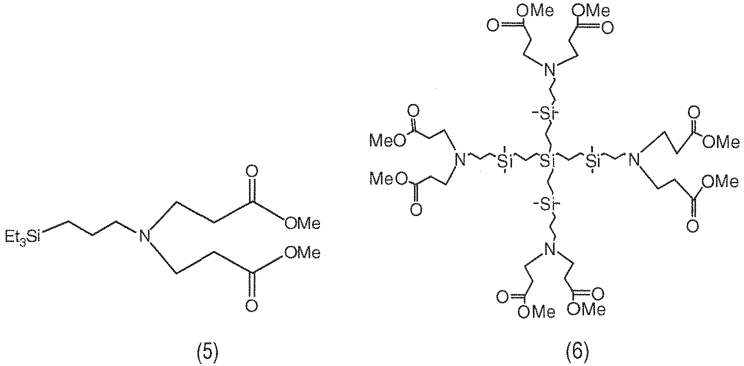

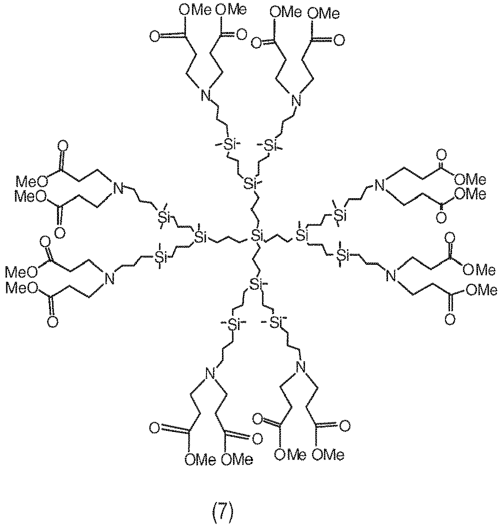

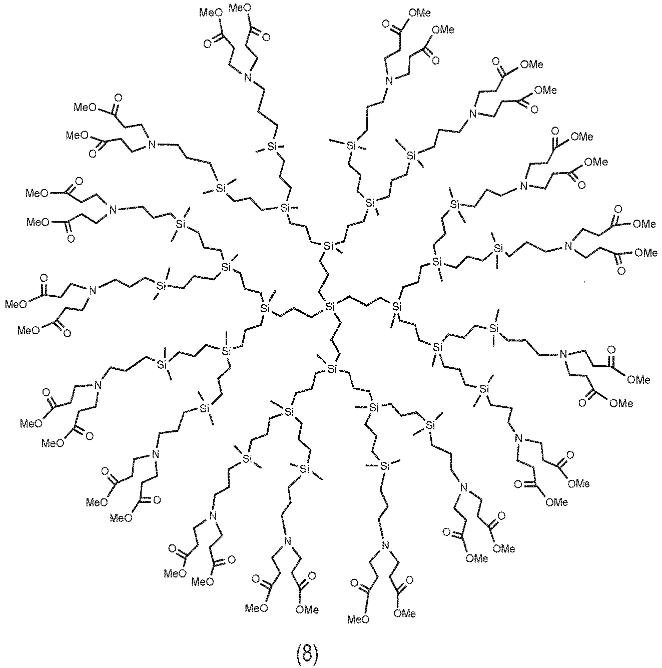

The derivatives **5-8,** can be transformed into the corresponding anionic carboxylate derivatives by treatment with NaOH, the resulting compounds Gn-[Si(CH₂)₃N(CH₂ CH₂COONa)₂]ₓ: triethylsilane Et₃-[Si(CH₂)₃N(CH₂CH₂COONa)₂] **(9);** G0-[Si(CH₂)₃N(CH₂CH₂COONa)₂]₄ n = 0 and x = 4 **(10);** G1-[Si(CH₂)₃N(CH₂CH₂COONa)₂]₈ n = 1 and x = 8 **(11);** G2-[Si(CH₂)₃N(CH₂CH₂COONa)₂]ₓ n = 2 and x=16 **(12),** are isolated as white solids soluble in water with high yield (Scheme 2.) Additionally treating compounds **9-12** with an aqueous solution of HCl 1 M lead cationic derivatives Gn-[Si(CH₂)₃N⁺H(CH₂CH₂COOH)₂]ₓ: triethylsilyl Et₃-[Si(CH₂)₃N⁺H(CH₂CH₂COOH)₂] **(13);** G0-[Si(CH₂)₃N⁺H(CH₂CH₂G00H)₂]₄, n = 0 and x = 4 **(14);** G1-[Si(CH₂)₃N⁺H(CH₂CH₂COOH)₂]₈ n = 2 and x = 8 **(15);** G2-[Si(CH₂)₃N⁺H(CH₂CH₂COOH)₂]₁₆ n = 3 and x = 16 **(16))** (Scheme 2).

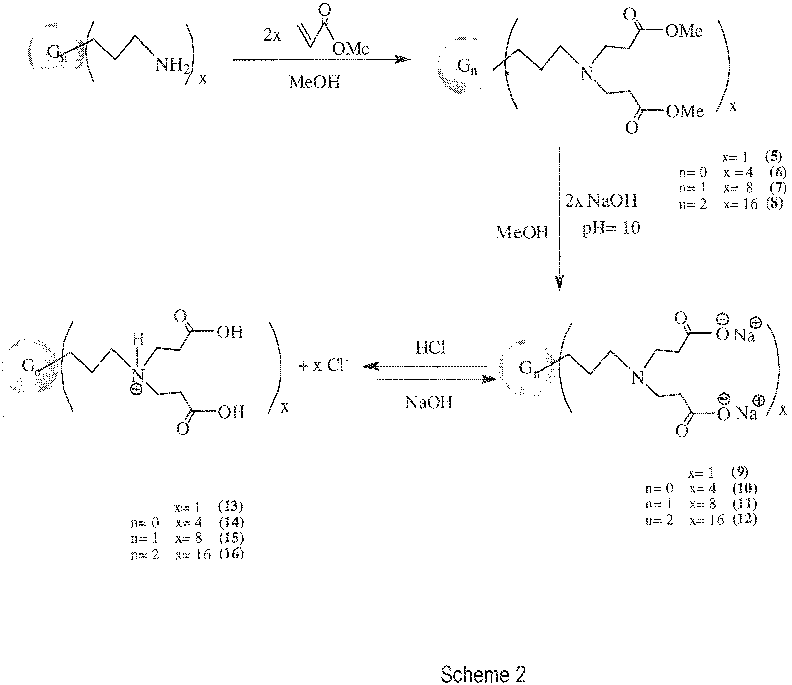

Derivatives **13-16** are also converted in the anionic dendrimers, **9-12,** by reversible process by treatment with a solution of NaOH, and reverted to the first compounds if again treated with an aqueous solution of HCl 1 M (Scheme 2).

As an example dendrimers **13** and **14** are represented as follows:

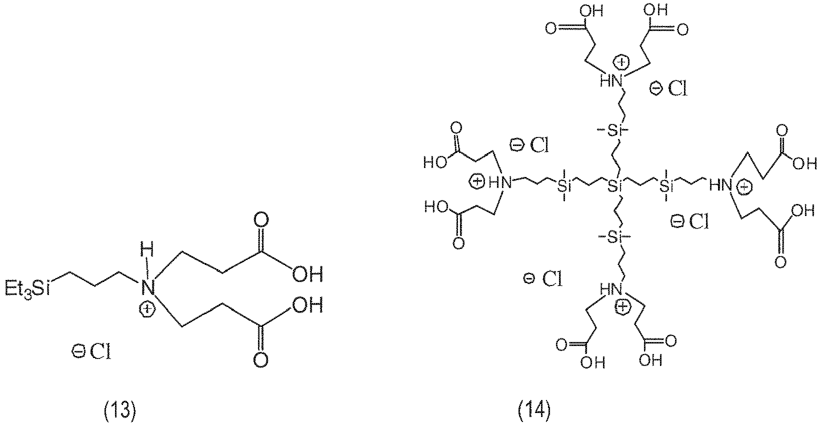

### METHOD 1 (a) sulfonate dendrimers.

The synthesis of anionic dendrimers of different generations with sulfonate end groups was carried out by a Michael type addition reaction of sodium vinyl sulfonate to dendrimer with terminal amino groups -NH₂, **1** to **4.** The reaction is carried out in an ethanol-water mixture, heating at 120 °C, obtaining the resulting anionic derivates Gn-[Si(CH₂)₃N(CH₂CH₂SO₃Na)₂]ₓ (Triethylsilyl Et₃-[Si(CH₂)₃N(CH₂CH₂SO₃Na)₂] (17); G0-[Si(CH₂)₃N(CH₂CH₂SO₃Na)₂]₄ n = 0, *x =* 4 (18); G1-[Si(CH₂)₃N(CH₂CH₂SO₃Na)₂]₈ n = 1 and *x*=8 (19); G2-[Si(CH₂)₃N(CH₂CH₂SO₃Na)₂]₁₆ n = 2 and *x*= 16 **(20)),** as white solids soluble in water in a high yield and can be stored for long periods of time without showing signs of decomposition. (Scheme 3).

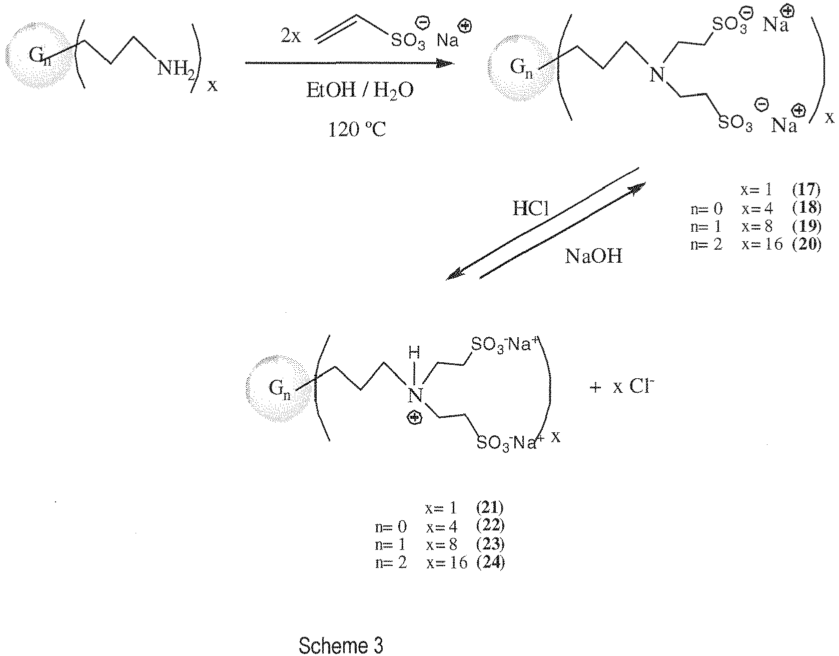

Analogously to the carboxylate derivates, the anionic dendrimers 17-20, can be protonated by treatment with an excess of a 1M HCl solution in water, yielding cationic dendrimers Gn-[Si(CH₂)₃N⁺H(CH₂CH₂SO₃⁻Na⁺)₂]ₓ (triehylsilyl Et₃-[Si(CH₂)₃N⁺H(CH₂CH₂SO₃⁻Na⁺)₂] (21); G0-[Si(CH₂)₃N⁺H(CH₂CH₂SO3⁻Na⁺)₂]₄ n = 0 and *x* = 4 (22); G1-[Si(CH₂)₃N⁺H(CH₂CH₂SO₃⁻Na⁺)₂]₈ n = 2 and x = 8 (23); G2-[Si(CH₂)₃N⁺H(CH₂CH₂SO₃⁻Na⁺)₂]₁₆ n=3 and x = 16 **(24)),** which can revert back to the first by treatment with a solution of NaOH (Scheme 3).

### METHOD 1 (a) phosphonate dendrimers.

The synthesis of dendrimers with anionic phosphonate groups at the periphery can not be obtained from dendrimers with terminal-NH₂ by hydrosilylation with allylamine, the of carbosilane dendrimers with terminal-NH₂ prepared by hydrosilylation with allylamine, because the reaction of these derivatives with formaldehyde and dimethyl phosphite, HPO(OMe)₂, does not lead to the functionalization of dendritic systems with phosphonate groups

### METHOD 1 (b): Synthesis of anionic dendrimers from dendrimers with terminal amino groups obtained by coupling of propargyl amine groups on azide-terminated dendrimers via click chemistry.

This synthetic route initially involves the preparation of carbosilane dendrimers, grown from a tetraallylsilane core with terminal azide groups. The preparation of these azide derivatives was carried out from dendrimers containing Si-H terminal bonds of the type Gn-[Si-H]ₓ in two steps (Scheme 4). First the hydrosilylation reaction of nG-[Si-H]ₓ with 4-bromobutene leads to different generations of carbosilane dendrimers with terminal CH₂Br groups, Gn-[Si-CH₂CH₂CH₂CH₂Br]ₓ (triehylsilyl Et₃-[Si-CH₂CH₂CH₂CH₂Br] (**25**); G0-[Si-CH₂CH₂CH₂CH₂Br]₄ n = 0 and x = 4 (**26**); G1-[Si-CH₂CH₂CH₂CH₂Br]₈ n = 1 and x = 8 (27); G2-[Si-CH₂CH₂CH₂CH₂Br]₁₆ n = 2 and x = 16 (**28**)). The subsequent reaction of derivatives **25-28** with sodium azide leads to carbosilane dendrimers with terminal azide groups Gn-[Si-CH₂CH₂CH₂CH₂N₃]ₓ (triethylsilyl Et₃-[Si-CH₂CH₂CH₂CH₂N_{3]} (**29**); G0-[Si-CH₂CH₂CH₂CH₂N₃]₄ n=0 and x=4 (30); G1-[Si-CH₂CH₂CH₂CH₂N₃]₈ n =1 and x =8 (**31**); G2-[Si-CH₂CH₂CH₂CH₂N₃]₁₆ n=2 and x = 16 (**32**)) (see Scheme **4**) 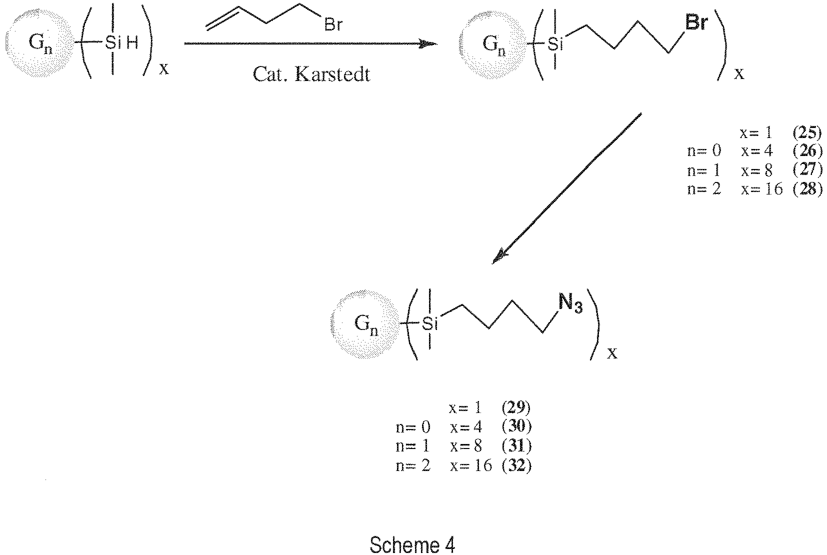 The compounds **25** to **32** are air-stable oily solids, soluble in most commonly used organic solvents and can be stored without decomposition for extended periods of time.

Once prepared dendrimers with terminal azide groups, the synthesis of dendrimers with amino groups (-NH₂) on the surface is produced by a type "click chemistry" reaction between the **29-32** compound and propargyl amine compounds, leading to derivatives *ck*-Gn-[Si(CH₂)₄(N₃C₂H)CH₂NH₂]ₓ (triethylsilyl Et₃-[Si(CH₂)₄(N₃C₂H)CH₂NH_{2]} (33); *ck*-G0-[Si(CH₂)₄(N₃C₂H)CH₂NH₂]₄ n = 0 and x = 4 **(34);** *ck*-G1-[Si(CH₂)₄(N₃C₂H)CH₂NH₂]₈ n = 2 and x = **8 (35);** *ck*-G3-[Si(CH₂)₄(N₃C₂N)CH₂NH₂]₁₆ n = 3 and x = 16 **(36))** (see Scheme 5)

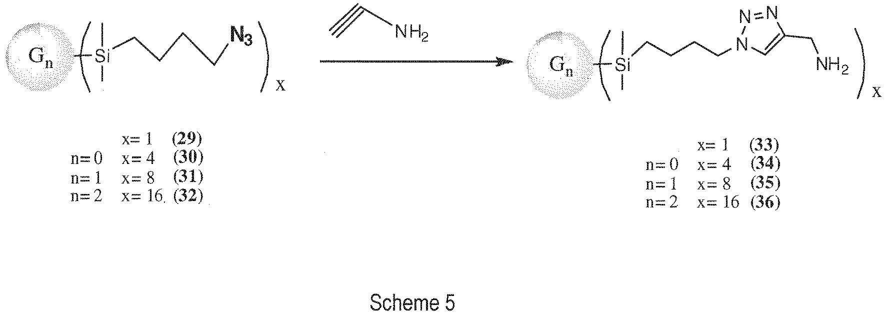

These dendrimers can be simplified by representing them as ck-Gn-(Fp) x, where: ck indicates that dendrimers are obtained by clik coupling between an azide group and a carbon-carbon triple bond and n, G, F, P x are those described above and their structures are as above.

The preparation of anionic dendrimers following this synthetic route start with the synthesis of carbosilane dendrimers with terminal amino groups *ck*-Gn-[Si(CH₂)₄(N₃C₂H)CH₂NH₂]ₓ, **(33)** to **(36)**. From these compounds, the anionic dendrimers obtained with carboxylate, sulfonate or phosphonate groups finally occurs following similar procedures to those described in the previous section for the dendrimers with terminal NH₂ groups obtained from hydrosilylation of allylamine. Thus we have obtained families of compounds that are described below.

### Method 1 (b) for anionic dendrimers with carboxylate groups.

The Michael type addition reaction of methyl acrylate on NH₂ terminated dendrimers, **33-36,** using the reaction conditions described in Scheme 5 lead to the dendrimers *ck*-Gn-[Si(CH₂)₄(N₃C₂H)CH₂N(CH₂CH₂COOMe)₂]ₓ, (triethylsilyl Et₃-[Si(CH₂)₄(N₃C₂H)CH₂N(CH₂CH₂COOMe)₂] **(37);** *ck*-G0-Si(CH₂)₄(N₃C₂H)CH₂N(CH₂CH₂COOMe)₂]₄ n=0 and *x*=4 **(38);** *ck*-G1-[Si(CH₂)₄(N₃C₂H)CH₂N(CH₂CH₂COOMe)₂]₈ n=1 and x=8 **(39);** *ck*-G2-[Si(CH₂)₄(N₃C₂H)CH₂N(CH₂CH₂COOMe)₂]₁₆ n=2 and x=16 **(40))** as colorless oils soluble in common organic solvents but insoluble in water.

The derivatives **37-40**, can be transformed into the corresponding anionic carboxylate derivatives by treatment with NaOH, to obtain the compounds *ck*-Gn-[Si(CH₂)₄(N₃C₂H)CH₂N(CH₂CH₂COONa)₂]ₓ, (triethylsilyl Et₃-[Si(CH₂)₄(N₃C₂H)CH₂N(CH₂CH₂COONa)₂] **(41);** *ck*-G0-[Si(CH₂)₄(N₃C₂H)CH₂N(CH₂CH₂COONa)₂]₄ n=0 and x=4 **(42)**; *ck*-G1-[Si(CH₂)₄(N₃C₂H)CH₂N(CH₂CH₂COONa)₂]₈ n=1 and x=8 **(43);** *ck-*G2-[Si(CH₂)₄(N₃C₂H)CH₂N(CH₂CH₂COONa)₂]₁₆ n=2 and x6 **(44)),** which are isolated as white solids soluble in water in high yield (Scheme 6).

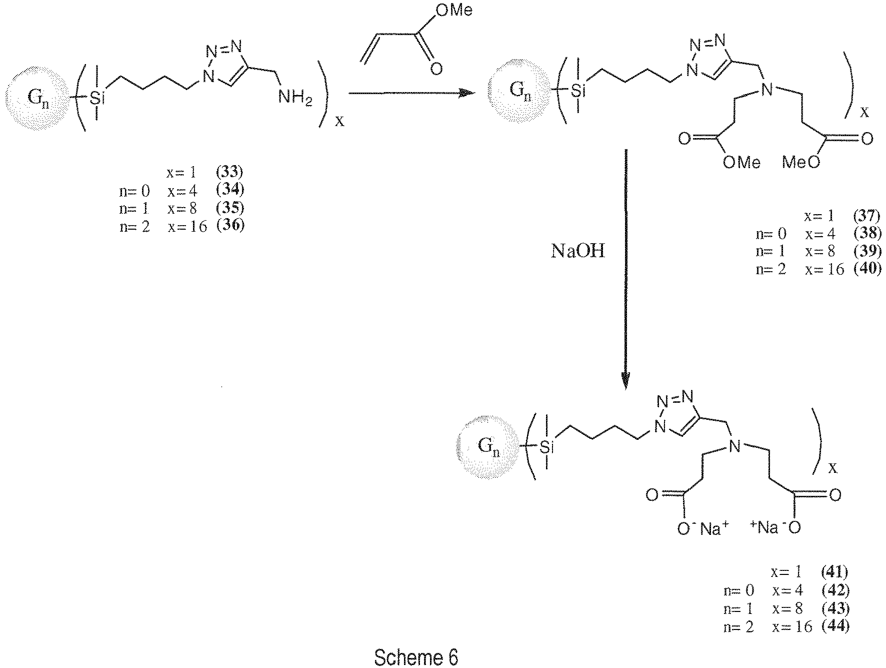

### Method 1 (b) for anionic dendrimers with sulfonate groups.

Michael type addition of sodium vinyl sulfonate to -NH₂-terminated dendrimers, **33-36,** in a water-ethanol mixture at 120 °C leads to the anionic derivatives *ck*-Gn-[Si(CH₂)₄(N₃C₂H)CH₂N(CH₂CH₂SO₃Na)₂]ₓ (triethylsilyl , x = 1 (**45**); n =1, x = 4 (**46**); n = 2, x = 8 (47); n = 3, x = 16 (48)), as white solids soluble in water in high yield and can be stored for long periods of time without showing signs of decomposition (Scheme 7).

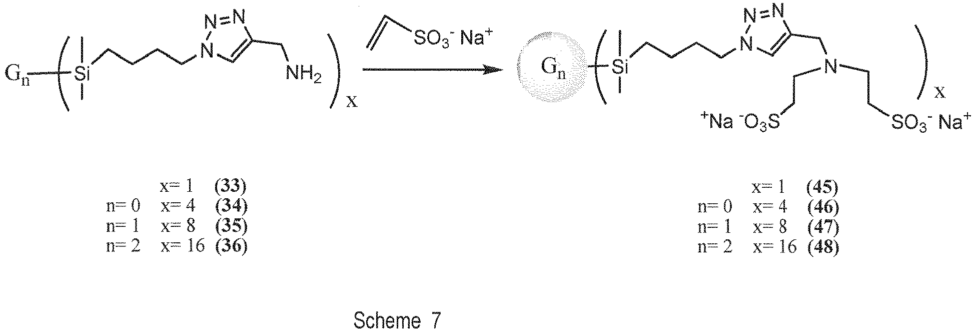

### Method 1 (b) for anionic dendrimers with phosphonate groups.

Dendrimers with terminal -NH₂ groups, **33-36**, react with formaldehyde in THF for 1 hour at room temperature, then methyl phosphite, HPO(OMe)₂, is added "*in situ*" and the mixture is heated at 40 °C for 3 hours, to obtain anionic derivatives *ck*-Gn-[Si(GH₂)₄(N₃C₂H)CH₂N(CH₂PO(OMe)₂)₂]ₓ, (triehylsilyl Et₃-[Si(CH₂)₄(N₃C₂H)CH₂N(CH₂PO(OMe)₂)₂] **(49)**; *ck*-G0-[Si(CH₂)₄(N₃C₂H)CH₂N(CH₂PO(OMe)₂)₂]₄ n=0 and x=4 (**50**); *ck*-G1-[Si(CH₂)₄(N₃C₂H)CH₂N(CH₂PO(OMe)₂)₂]₈ n=1 and x=8 (**51**); *ck*-G2-[Si(CH₂)₄(N₃C₂H)CH₂N(CH₂PO(OMe)₂)₂]₁₆ n=2 and x=16 (**52)**), as colorless oils in high yield and can be stored for long periods of time without showing signs of decomposition (scheme 8). The derivatives **49-52** can be converted into anionic carbosilane dendrimers with terminal phosphonate groups *ck*-Gn-[Si(CH₂)₄(N₃C₂H)CH₂N(CH₂PO(ONa)₂)₂]ₓ (triehylsilyl Et₃-[Si(CH₂)₄(N₃C₂H)CH₂N(CH₂PO(ONa)₂)₂] **(53);** *ck*-G0-[Si(CH₂)₄(N₃C₂H)CH₂N(CH₂PO(ONa)₂)₂]₄ n=0 and x=4 **(54)**; *ck-*G1-[Si(CH₂)₄(N₃C₂H)CH₂N(CH₂PO(ONa)₂)₂]₈ n=1 and x=8 **(55);** *ck*-G2-[Si(CH₂)₄(N₃C₂H)CH₂N(CH₂PO(ONa)₂)₂]₁₆ n=2 and x=16 **(56))** (Scheme 8), by treatment with NaOH. These dendrimers are isolated as white solids soluble in water and can be stored for long periods of time without showing signs of decomposition.

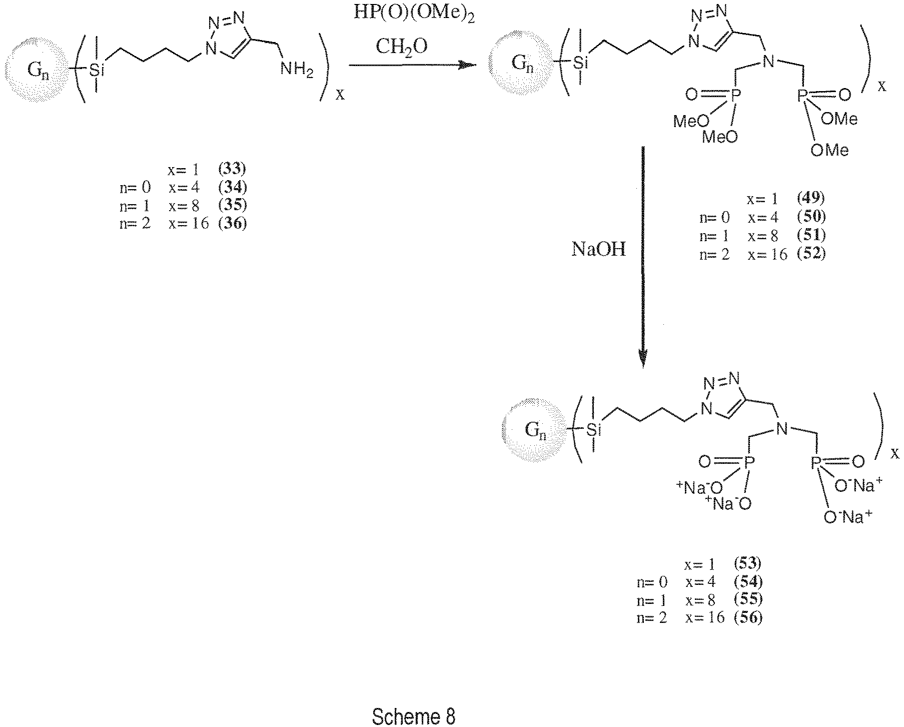

### METHOD 2 - Functionalization of organic amines with anionic groups and subsequent anchoring to a carbosilane dendrimer.

To proceed to the synthesis of these compounds, it was initially carried out the functionalization of a series of organic amines, aliphatic and aromatic, with anionic groups or precursors of anionic groups. These amines also have other functional group that will allow the binding to a carbosilane dendrimer through their terminal groups.

Allyl derivates with carboxylate (A), sulfonate (B) phosphonate (C) groups were synthesized and could be used for attachment to a carbosilane scalfold by hydrosilylation reaction with dendrimers containing terminal Si-H bonds, the Gn-(SiH)ₓ (triethyl silane, G0, G1, G2, with x = 1, 4, 8 and 16, respectively).

Another family of compounds synthesized are those derived from the functionalization of propargyl amine with carboxylate (D), sulfonate (E) or phosphonate (F) groups. In this case, the presence of a carbon-carbon triple bond in these derivatives allows binding to carbosilane dendrimers with terminal azide groups, Gn-[Si-CH₂CH₂CH₂CH₂N₃]ₓ, by a coupling reaction between the propargyl derivative and the azide group of the dendrimer.

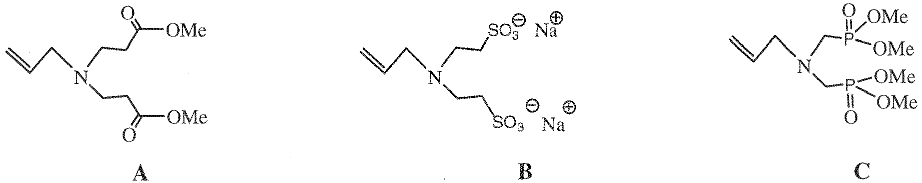

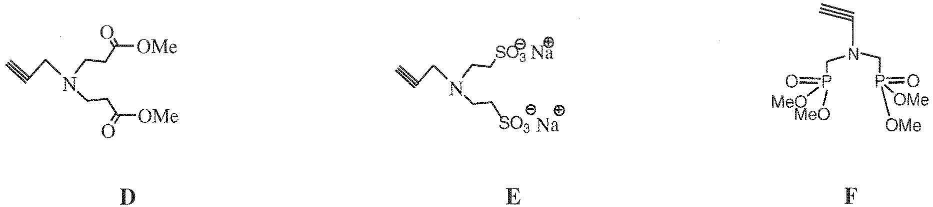

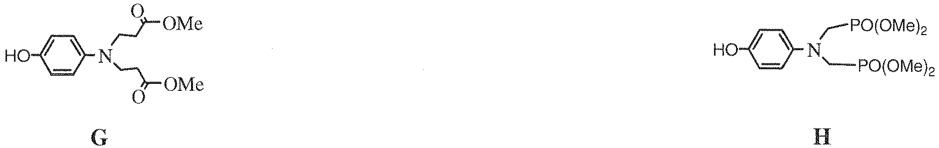

A third type of compounds prepared are those obtained from the group-NH₂ of 4-amino phenol derivatives with carboxylate (G) or phosphonate (H) groups. In this case, the binding of these compounds occurs by phenolisis reaction to carbosilane dendrimers containing Si-Cl or SiCH₂-Cl terminal bonds (Scheme 11).

### Method 2 (a). Functionalization of allylamine.

The synthesis of compounds A and B, has been carry out by a Michael addition type reaction of allylamine with methyl acrylate, and sodium vinyl sulfonate, respectively. In the synthesis of the derivate A, the Michael addition is completed in THF at room temperature and in just 3 hours. The derivative B, is prepared by Michael addition of sodium vinyl sulfonate to allylamine. In this case the reaction is carried out in H₂O as solvent, at 120 °C for 48 hours. In the case of the derivative C, the preparation is carried out in a two-step reaction, starting from allylamine. Initially allylamine is reacted with formaldehyde in THF for 1 hour at room temperature and then dimethyl phosphite is added *"in situ"* and the mixture is heated at 40 °C for 3 hours, to obtain the derived C as a colorless oil.

After obtaining the A-C allyl derivatives, the hydrosilylation reaction of these derivatives with carbosilane dendrimers grown from a tetrallylsilane core with terminal Si-H bonds of generations 0 to 2, Gn-(SiH)x, (triethylsilane, G0, G1, G2, x = 1, 4, 8 and 16, respectively), leads to the synthesis of carbosilane dendrimers functionalized with methyl acrylate groups, **5-8**, or sodium sulfonate, **17-20**, that have been already described in the previous section (see Scheme 9).

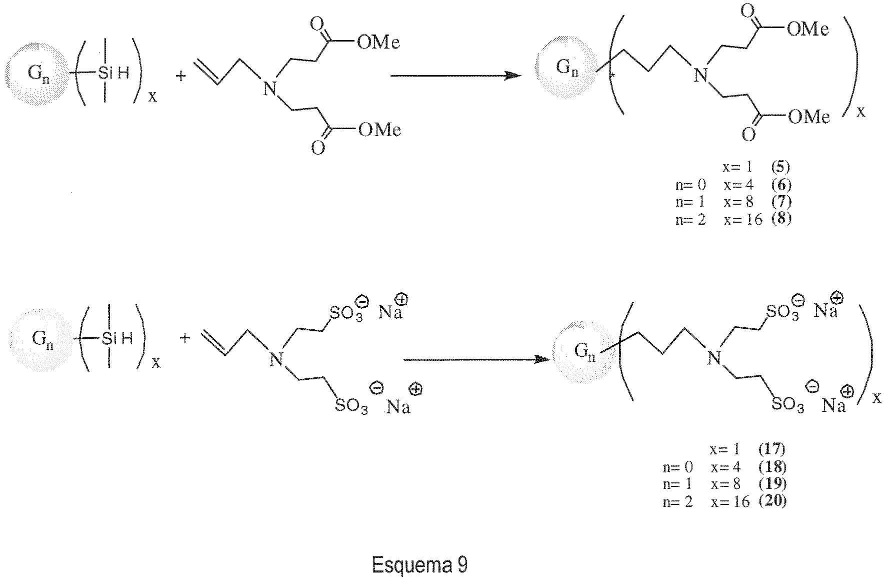

The derivates **5-8** can be converted into the corresponding anionic dendrimers with carboxylate groups, **9-12**, by treatment with NaOH solution (see Scheme 2).

### Method 2 (b). Functionalization of propargyl amino.

The synthesis of compounds **D** and **E** was carried out by a Michael type addition reaction of propargylamine to methyl acrylate and sodium vinyl sulfonate, respectively. In the synthesis of the derivative **D**, the Michael addition is completed in THF at room temperature and in just 3 hours. The derivative **E** is prepared by Michael addition of sodium vinyl sulfonate on propargylamine. In this case the reaction is carried out in H₂O as solvent, at 120 °C for 48 hours. In the case of **F,** the synthesis was carried out in two steps from propargylamine. Propargylamine initially reacted with formaldehyde in THF for 1 hour at room temperature and dimethyl phosphite is added *"in situ"* and the mixture is heated at 40 °C for 3 hours, to obtain derivate **F** as a colorless oil.

After obtaining compounds **D**-**F**, their click coupling to dendrimers containing a tetrallylsilane core and azide terminal groups of generations 0 to 2, Gn-[Si-CH₂CH₂CH₂CH₂N_{3]x}, (triethylsilyl Et₃-[Si-CH₂CH₂CH₂CH₂N₃] **(29);** G0-[Si-CH₂CH₂CH₂CH₂N₃]₄ n=0 and x=4 **(30);** G1-[Si-CH₂CH₂CH₂CH₂N₃]₈ n=1 and x=8 (31); G2-[Si-CH₂CH₂CH₂CH₂N₃]₁₆ n=2, x=16 **(32))** leads to the synthesis of carbosilane dendrimers functionalized with ester groups, **37-40,** or sodium sulfonate, **45-48,** or methyl phosphonate groups *ck*-Gn-[Si(CH₂)₄(N₃C₂H)CH₂N(CH₂PO(OMe)₂)₂]ₓ (triethylsilyl Et₃-[Si(CH₂)₄(N₃C₂H)CH₂N(CH₂PO(OMe)₂)₂] **(49);** *ck*-G0-[Si(CH₂)₄(N₃C₂H)CH₂N(CH₂PO(OMe)₂)₂]₄ n=0 and x=4 **(50)**; *ck-*G1-[Si(CH₂)₄(N₃C₂H)CH₂N(CH₂PO(OMe)₂)₂]₈ n=1 and x=8 **(51)**; *ck*-G2-[Si(CH₂)₄(N₃C₂H)CH₂N(CH₂PO(OMe)₂)₂]₁₆ n =2 and x =16 **(52)).** (Scheme 10) 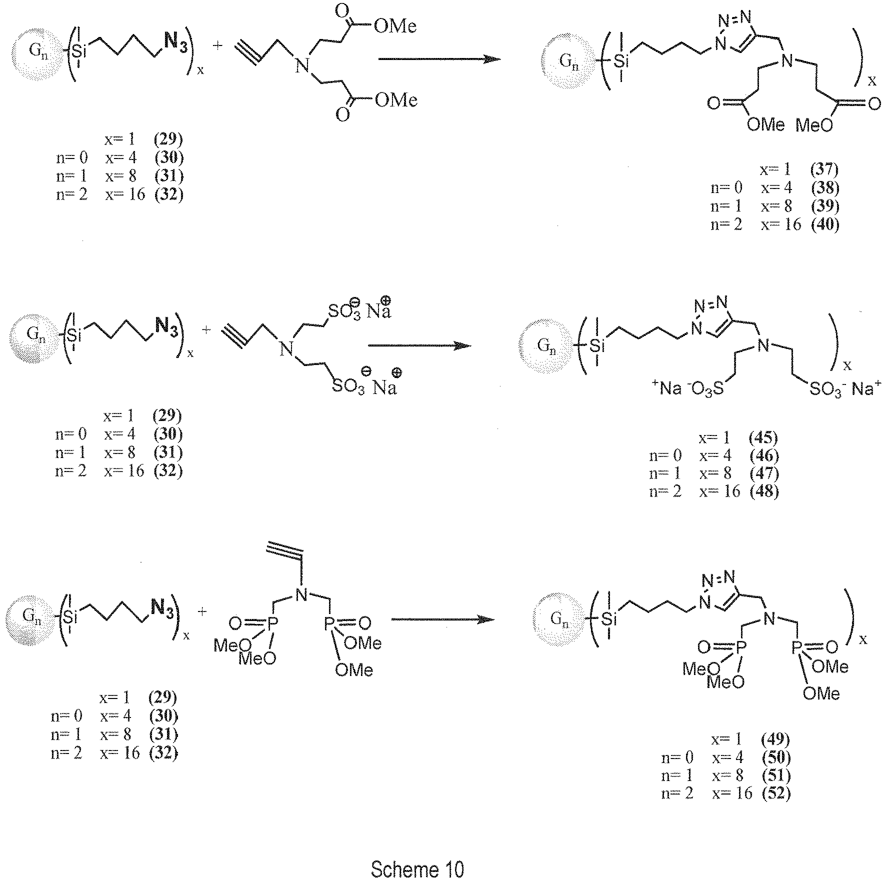 These dendrimers with carboxylate, sulfonate or phosphonate groups have been described previously in this invention.

### Method 2 (c) Functionalization of 4-aminophenol.

The synthesis of compound **G** was carried out by a Michael type addition reaction of methyl acrylate on 4-amino phenol. This derivative is described in the literature. The derivative **H** is prepared in two steps from 4-aminophenol. Initially 4-aminophenol reacted with formaldehyde in THF for 1 hour at room temperature and then dimethyl phosphonate is added "in situ" and the mixture is heated at 40 °C for 3 hours, to obtain the compound **H** as a colorless oil.
After obtaining the derivatives **G** and **H,** the phenolisis reaction of these derivatives with carbosilane dendrimers grown from a tetraallylsilane core with terminal Si-Cl or Si-CH₂Cl groups, of generations 0 to 2, gave the synthesis of carbosilane dendrimers functionalized with methyl acrylate groups, Gn-[SiCH₂-OC₆H₄-N(CH₂CH₂COOMe)₂]ₓ (triethylsilyl Et₃-[SiCH₂-OC₆H₄-N(CH₂CH₂COOMe)₂] **(57);** G0-[SiCH₂-OC₆H₄-N(CH₂CH₂COOMe)₂]₄ n=0 and x=4 **(58);** G1-[SiCH₂-OC₆H₄-N(CH₂CH₂COOMe)₂]₈ n=1 and x=8 **(59);** G2-[SiCH₂-OC₆H₄-N(CH₂CH₂COOMe)₂]₁₆ n=2 and x=16 **(60)),** or methyl phosponate groups Gn-[SiCH₂-OC₆H₄-N(CH₂PO(OMe)₂)₂]ₓ (triethylsilyl Et₃-[SiCH₂-OC₆H₄-N(CH₂PO(OMe)₂)₂] **(61);** G0-[SiCH₂-OC₆H₄-N(CH₂PO(OMe)₂)₂]₄ n =0 and x = 4 **(62);** G1-[SiCH₂-OC₆H₄-N(CH₂PO(OMe)₂)₂]₈ n =1 and x = 8 **(63);** G2-[SiCH₂-OC₆H₄-N(CH₂PO(OMe)₂)₂]₁₆ n =2 and x = 16 **(64)**), Scheme 11. The compounds **57-64** were obtained as colorless oils soluble in most common organic solvents but insoluble in water.

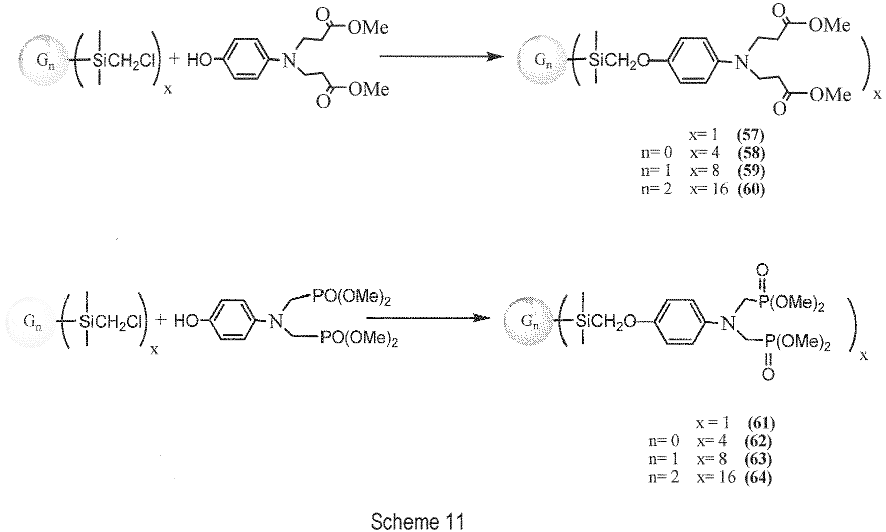

### EXAMPLE 1: Synthesis of the invention dendrimers grown from a tetraallylsilane core.

The synthesis was carried out following two methods listed below:

### METHOD 1: Synthesis of carbosilane dendrimers from a tetrallylsilane core with terminal amino groups,

The synthesis of carbosilane dendrimers with terminal amino groups was carried out by two strategies: 1) hydrosilylation of allyl amines to different generation of carbosilane dendrimers containing terminal Si-H bonds, or 2) using the click chemistry methodology to bind propargylamine to different generation carbosilane dendrimers containing terminal azides.
Some examples describing the synthesis of these compounds are listed below.

### SYNTHESIS AND CHARACTERIZATION OF CARBOSILANE DENDRIMERS.

Structural characterization of dendrimers has been carried out using elemental analysis, ¹H, ¹³C, ²⁹Si, ³¹P and ¹⁵N- RMN spectroscopies and mass spectrometry. The spectroscopic and analytical data were consistent with proposed structures for the derivatives.

### Example 1.2: Synthesis of G₁-[Si(CH₂)₃N(CH₂CH₂COOMe)₂]₄ (6)

### Method 1.

Over a solution of G₁-[(CH₂)₃NH₂]₄ (0.88 g, 1.34 mmol) in MeOH, prepared according to literature references (S.W. Krska, D. Seyferth, J. Am. Chem. Soc., 1998, 120, 3604-3612), was added methylacrilate CH₂=CHCOOMe (1.0 ml, 11.1 mmol). The reaction mixture was stirred at room temperature during 12 h. Afterward, volatiles were removed under vacuum and compound **6** (1.79 g, 99%) was obtained.

### Method 2

CH₂=CHCH₂N(CH₂CH₂COOMe)₂ (0.5 g, 2.18 mmol) and one drop of Karsted [(3-3.5% Pt) in poly(dimethylsiloxane)] catalyst were added to a solution of first-generation hydrogen-terminated dendrimer G₁-[Si-H]₄ (0.24 g, 0.05 mmol) in Toluene. The reaction mixture was stirred at room temperature during 12 h. Afterward, volatiles were removed under vacuum and compound 6 (0.58 g, 80%) was obtained.

¹H-NMR (CDCl₃) δ 3.64 (s, 24H, -OMe), 2.75 (t, 16H, -NC*H*₂CH₂COOMe), 2.42 (m, 16H, -NGH₂G*H*₂GOOMe and -SiCH₂CH₂CH₂N-), 1.32 (m, 16H, SiCH₂CH₂CH₂Si- and -SiCH₂CH₂CH₂N-), 0.54 (m, 24H, SiC*H*₂CH₂C*H*₂Si- and -SiC*H*₂CH₂CH₂N-), 0.37 (m, 8H, SiC*H*₂CH₂CH₂Si-), -0.06 (s, 24H, -SiMe₂). ¹³C {¹H}-NMR (CDCl₃): δ 173.0 (COOMe), 57.5 (SiCH₂CH₂CH₂N-), 51.5 (-OMe), 49.3 (-N*C*H₂CH₂COOMe), 32.4 (-NCH₂*C*H₂COOMe), 21.6 (-SiCH₂CH₂CH₂N-), 20.3, 18.5, 18.0 (Si(CH₂)₃Si-), 13.0 (-Si*C*H₂CH₂CH₂N-), -3.3 (-SiMe₂) . ²⁹Si(¹H)-NMR (CDCl₃): δ 0.8 (G₀-Si), 1.9 (G₁-Si). Anal. Calcd. C₆₄H₁₂₈N₄O₁₆Si₅: %: C, 56.93; H, 9.56; N, 4.15; Exp. %: C 57.09; H, 10.06; N, 4.34. GPC: PDI exp = 1.04.

### Example 1.2: Synthesis of G₂-[Si(CH₂)₃N(CH₂CH₂COOMe)₂]₈ (7)

This second-generation dendrimer was prepared using a similar method to that described for 6, starting from G₂-[(CH₂)₃NH₂]₈ (0.65 g, 0.39 mmol) dissolved in methanol and excess of methylacrilate CH₂=CHCOOMe (1 ml. 11.1 mmol). Compound 7 was isolated as colorless oil (1.09 g, 92%).

¹H-NMR (CDCl₃): δ 3.64 (s, 48H, -OMe), 2.75 (m, 32H, -NC*H*₂CH₂COOMe), 2.42 (m, 48H, - NCH₂C*H*₂COOMe and -SiCH₂CH₂C*H*₂N-), 1.31 (m, 40H, SiCH₂C*H*₂CH₂Si- and -SiCH₂C*H*₂CH₂N-), 0.53 (m, 48H, SiC*H*₂CH₂C*H*₂Si- and -SiC*H*₂CH₂CH₂Si-), 0.38 (m, 16H, SiC*H*₂CH₂CH₂N-), -0.06 (s, 48H, -SiMe₂), -0.1 (s, 12H, - SiMe). ¹³C {1H}-NMR (GDGl₃}: δ 173.0 (-*C*OOMe), 57.5 ((-SiCH₂CH₂CH₂N--), 51.50Me), 49.2 (-NCH₂CH₂COOMe), 32.5 (-NCH₂*C*H₂COOMe), 21.5 (-SiCH₂*C*H₂CH₂N-), 20.1 - 17.7 (Si(CH₂)₃Si-), 12.8 (-Si*C*H₂CH₂CH₂N-), -3.3 (-SiMe₂) - 5.0 (-SiMe). ²⁹Si{¹H}-NMR (CDClₛ): δ (G₀-Si not observed), 0.78 (G₁-Si), 1.88 (G₂-Si). Anal. Calcd. C₁₄₄H₂₉₂N₈O₃₂Si₁₃: %: C, 57.40; H, 9.77; N, 3.72; Exp. %: C 56.76; H, 9.78; N, 3.88. GPC: PDI exp =1.3

### Example 1.3: Synthesis of G₃₋[Si(CH₂)₃N(CH₂CH₂COOMe)₂]₁₆ (8)

This third-generation dendrimer was prepared using a similar method to that described for first and second generation dendrimers, starting from G₃-[(CH₂)₃NH₂]₁₆ (0.25 g, 0.06 mmol) dissolved in methanol and excess of methylacrilate CH₂=CHCOOMe (1 ml. 11.1 mmol). Compound 8 was isolated as colorless oil (0.39 g, 99%).

¹H-NMR (GDCl₃}: δ 3.63 (s, 96H, -OMe), 2.74 (m, 64H, -NC*H*₂CH₂COOMe), 2.41 (m, 96H, - NCH₂C*H*₂COOMe and -SiCH₂CH₂C*H*₂N-), 1.31 (m, 88H, SiCH₂C*H*₂CH₂Si- and -SiCH₂C*H*₂CH₂N-), 0.53 (m, 112H, SiC*H*₂CH₂C*H*₂Si- and -SiC*H*₂CH₂CH₂Si-), 0.37 (m, 32H, SiC*H*₂CH₂CH₂N-), -0.07 (s, 96H, -SiMe₂), -0.1 (s, 36H, - SiMe). ¹³C {¹H}-NMR (CDCl₃): 5 173.0 (-COOMe), 57.5 (-SiCH₂CH₂CH₂N-), 51.5 (-OMe), 49.2 (-NCH₂CH₂COOMe), 32.5 (-NCH₂*C*H₂COOMe), 21.5 (-SiGH₂*C*H₂GH₂N-), 20.1 -17.9 (Si(CH₂)₃Si-), 12.8 (-Si*C*H₂CH₂CH₂N-), -3.3 (-SiMe₂) - 5.0 (-SiMe). ²⁹Si{¹H}-NMR (CDCl₃): δ (G₀-Si and G₁-Si not observed), 0.8 (G₂-Si), 1.9 (G₃-Si). Anal. Calcd. G₃₀₄H₆₂₀N₁₆O₆₄Si₂₉. %: C, 57.60; H, 9.86; N, 3.54; Exp. %: C 58.38; H, 9.86; N, 3.08. GPC: PDI exp = 1.3

### Example 1.4: Synthesis of G₁-[Si(CH₂)₃N(CH₂CH₂COONa)₂]₄ (10)

A solution of NaOH (pH=10) was added to an ethanol solution of dendrimer 6 (1.80 g, 1.34 mmol). The resulting solution was stirred for 2h at room temperature and then evaporated under reduced pressure to remove residual solvent. The residue was washed with Et₂O and dried under vacuum to give 10 as a white solid (1,86 g, 98%) soluble in water.

¹H-NMR (D₂O): δ 2.57 (m, 8H, -NC*H*₂CH₂COO⁻Na⁺), 2.72 (m, 8H, -SiCH₂CH₂C*H*₂N-), 2.16 (m, 16H, - NCH₂C*H*₂COO⁻Na⁺), 1.25 (m, 16H, -SiCH₂C*H*₂CH₂N-, SiCH₂C*H*₂CH₂Si-), 0.43 (m, 8H, SiC*H*₂CH₂C*H*₂Si-), 0.27 (t, 8H, SiC*H*₂CH₂CH₂N), -0.20 (s, 24H, -SiMe₂). ¹³C {¹H}-NMR (D₂O): δ 179.9 (-NCH₂CH₂COO⁻Na⁺), 55.3 (-SiCH₂CH₂CH₂N-), 48.6 (-N*C*H₂CH₂COO⁻Na⁺), 32.7 (-NCH₂*C*H₂COO⁻Na⁺), 19.0 (-SiCH₂*C*H₂CH₂N-), 18.4, 17.5 and 15.9 (Si(CH₂)₃Si), 11.4 (-SiCH₂CH₂CH₂N-), -4.8 (-SiMe₂) ²⁹Si{¹H}-NMR (D₂O): δ 1.1 (G₀-Si), 1.8 (G₁-Si). ¹⁵N{¹H}-NMR (D₂O): δ -322.6. Anal. Calcd. C₅₆H₁₀₄N₄Na₈O₁₆Si₅: %: C, 47.57; H, 7.41; N, 3.96; Exp. %: C 46,15; H, 7.53; N, 3.62.

### Example 1.5: Synthesis of G₂-[Si(CH₂)₃N(CH₂CH₂COONa)₂]₈_(11)

This second-generation dendrimer was prepared using a similar method to that described for 10 starting from second-generation dendrimer 7 (1.05 g, 0.35 mmol) in ethanol and a solution of NaOH pH=10. The resulting solution was stirred for 2h at room temperature and then evaporated under reduced pressure to remove residual solvent. The residue was washed with Et₂O and dried under vacuum to give 11 as a white solid (1.06 g, 97%) soluble in water.

¹H-NMR (D₂O): δ 2.61 (m, 32H, -NC*H*₂CH₂COO⁻Na⁺), 2.31 (m, 16H, -SiCH₂CH₂C*H*₂N-), 2.17 (m, 32H, - NCH₂C*H*₂COO⁻Na⁺), 1.31 (m, 16H, -SiCH₂C*H*₂CH₂N-), 1.20 (m, 24H, SiCH₂C*H*₂CH₂Si-), 0.42 (m, 48H, SiC*H*₂CH₂C*H*₂Si-), 0.26 (m, 16H, -SiC*H*₂CH₂CH₂N-), -0.17 (s broad, 48H, -SiMe₂), -0,21 (s broad, 12H, -SiMe). ¹³C {¹H}-NMR (D₂O): δ 180.5 (-NCH₂CH₂COO⁻Na⁺), 56.8 (-SiCH₂CH₂*C*H₂N-), 49.7 (-NCH₂CH₂COO⁻Na⁺), 33.7 (-NCH₂*C*H₂COO⁻Na⁺), 19.9 (-SiCH₂*C*H₂CH₂N-), 19.3 - 17.9 (Si(CH₂)₃Si), 12.7 (-Si*C*H₂CH₂CH₂N-), -3.4 (-SiMe₂), -4.4 ( -SiMe). ²⁹Si{¹H}-NMR (D₂O): δ (G₀-Si not observed), 1.2 (G₁-Si), 1.9 (G₂-Si). ¹⁵N{¹H}-NMR (D₂O): δ -329. Anal. Calcd. C₁₂₈H₂₄₄N₈Na₁₆O₃₂Si₁₃: %: C, 48.96; H, 7.83; N, 3.57; Exp. %: C, 49.03; H, 7.79; N, 3.31.

### Example 1.6: Synthesis of G₃[Si(CH₂)₃N(CH₂CH₂COONa)₂]₁₆ (12)

This third-generation dendrimer was prepared using a similar method to that described for first and second generation dendrimers, starting from dendrimer 8 (0.39 g, 0.07 mmol) dissolved in ethanol and excess of NaOH solution (pH=10). The resulting solution was stirred for 2h at room temperature and then evaporated under reduced pressure to remove residual solvent. The residue was washed with Et₂O and dried under vacuum to give 12 as a white solid (1.07 g, 95%) soluble in water.

¹H-NMR (D₂O): δ 2.54 (m, 64H, -NC*H*₂CH₂COO⁻Na⁺), 2.22 (m, 32H, -SiCH₂CH₂C*H*₂N-), 2.14 (m, 64H, - NCH₂C*H*₂COO⁻Na⁺), 1.20 (m, 88H, -SiCH₂C*H*₂CH₂N-, SiCH₂C*H*₂CH₂Si-), 0.42 (m, 112H, Si*CH*₂CH₂C*H*₂Si-), 0.25 (m, 32H, -SiC*H*₂CH₂CH₂N-), -0.17 (s broad, 96H, -SiMe₂), -0,25 (s broad, 36H, -SiMe). ¹³C {¹H}-NMR (D₂O): δ 181.2 (-NCH₂CH₂COO⁻Na⁺), 57.1 (-SiCH₂CH₂CH₂N-), 49.6 (-NCH₂CH₂COO⁻Na⁺), 34.3 (-NCH₂CH₂COO⁻Na⁺), 20.3 (-SiCH₂CH₂CH₂N-), 19.5-18.0 (Si(CH₂)₃Si), 13.0 (-SiCH₂CH₂CH₂N), -3.4(-SiMe₂), -4.5 (-SiMe). ²⁹Si{¹H}-NMR (D₂O): δ (G₀-Si and G₁-Si not observed), 1.1 (G₂-Si), 2.0 (G₃-Si).

### Example 1.7: Synthesis of G₁-[Si(CH₂)₃N⁺H(CH₂CH₂COOH)₂]₄ 4 Cl^{·}(14)

Over solution of dendrimer **10** (1.36 g, 1.01 mmol) in methanol was added an excess of HCl solution at 37% (2.36 mmol). The resulting solution was stirred for 3h at room temperature and then evaporated under reduced pressure to remove residual solvent heating at 50 °C. The residue was washed with fresh Et₂O (2 x 25ml) and dried under vacuum to give **14** as a white solid (1.17 g, 84%).

¹H-NMR (D₂O): δ 3.30 (m, 16H, -N⁺HC*H*₂CH₂COOH), 2.99 (m, 8H, -SiCH₂CH₂C*H*₂N⁺H-), 2.70 (m, 16H, - N+HCH₂C*H*₂COOH), 1.57 (m, 8H, -SiCH₂C*H*₂CH₂N⁺H-), 1,21 (m, 8H, SiCH₂C*H*₂CH₂Si-), 0.44 (m, 24H, SiC*H*₂CH₂C*H*₂Si- and -SiC*H*₂CH₂CH₂N⁺H-), -0.15 (s broad, 6H, -SiMe₂). ¹³C {¹H}-NMR (D₂O): δ 174.0 (-COOH), 55.2 (-SiCH₂CH₂CH₂N⁺H-), 49.1 (-N⁺HCH₂CH₂COOH), 28.4 (-N+HCH₂CH₂COOH), 19.6, 18.5, 18.2 (Si(CH₂)₃Si), 17.2 (-SiCH₂CH₂CH₂N⁺H-), 11.5 (-SiCH₂CH₂CH₂N⁺H-), -3.6 (-SiMe₂). ¹⁵N{¹H}-NMR (D₂O): δ -325 (-N⁺H(CH₂CH₂COOH)₂). ²⁹Si{¹H}-NMR (D₂O): δ (G₀-Si not observed), 2.2 (G₁-Si). Anal, Calcd. C₅₆H₁₁₆Cl₄N₄O₁₆Si₅: %: C, 48.61; H, 8.45; N, 4.05; Exp. C, 48.78; H, 8.41; N, 4.00.

### Example 1.8: Synthesis of G₂-[Si(CH₂)₃N+H(CH₂CH₂COOH)₂]₈ 8 Cl-(15)

This second-generation dendrimer was prepared using a similar method to that described for **14** starting from second-generation dendrimer **11** (1.0 g, 0.33 mmol) in methanol and excess of HCl solution at 37% (2.65 mmol) to obtain the dendrimer **15** as a white solid soluble in water (0.98 g, 96%).

¹H-NMR D₂O): δ 3.30 (m, 32H, -N⁺HC*H*₂CH₂COOH), 3.01 (m, 16H, -SiCH₂CH₂CH₂N⁺H-), 2.74 (m, 32H, - N⁺HCH₂CH₂COOH), 1.57 (m, 16H, -SiCH₂C*H*₂CH₂N⁺H-), 1.20 (m, 24H, SiCH₂C*H*₂CH₂Si- and -SiCH₂C*H*₂CH₂Si-), 0.42 (m, 64H, SiC*H*₂CH₂C*H*₂Si- and -SiC*H*₂CH₂CH₂N⁺H-), -0.14 (s broad, 60H, -SiMe₂ and -SiMe). ¹³C{¹H}-NMR (D₂O): δ 173.8 (-COOH), 55.3 (-SiCH₂CH₂CH₂N⁺H-), 49.1 (-N⁺H*C*H₂CH₂COOH), 28.4 (-N⁺HCH₂*C*H₂COOH), 19.6-17.0 (Si(CH₂)₃Si- and -SiCH₂*C*H₂CH₂N⁺H-), 11.6 (-Si*C*H₂CH₂CH₂N⁺H-)= -3.5 (-SiMe₂ and -SiMe). ²⁹Si {¹H)-NMR (D₂O): δ (G₀-Si not observed), 1.1 (G₁-Si). 2.2 (G₂-Si). Anal. Calcd. C₁₂₈H₂₆₈Cl₈N₈O₃₂Si₁₃: %: C, 49.91; H, 8.77; N, 3.64; Exp. %: C, 50.64; H, 8.61; N, 3.45.

### Example 1.9: Synthesis of G₃-[Si(CH₂)₃N⁺H(CH₂CH₂COOH)₂]₁₆ 16 Cl^{.} (16)

This third generation dendrimer was prepared using a similar method to that described for first and second generation dendrimers **14** and **15,** starting from a solution of dendrimer **12** (1.0 g, 0.17 mmol) in MeOH and excess of a HCl solution at 37% (2.52 mmol) to obtain the dendrimer **16** as a white solid soluble in water (0.8 g, 86%).

¹H-NMR (D₂O): δ 3.29 (m, 64H, -N⁺HC*H*₂CH₂COOH), 3.0 (m, 32H, -SiCH₂CH₂C*H*₂N⁺H-), 2.71 (m, 32H, - N⁺HCH₂C*H*₂COOH), 1.57 (m, 32H, -SiCH₂C*H*₂CH₂N⁺H-), 1.19 (m, 56H, SiCH₂C*H*₂CH₂Si- and -SiCH₂C*H*₂CH₂Si-), 0.40 (m, 144H, SiC*H*₂CH₂C*H*₂Si- and -SiC*H*₂CH₂CH₂N⁺H-), -0.14 (s broad, 132H, -SiMe₂ and -SiMe). ¹³C{¹H}-NMR (D₂O): δ 174.6 (-COOH), 55.8 (-SiCH₂CH₂*C*H₂N⁺H-), 49.4 (-N⁺H*C*H₂CH₂COOH), 28.9 (-N⁺HCH₂*C*H₂COOH), 19.8-17.2 (Si(CH₂)₃Si- and -SiCH₂CH₂CH₂N⁺H-), 11.6 (-SiCH₂CH₂CH₂N+H-), -3.5 (-SiMe₂), -4.4 (-SiMe). ²⁹Si {¹H}-NMR (D₂O): δ (G₀-Si, G₁-Si and G₂-Si not observed), 2.5 (G₃-Si). Anal. Calcd. C₂₇₂H₅₇₂Cl₁₆N₁₆O₆₄Si₂₉: %: C, 50.47; H, 8.91; N, 3.46; Exp. %: C, 50.99; H, 8.61; N, 2.99.

### Example 1.10: Synthesis of G₁-[Si(CH₂)₃N(CH₂CH₂SO₃Na)₂]₄ (18)

A solution of sodium vinylsulfonate, CH₂=CHSO₃Na ∼30% wt in H₂O (5.16 ml, 14.0 mmol) was added to a solution of G₁-[CH₂CH₂CH₂NH₂]₄ (1.16 g, 1.75 mmol) in ethanol. The resulting solution was stirred for 3 days at 120 °C. Then the solution was filtered and the residue was washed with MeOH and dried under vacuum to give 18 as a white solid (2.25 g, 73%).

¹H-NMR (D₂O): δ 2.86 (m, 16H, -NCH₂C*H*₂SO₃⁻Na⁺), 2.77 (m, 16H, -NC*H*₂CH₂SO₃⁻Na⁺), 2.27 (m, 8H, - SiCH₂CH₂C*H*₂N-), 1.39 (m, 8H, -SiCH₂C*H*₂CH₂N-), 1.21 (m, 8H, SiCH₂C*H*₂CH₂Si-) 0.42 (m, 9H, SiC*H*₂CH₂C*H*₂Si- and -SiC*H*₂CH₂CH₂N-), -0.20 (s, 24H, -SiMe₂). ¹³C{¹H}-NMR (D₂O): δ 56.5 (-SiCH₂CH₂*C*H₂N-), 47.8 (-NCH₂*C*H₂SO₃⁻Na⁺), 47.0 (-N*C*H₂CH₂SO₃⁻Na⁺), 20.4 (SiCH₂*C*H₂CH₂Si-), 19.4, 16.9 (-Si*C*H₂CH₂CH₂N- and SiCH₂CH₂CH₂Si-), 18.5 (-SiCH₂CH₂CH₂N-), 12.3 (Si*C*H₂CH₂CH₂Si-), -3.8 (-SiMe₂). ¹⁵N{¹H}-NMR (D₂O): δ -339.1 (-NCH₂CH₂SO₃⁻Na⁺). ²⁹Si{¹H}-NMR (D₂O): δ (G₀-Si not observed), 1.8 (G₁-Si). Anal. Calcd. C₅₂H₁₁₆N₄Na₈O₂₄S₈Si₅: %: C, 35.44; H, 6.63; N, 3.18; S, 14.56; Exp. %: C, 34.54; H, 6.56; N, 3.02; S, 15.28.

### Example 1.11: Synthesis of G₂-[Si(CH₂)₃N(CH₂CH₂SO₃Na)₂]₈ (19)

A solution of sodium vinylsulfonate, CH₂=CHSO₃Na ∼30% wt in H₂O (1.8 ml, 4.91 mmol) was added to a solution of G₂-[CH₂CH₂CH₂NH₂]₈ (0.5 g, 0.31 mmol) in ethanol. The resulting solution was stirred for 3 days at 120 °C. Then the solution was filtered and the residue was washed with MeOH and dried under vacuum to give **19** as a white solid (0.91 g, 80%).

¹H-NMR (D₂O): δ 2.86 (m, 32H, -NCH₂C*H*₂SO₃⁻Na⁺), 2.76 (m, 32H, -NC*H*₂CH₂SO₃⁻Na⁺, 2.28 (m, 16H, - SiCH₂CH₂C*H*₂N-), 1.21 (m, 40H, -SiCH₂C*H*₂CH₂N-, -SiCH₂C*H*₂CH₂Si- and SiCH₂C*H*₂CH₂Si-), 0.43 (m, 48H, SiC*H*₂CH₂C*H*₂Si- and -SiC*H*₂CH₂C*H*₂Si-), 0.28 (m, 16H, -SiC*H*₂CH₂CH₂N-), -0.16 (s broad, 48H, -SiMe₂), -0.21 (s broad, 12H, -SiMe). ¹³C{¹H}-NMR(D₂O): δ 56.9 (-SiCH₂CH₂*C*H₂N-), 48.0 (-NCH₂*C*H₂SO₃⁻Na⁺), 46.7 (-N*C*H₂CH₂SO₃-Na⁺), 20.1 (-SiCH₂*C*H₂CH₂N-), 18.5-17.7 (Si(CH₂)₃Si), 12.4 (-Si*C*H₂CH₂CH₂N-), -3.5 (SiMe₂), -4.4 (SiMe). ¹⁵N{¹H}-NMR (D₂O): δ -348.5 (-NCH₂CH₂SO₃⁻Na⁺). ²⁹Si{¹H}-NMR (D₂O): δ (G₀-Si not observed), 1.1 (G₁-Si), 2.1 (G₂-Si). Anal. Calcd. C₁₁₂H₂₄₄N₈Na₁₆O₄₈S₁₆Si₁₃: %: C, 36.19; H, 6.62; N, 3.01; S, 13.80; Exp.. %: C, 36.06; H, 7.04; N, 2.63; S, 13.91.

### Example 1.12: Synthesis of G₃-[Si(CH₂)₃N(CH₂CH₂SO₃Na)₂]₁₆ (20)

A solution of Sodium vinylsulfonate, CH₂=CHSO₃Na ∼30% wt in H₂O (1.70 ml, 4.64 mmol) was added to a solution of G₃-[CH₂CH₂CH₂NH₂]₁₆ (0.52 g, 0.14 mmol) in ethanol. The resulting solution was stirred for 3 days at 120 °C. Then the solution was filtered and the residue was washed with MeOH and dried under vacuum to give **20** as a white solid (0.79 g, 73%).

¹H-NMR (D₂O): δ 2.85 (m, 64H, -NCH₂C*H*₂SO₃⁻Na⁺), 2.78 (m, 64H, -NC*H*₂CH₂SO₃⁻Na⁺), 2.27 (m, 32H, - SiCH₂CH₂C*H*₂N-), 1.24 (m, 88H, -SiCH₂C*H*₂CH₂N-, -SiCH₂C*H*₂CH₂Si- and SiCH₂C*H*₂CH₂Si-), 0.44 (m, 144H, SiC*H*₂CH₂C*H*₂Si-, -SiC*H*₂CH₂C*H*₂Si- and -SiC*H*₂CH₂CH₂N-), -0.15 (s broad, 132H, -SiMe₂ and -SiMe). ¹³C {¹H}-NMR (D₂O): δ 57.1 (-SiCH₂CH₂*C*H₂N-), 47.9 (-NCH₂CH₂SO₃⁻Na⁺), 47.1 (-N*C*H₂CH₂SO₃⁻Na⁺), 20.6 (-SiCH₂*C*H₂CH₂N-), 20.0-18.0 (Si(*C*H₂)₃Si), 12.7 (-Si*C*H₂CH₂CH₂Si-), -3.4 (-SiMe₂), -4.4 (-SiMe). ¹⁵N{¹H}-NMR (D₂O): δ -349.7. ²⁹Si{¹H}-NMR (D₂O): δ (G₀-Si and G₁-Si not observed), 1.2 (G₂-Si), 2.0 (G₃-Si). Anal. Calcd. C₂₂₄H₄₉₂N₁₆Na₃₂O₉₆S₃₂Si₂₉: %: C, 35.76; H, 6.59; N, 2.98; S, 13.64; Exp.. %: C, 36.93; H, 7.19; N, 2.58; S, 12.86.

### Example 1.13: Synthesis of G₁-[Si(CH₂)₃N⁺H(CH₂CH₂SO₃⁻Na⁺)]₄ 4 Cl⁻(22)

Over a water solution of dendrimer **18** (0.05 g, 0.03 mmol) was added an excess of HCl solution 1 M in Et₂O (0.5 ml, 0.23 mmol). The resulting solution was stirred at room temperature for 12h and evaporated under reduced pressure to remove residual solvent. The residue was washed with Et₂O (2 x 25 ml), and was purified by column chromatography of sephadex with H₂O as eluent. The dendrimer **22** was obtained as white solid soluble in water (0.04 g, 80%).

¹H-NMR (D₂O): δ 3.44 (m, 16H, -N⁺HCH₂C*H*₂SO₃⁻Na⁺), 3.16 (m, 24H, -N⁺HC*H*₂CH₂SO₃⁻Na⁺ and - SiCH₂CH₂C*H*₂N⁺H-), 1.56 (m, 16H, -SiCH₂C*H*₂CH₂N⁺H- and - SO₃⁻Na⁺), 1.21 (m, 8H, SiCH₂C*H*₂CH₂Si-) 0.42 (m, 9H, SiC*H*₂CH₂C*H*₂Si- and -SiC*H*₂CH₂CH₂N⁺H-), -0.16 (s broad, 24H, -SiMe₂), ¹³C{¹H}-NMR (D₂O): δ 56.5 (-SiCH₂CH₂CH₂N⁺H-), 49.4 (-N⁺HCH₂*C*H₂SO₃⁻Na⁺), 44.8 (-N⁺H*C*H₂CH₂SO₃⁻Na⁺), 19.6 (SiCH₂*C*H₂CH₂Si-), 18.5, 18.2 (-Si*C*H₂CH₂CH₂N⁺H- and SiCH₂CH₂*C*H₂Si-), 17.2 (-SiCH₂*C*H₂CH₂N⁺H-), 11.4 (Si*C*H₂CH₂CH₂Si-), -3.5 (-SiMe₂). ¹⁵N{¹H}-NMR (D₂O): δ-338.5 (N⁺HC*H*₂CH₂SO₃⁻Na⁺). ²⁹Si{¹H}-NMR (D₂O): δ (G₀-Si not observed), 2.3 (G₁-Si). Anal. Calcd. C₄₆H₁₁₂Cl₄N₄O₂₄S₈Si₅. %: C, 33.60; H, 6.87; N, 3.41; S, 15.60; Exp. %: C, 32.52; H, 5.87; N, 3.72; S, 14.58.

### Example 1.14: Synthesis of G₂-[Si(CH₂)₃N⁺H(CH₂CH₂SO₃⁻Na⁺)₂]₈ 8 Cl⁻ (23)

This second generation dendrimer was prepared using a similar method to that described for 22 starting from second generation dendrimer **19** (0.07 g, 0.02 mmol) in water and excess of HCl solution 1 M in Et₂O (0.5 ml, 0.3 mmol) to obtain the dendrimer **23** as a white solid soluble in water (0.04 g, 60%).

¹H-NMR (D₂O): δ 3.47 (m, 32H, -N⁺HCH₂CH₂SO₃⁻Na⁺), 3.17 (m, 48H, -N⁺HCH₂CH₂SO₃⁻Na⁺ and - SiCH₂CH₂CH₂N⁺H⁻), 1.58 (m, 32H, -SiCH₂CH₂CH₂N⁺H- and - SO₃⁻Na⁺), 1.23 (m, 24H, -SiCH₂CH₂CH₂Si- and SiCH₂CH₂CH₂Si-), 0.46 (m, 64H, SiCH₂CH₂CH₂Si-, -SiCH₂CH₂CH₂Si- and ₋SiCH₂CH₂CH₂N⁺H-), -0.11 (s broad, 60H, -SiMe₂ and -SiMe). ¹³C{¹H}-NMR (Dz0): δ 55.6 (-SiCH₂CH₂CH₂N⁺H-), 48.4 (-N+HCH₂CH₂SO₃⁻Na⁺), 43.9 (-N⁺HCH₂CH₂S0₃⁻Na⁺), 20.5 - 17.1 (-SiCH₂CH₂CH₂N⁺H-, and Si(CH₂)₃Si), 10.6 {-SiCH₂CH₂CH₂N⁺H-), -3.3 (-SiMe₂ and -SiMe). ²⁹Si{¹H}-NMR (D₂O): δ (G₀-Si not observed), 0.7 (G₁-Si), 2.2 (G₂-Si). Anal. Calcd. C₁₁₂H₂₆₈Cl₈N₈O₄₈S₁₆Si₁₃. %: C, 36.78; H, 7.39; N, 3.06; S, 14.03; Exp. %: C, 35.93; H, 7.46; N, 3.43; S, 13.97.

### Example 1.15: Synthesis of G₃-[Si(CH₂)₃N⁺H(CH₂CH₂SO₃Na⁺)₂]₁₆ 16 Cl⁻ (24)

This third generation dendrimer was prepared using a similar method to that described for first and second generation dendrimers **22** and **23,** starting from a solution of dendrimer **20** (0.06 g, 0.008 mmol) in water and excess of HCl solution 1M in Et₂O (0.5 ml, 0.3 mmol) to obtain the dendrimer **24** as a white solid soluble in water (0.04 g, 60%).

¹H-NMR (D₂O): δ 3.47 (m, 64H, -N⁺HCH₂CH₂SO₃⁻Na⁺), 3.16 (m, 96H, -N⁺HCH₂CH₂SO₃⁻Na⁺ and - SiCH₂CH₂CH₂N⁺H-), 1.57 (m, 64H, -SiCH₂CH₂CH₂N⁺H- and -SO₃⁻Na⁺), 1.21(m, 56H, -SiCH₂CH₂CH₂Si- and SiCH₂CH₂CH₂Si-), 0.44 (m, 144H, SiCH₂CH₂CH₂Si-, -SiCH₂CH₂CH₂Si- and -SiCH₂CH₂CH₂N⁺H-), -0.11 (s broad, 132H, -SiMe₂ and -SiMe). ¹³C{¹H}-NMR (D₂O): δ 56.5 (-SiCH₂CH₂CH₂N⁺H-), 45.1 and 44.9 (N⁺HCH₂CH₂SO₃⁻Na⁺ and -N⁺HCH₂CH₂SO₃⁻Na⁺), 20.0-17.8 (-SiCH₂CH₂CH₂N⁺H and Si(CH₂)₃Si), 11.6 (SiCH₂CH₂CH₂Si), -3.3 (-SiMe₂), -4.4 (-SiMe). Anal. Calcd. C₂₂₄H₅₄₀Cl₁₆N₁₆O₉₆S₃₂Si₂₉. %: C, 36.34; H, 7.35; N, 3.03; S, 13.86; Exp. %: C, 35.87; H, 7.84; N, 3.34; S, 12.96.

### Example 1.16: Synthesis of G₁-[SiCH₂CH₂CH₂CH₂Br]₄ (26)

Over a solution of dendrimer G₁-[Si-H]₄ (3.52 mmol) dissolved in hexane (5 mL) was added 1.61 mL of 4-Br-1-butene (15.85 mmol) in hexane and five drops of Karstedt's catalyst and stirred during five days at 60° C. Afterward, volatiles were removed under vacuum, dichloromethane was added and the solution was filtered through active carbon. Removal of volatiles under vacuum gave 26 as a pale yellow liquid (2.32 g, 68%).

¹H-NMR (CDCl₃): δ 3.40 (8H, t, CH₂Br), 1.85 (8H, m, SiCH₂CH₂CH₂CH₂Br), 1.37 (16H, m, SiCH₂CH₂CH₂CH₂Br y SiCH₂CH₂CH₂Si), 0.51 (24H, m, CH₂ bonded to Si), -0.05 (24H, s, SiCH₃). ¹³C{¹H}-NMR (CDCl₃): δ 36.4 (CH₂Br), 33.7 (SiCH₂CH₂CH₂CH₂Br), 22.5(SiCH₂CH₂CH₂CH₂Br), 20.2 (SiCH₂), 18.6 (SiCH₂), 17.6 (SiCH₂), 14.5 (SiCH₂), -3.281 (SiCH₃). Anal. Calcd. C₃₆H₈₀Br₄Si₅. %: C, 44.44; H, 8.29; Exp. %: C, 44.24; H, 8.18.

### Example 1.17: Synthesis of G₂-[SiCH₂CH₂CH₂CH₂Br]₈ (27)

This second generation dendrimer was prepared using a similar method to that described for 26 starting from 0.38 g of G₂-[Si-H]₈ dissolved in hexane (10 mL), 0.28 mL of 4-Br-1-butene (2.7 mmol) and two drops of Karstedt's catalyst and stirred during 16h at 60° C. Afterward, volatiles were removed under vacuum, dichloromethane was added and the solution was filtered through active carbon. Removal of volatiles under vacuum gave 27 as a pale yellow liquid (0.61 g, 83%).

¹H-NMR (CDCl₃): δ □3.40 (16H, t, CH₂Br), 1.85 (16H, m, SiCH₂CH₂CH₂CH₂Br), 1.31 (40H, m, SiCH₂CH₂CH₂CH₂Br y SiCH₂GH₂CH₂Si), 0.53 (64H, m, CH₂ bonded to Si), -0.05 (48H, s, Si(CH₃)₂), -0.09 (12H, s, SiCH₃). ¹³C{¹H}-RMN (CDCl₃): δ 36.8 (CH₂Br), 34.0 (SiCH₂CH₂CH₂CH₂Br), 22.93 (SiCH₂CH₂), 20.5(SiCH₂CH₂), 19.6(SiCH₂CH₂), 19.3 (SiCH₂), 19.1 (SiCH₂), 18.9 (SiCH₂), 18.2 (SiCH₂), 14.9 (SiCH₂), -2.8 (SiCH₃), -4.5 (SiCH₃). Anal. Calcd. C₈₈H₁₉₆Br₈Si₁₃. %: C, 46.79; H, 8.75; Exp. %: C, 46.51; H, 8.75.

### Example 1.18: Synthesis of G3-[SiCH₂CH₂CH₂CH₂Br]₁₆ (28)

This third generation dendrimer was prepared using a similar method to that described for 26 starting from 0.81 g of G₃-[Si-H]₁₆ (0.31 mmol) dissolved in hexane (20 mL), 0.51 mL of 4-Br-1-butene (5.04 mmol) and two drops of Karstedt's catalyst and stirred during 16h at 60 °C. Afterward, volatiles were removed under vacuum, and dichloromethane was added and the solution was filtered through active carbon. Removal of volatiles under vacuum gave 28 as a pale yellow liquid (1.27 g, 86%).

¹H-NMR (CDCl₃): δ 3.40 (32H, t, CH₂Br), 1.85 (32H, m, SiCH₂CH₂CH₂CH₂Br), 1.30 (88H, m, SiCH₂CH₂CH₂CH₂Br y SiCH₂CH₂CH₂Si), 0.53 (144H, m, CH₂ bonded to Si), -0.05 (120H, s, Si(CH₃)₂ y SiCH₃), -0.09 (12H, s, SiCH₃). ¹³C {¹H}-NMR (CDCl₃): δ 36.4 (CH₂Br), 33.6 (SiCH₂CH₂CH₂CH₂Br), 22.5 (SiCH₂CH₂), 20.1 (SiCH₂CH₂), 19.0-14.5 (SiCH₂), -3.3 (SiCH₃), -4.8 (SiCH₃, both signals). Anal. Calcd. C₁₉₂H₄₂₈Br₁₆Si₂₉, %: C, 47.74; H, 8.93; Exp. %: C, 48.62; H, 9.00.

### Example 1.19: Synthesis of G₁-[SiCH₂CH₂CH₂CH₂N₃]₄ (30)

Over a solution of dendrimer 26 (0.52 mmol) in DMF was added 0.34 g of sodium azide (5.16 mmol) and a small amount of Kl and stirred during five days at 90 °C into a sealed ampoule for 3 days under vacuum. Afterward, volatiles were removed under vacuum and 10 mL of dichloromethane was added and extracted twice with water. The organic phase was separated and was dried over MgSO₄. The solution was filtered and the volatiles were removed under vacuum, yielding 30 as pale yellow oil (0.35 g, 81%).

¹H-NMR (CDCl₃): δ □3.24 (8H, t, CH₂N₃), 1.60 (8H, m, SiCH₂CH₂CH₂CH₂N₃), 1.35 (16H, m, SiCH₂CH₂CH₂CH₂N₃ and SiCH₂CH₂CH₂Si), 0.52 (24H, m, CH₂ bonded to Si), -0.06 (24H, s, Si(CH₃)₂). ¹³C{¹H}-NMR (CDCl₃): δ 51.1 (CH₂N₃), 32.6 (SiCH₂CH₂CH₂CH₂N₃), 21.1 (SiCH₂CH₂), 20.2 (SiCH₂CH₂), 18.6 (SiCH₂), 17.5 (SiCH₂), 15.0 (SiCH₂,-3.3 (SiCH₃).

### Example 1.20: Synthesis of G₂₋[SiCH₂CH₂CH₂CH₂N₃]₈ (31)

This second generation dendrimer was prepared using a similar method to that described for 30 starting from 27 (1.00 g, 0.44 mmol) dissolved in DMF (30 mL), 0.43 g of sodium azide (6.64 mmol) and a small amount of Kl. The reaction was stirred during five days at 90° C into a sealed ampoule for 16h under vacuum. Afterward, volatiles were removed under vacuum and 30mL of dichloromethane was added and extracted twice with water (2x15mL). The organic phase was separated and was dried over MgSO₄. The solution was filtered and the volatiles were removed under vacuum, to obtain 31 as a pale yellow oil (0.67 g, 78%).

¹H-NMR (CDCl₃): δ □3.24 (16H, t, CH₂N₃), 1.60 (16H, m, SiCH₂CH₂CH₂CH₂N₃), 1.29 (40H, m, SiCH₂CH₂CH₂CH₂N₃ y SiCH₂CH₂CH₂Si), 0.51 (64H, m, CH₂ bonded to Si), -0.06 (48H, s, Si(CH₃)₂), -0.10 (24H, s, SiCH₃). ¹³C{¹H}-NMR (CDCl₃): δ 51.1 (CH₂N₃), 32.6 (SiCH₂CH₂CH₂CH₂N₃), 21.2 (SiCH₂CH₂), 20.5 (SiCH₂CH₂), 19.2 (SiCH₂*C*H₂),18.8 (Si*C*H₂),18.6 (Si*C*H₂), 18.5 (Si*C*H₂), 17.7 (Si*C*H₂), 15.0 (Si*C*H₂), -3.3 (Si(*C*H₃)₂), -4.9 (Si*C*H₃). Anal. Calcd. C₈₈H₁₉₆N₂₄Si_{13.} %: C, 54.04; H, 10.10; N, 17.19; Exp. %: C, 54.81; H, 9.72; N, 12.94.

### Example 1.21: Synthesis of G₃-[SiCH₂CH₂CH₂CH₂N₃]16 (32)

This third generation dendrimer was prepared using a similar method to that described for 30 starting from 28 (1.82 g, 0.38 mmol) dissolved in DMF-THF (30 mL/10 mL), 0.50 g of sodium azide (7.70 mmol) and a small amount of Kl. The reaction was stirred during five days at 90 °C into a sealed ampoule for 16h under vacuum. Afterward, volatiles were removed under vacuum and 30mL of dichloromethane was added and extracted twice with water (2x15mL). The organic phase was separated and was dried over MgS0₄. The solution was filtered and the volatiles were removed under vacuum, to obtain 32 as a pale yellow oil (1.33 g, 82%).

¹H-NMR (CDCl₃): δ 3.24 (32H, t, *CH*₂N₃),1.60 (32H, m, SiCH₂CH₂*CH₂*CH₂N₃), 1.32 (88H, m, SiCH₂*CH*₂CH₂CH₂N₃ y SiCH₂C*H*₂CH₂Si), 0.54 (144H, m, C*H*₂ bonded to Si), -0.05 (120H, s, Si(C*H₃*)₂ y SiCH₃), -0.09 (12H, s, SiC*H*₃). ¹³C{¹H}-NMR (CDCl₃): δ 51.1 (CH₂N₃), 32.6 (SiCH₂CH₂*C*H₂CH₂N₃), 21.2 (SiCH₂*C*H₂), 20.0 (SiCH₂*C*H₂), 19.0-17.5 (Si*C*H₂) 15.0 (Si*C*H₂), -3.3 (Si(*C*H₃)₂), -4.9 (Si*C*H₃). Anal. Calcd C₁₉₂H₄₂₈N₄₈Si₂₉. %: C, 54.59; H, 10.21; N, 15.92; Exp. %: C, 56.08; H, 9.58; N, 11.08.

### Example 1.22: Synthesis of ck-G₁-[Si(CH₂)₄(N₃C₂H)CH₂NH₂]₄(34)

Over a mixture of 0.31 g (0.38 mmol) of G₁-[SiCH₂CH₂CH₂CH₂N₃]₄ dissolved in THF (10 mL) and 0.11 mL (1.66 mmol) of propargylamine was added a water solution (1.25 mL) of sodium ascorbate (0.04 g, 0.11 mmol) and a solution of C_{U}S0₄ (0.02 mg, 0.07 mmol) in 1.25 mL of water. The reaction mixture was stirred at room temperature during 16h. Afterward, 2 mL of NH₃ solution (15%) was added and stirred during 10 min. The resulting solution was extracted with EtOAc (3x10 mL).The combined organic phase was dried with sodium sulfate, concentrated, and the solvent removed under vacuum to afford the first generation dendrimer 34 as a yellow oil.

¹H-NMR (CDCl₃): δ □7.41 (4H, s, NCHCN), 4.30 (8H, t, CH₂C*H*₂N), 3.97 (8H, sb, N*H*₂), 1.88 ((8H, m, C*H*₂CH₂N), 1.27 (16H, m, SiCH₂C*H*₂), 0.54 (24H, m, SiC*H*₂), 0.08 (24H, s, SiC*H*₃).

### Example 1.23: Synthesis of ck-G₁-[Si(CH₂)₄(N₃C₂H)CH₂N(CH₂CH₂COOMe)₂]₄ (38)

This first generation dendrimer 38 was prepared using a similar method to that described for 34 starting from G₁-[SiCH₂CH₂CH₂CH₂N3]₄ (0.30 g, 0.37 mmol) in 5 ml of THF, the propargylamine funcionalizated with ester groups (D) (0.33 g, 1.46 mmol), a water solution (0.75 mL) of sodium ascorbate (35 mg, 0.18 mmol) and a solution of CuS0₄ (18 mg, 0.073 mmol) in 0.75 mL of water. The reaction mixture was stirred at room temperature during 16h. Afterward, 4 mL of NH₃ solution (15%) was added and stirred during 10 min. The resulting solution was extracted with EtOAc (3x10 mL).The combined organic phase was dried with sodium sulfate, concentrated, and the solvent removed under vacuum. Compound **38** was purified by Gel Permeation Chromatography, to afford the desired product as yellow oil (0.43 g, 68%).

¹H-NMR (CDCl₃): δ □7.43 (4H, s, NC*H*CN), 4.3 (8H, t, CH₂C*H*₂N), 3.78 (8H, s, CC*H₂*N), 3.64 (24H, s, OC*H*3), 2.78 (16H, t, NCH₂*CH*₂C(O)), 2.47 (16H, t, NC*H₂*CH₂C(O)); 1.90 (8H, m, C*H*₂CH₂N), 1.27 (16H, m, SiCH₂C*H*₂), 0.52 (24H, m, SiC*H*₂), -0.08 (24H, s, SiC*H₃*). ¹³C {¹H}-NMR (CDCl₃): δ 172.8 (*C*O₂CH₃), 144.6 (NC*C*CH₂), 122.2 (N*C*CCH₂), 51.5 (O*C*H3), 49.9 (*C*H₂N), 48.9 (*C*H₂N), 48.7 (*C*H₂N), 34.0 (CH₂CH₂*C*H₂CH₂N), 32.6 (*C*H₂C0), 21.0 (CH₂*C*H₂CH₂Si), 20.1 (CH₂CH₂CH₂), 18.5 (SiCH₂), 17.5 (Si*C*H₂), 14.9 (Si*C*H₂), -3.4 (Si*C*H₃).

### Example 1.24: Synthesis of ck-G₁-[Si(CH₂)₄(N₃C₂H)CH₂N(CH₂PO(OMe)₂)₂]₄ (50)

This first generation dendrimer 50 was prepared using a similar method to that described for 34 starting from G₁-[SiCH₂CH₂CH₂CH₂N₃]₄ (0.30 g, 0.37 mmol) in 10 ml of TNF, the propargylamine funcionalizated with phospite groups (F) (0.44 g, 1.46 mmol), a water solution (1.25 mL) of sodium ascorbate (35 mg, 0.18 mmol) and a solution of C_{U}S0₄ (18 mg, 0.073 mmol) in 1.25 mL of water. The reaction mixture was stirred at room temperature during 16h. Afterward, 2 mL of NH₃ solution (15%) was added and stirred during 10 min. The resulting solution was extracted with EtOAc (3x10 mL). The combined organic phase was dried with sodium sulfate, concentrated, and the solvent removed under vacuum to afford 50 as yellow oil (0.16 g, 65%).

¹H-NMR (CDCl₃): δ □7.6 (4H, s, NCHCN), 4.32 (8H, t, CH₂C*H*₂N), 4.13 (8H, s, CC*H*₂N), 3.76 (48H, d, OC*H3*), 3.15 (4H, d, NC*H*₂P), 1.89 (8H, m, C*H*₂CH₂N), 1.27 (16H, m, SiCH₂C*H*₂), 0.54 (24H, m, SiC*H*₂), -0.08 (24H, s, SiC*H*₃). ¹³C{¹H}-NMR (CDCl₃): δ 143.1 (NC*C*CH₂), 123.0 (N*C*CCH₂), 52.7 (d, O*C*H₃), 51.2 (t, C*C*H₂N), 50.0, 49.0 (dd, N*C*H₂P), 34.1 (CH₂CH₂*C*H₂CH₂N), 21.0 (CH₂*C*H₂CH₂CH₂N), 20.1 (CH₂*C*H₂CH₂Si), 18.5 (Si*C*H₂), 17.5 (Si*C*H₂), 14.9 (Si*C*H₂), -3.4 (Si*C*H₃).

### EXAMPLE 2: Dendrimers from a polyphenolic core.

### A.- Synthesis of dendrons or dendritic wedges with a focal point and peripheral groups properly functionalized.

The synthetic procedure of dendritic wedges of generations 1-3 with terminal allyl groups from a bromoalkene, as the 4-bromobutene, is described in Scheme 12. In a first step, hydrosilylation of the double bond of the alkene with HSiMeCl₂ leads to formation of the dendritic wedge of first generation with terminal SiCl₂ groups, BrG₁Cl₂ (**65**), which can be transformed into the corresponding first generation dendritic wedge with terminal allyl groups by reaction with magnesium allyl bromide, BrMg(C₃H₅), and which we denominated BrG₁A₂ (**66**). The repetition of the two previous reactions with compound BrG₁A₂ leads to 2^{nd} generation dendrons, BrG₂Cl₄ (**67**) and BrG₂A₄ (**68**) and if they are repeated again, now on the BrG2A₄ wedge, the 3^{rd} generation dendrons BrG₃Cl₈ (**69**) and BrG₃A₈ (**70**) are obtained. 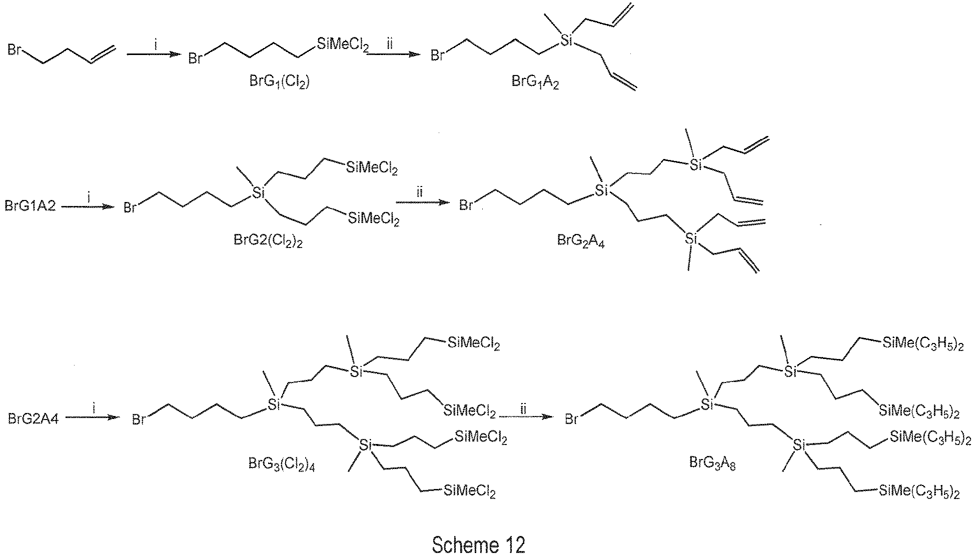 where: i) HSiMeCl₂, Platinum catalyst; ii) BrMg(C₃H₅)

Functionalization of previously described dendritic wedges with terminal Si-H bonds were done first by hydrosilylation of allyl terminated dendrons with chlorodimethylsilane, SiMe₂ClH, and second by Cl-H exchange with lithium aluminium hydride, LiAlH₄, as is described in Scheme 13. Thus, starting from 1^{st} generation dendron BrG₁A₂ (66), the dendritic wedge BrG₁H₂ (71) would be isolated, from 2^{nd} generation dendron 68 the dendritic wedge BrG₂H₄ (72) would be isolated and from 3^{rd} generation dendron 69 the dendritic wedge with 8 SI-H groups BrG₃H₈ (73) would be obtained. 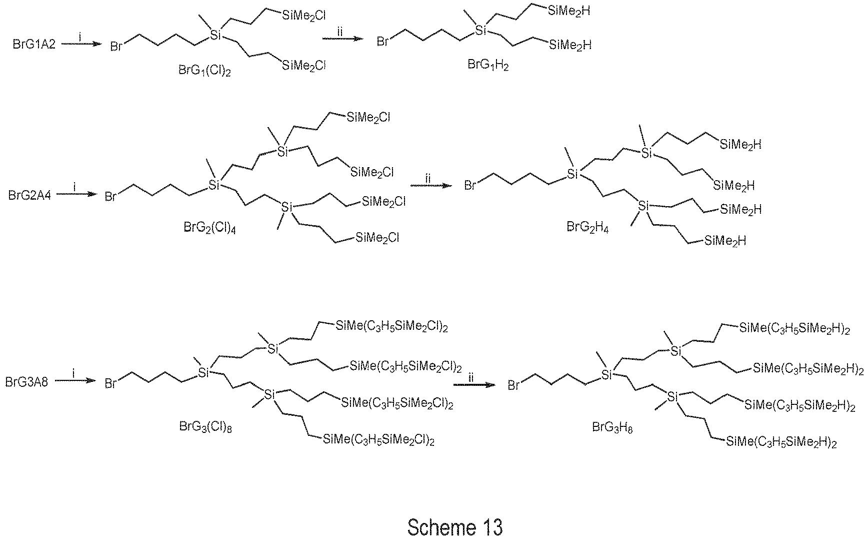 where: i) HSiMe₂Cl, platinum catalyst; ii) LiAlH₄.

A third type of dendritic wedges, which are reflected in this invention, are those that present in the focal point a functional group -CH₂Br, and are functionalized in the periphery with ester groups, which are the precursor groups of anionic ligands, as we have described in part I of this report. These dendrons have been prepared up to the third-generation following the synthetic route that is described in the Scheme 14. The reaction consist on hydrosilylation of carbosilane wedges with terminal Si-H bonds with an allylamine containing methyl acrylate groups, using the Karstedt's catalyst. The allylamine used in this synthesis is described as compound A.

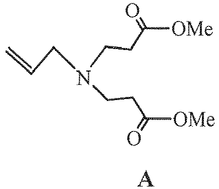

Thus, if the reaction is based on the first-generation dendritic wedge **71,** its reaction with compound A leads to the first-generation dendritic wedge decorated with 4 terminal methyl ester groups BrG₁(COOMe)₄ (**74**). If the starting compound is the second generation wedge 8, the second-generation wedge BrG₂(COOMe)₈ is obtained (**75**), which has 8 groups ester on the surface, and if the reactions is carried out with the third generation wedge 9 and the allylamine **A,** the third generation dendron BrG₃(COOMe)₁₆ (**76**) is obtained, which has 16 ester groups in the periphery. 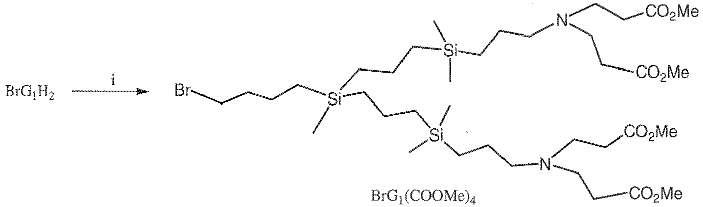 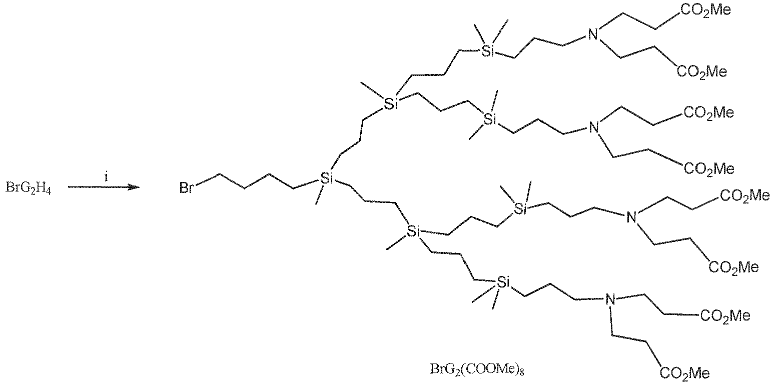 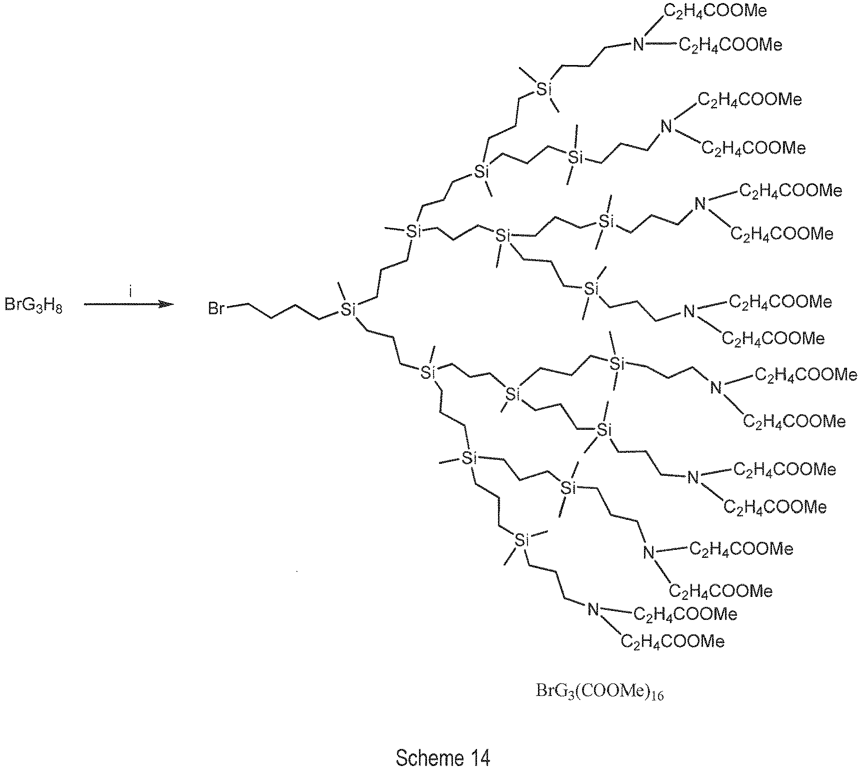 where: i) C₃H₅N(C₂H₄COOMe)₂, platinum catalyst.

The dendrons or dendritic wedges described in this section, **65-76,** have been characterized by elemental analysis of carbon, hydrogen and nitrogen, ¹H, ¹³C and ²⁹Si NMR spectroscopies, and mass spectrometry, which are detailed below:

### Synthesis of BrG₁(SiCl₂) (65).

To a solution of 4-Bromo-1-butene (4.00 g, 0.030 mol) in hexane (4 mL) cooled at 0° C is added HSiMeCl₂ (5.10 g, 0.045 mol) and Kardsted's catalyst (3% mol). The reaction is stirred at room temperature overnight and then volatiles removed under vacuum. Hexane is added (15 mL) and the solution is filtered through active carbon. The solvent was evaporated and compound BrG₁(SiCl₂) (**65**) is obtained as a colourless liquid (7.03 g, 95%).

¹H-NMR (CDCl₃): □□ 0.77 (s, 3 H, *Me*SiCl₂), 1.11 (m, 2 H, *CH*₂Si), 1.65 (m, 2 H, C*H*₂CH₂Si), 1.93 (m, 2 H, BrCH₂C*H*₂), 3.41 (t, J = 6.6 Hz, 2 H, BrC*H*₂); ¹³C- NMR{¹H} (CDCl₃): □□ 5.1 (*Me*SiCl₂), 20.6 (*C*H₂CH₂Si), 21.1(CH₂*C*H₂Si), 32.8 (Br*C*H₂CH₂), 34.9 (BrCH₂*C*H₂); ²⁹Si-NMR (CDCl₃): □□ 32.3 (MeS*i*Cl₂).

### Synthesis of BrG₁A₂(66).

To a solution of BrG₁(SiCl₂) (10.00 g, 0.040 mol) in Et₂O (25 mL) cooled at 0° C is slowly added BrMg(C₃H₅) (0.085 mol) and the mixture is stirred at room temperature overnight. Afterward, a cold solution of NH₄Cl in water (12%, 50 mL) is added. Next, a water/ether extraction (3 times) is carried out, the organic phase is dried with MgSO₄ and finally volatiles are removed under vacuum, obtaining compound BrG₁A₂ as a colourless liquid (8.35 g, 80%).

¹H-NMR (CDCl₃): □□□-0.02 (s, 3 H, *Me*Si(CH₂CHCH₂)₂), 0.53 (m, 2 H, C*H*₂Si), 1.44 (m, 2 H, C*H*₂CH₂Si), 1.54 (d, J = 8.5 Hz, 4 H, SiC*H*₂CH), 1.85 (m, 2 H, BrCH₂C*H*₂), 3.40 (t, J = 6.6 Hz, 2 H, BrC*H*₂), 4.84 (m, 4 H, CH=C*H*₂), 5.74 (m, 2 H, C*H*=CH₂); ¹³C-NMR{¹H} (CDCl₃): □□□-5.9 (e), 12.0 (d), 21.2 (f), 22.1 (c), 33.5 (a), 36.2 (b), 113.3 (h), 134.5 (g); ²⁹Si-NMR (CDCl₃): □□ 0.99 (Me*Si*). Anal. Calc. C₁₁H₂₁BrSi (261,27 g/mol): C, 50.57; H, 8.10; Exp.: C, 51.03; H, 8.17.

### Synthesis of BrG₂(SiCl₂)₂ (67).

This compound is prepared from BrG₁A (4.30 g, 0.016 mol), HSiMeCl₂ (3.41 g, 0.03 mol) and Kardsted's catalyst (3% mol) following the procedure described for the synthesis of BrG₁(SiCl₂), obtaining BrG₂(SiCl₂)₂ (67) as a colorless oil (7.60 g, 94 %).

¹H-NMR (CDCl₃): □□-0.03 (s, 3 H, *Me*Si), 0.53 (m, 2 H, SiC*H*₂), 0.66 (m, 4 H, SiC*H*₂), 0.75 (s, 6 H, *Me*SiCl₂), 1.19 (m, 4 H, C*H*₂SiCl₂), 1.50 (m, 6 H, C*H*₂), 1.85 (m, 2 H, BrCH₂C*H*₂), 3.40 (t, J = 6.6 Hz, 2 H, BrC*H*₂); ¹³C-NMR{¹H} (CDCl₃): □□-5.1 (*Me*Si), 5.1 (*Me*SiCl₂), 12.5 (Si*C*H₂), 17.3 (*C*H₂), 17.9 (*C*H₂), 22.3 (BrCH₂CH₂*C*H₂), 25.9 (*C*H₂SiCl₂), 33.7 (Br*C*H₂), 36.4 (BrCH₂*C*H₂); ²⁹Si-NMR (CDCl₃): □□ 2.3 (Me*Si*), 32.1 (MeS*i*Cl₂).

### Synthesis Of BrG₂A₄(68).

This compound is prepared from BrG₂(SiCl₂)₂ (6.25 g, 0.013 mol) and BrMg(C₃H₅) (0.055 mol) following the procedure described for the synthesis of BrG₁A₂, obtaining BrG₂A₄ as a colorless oil (5.10 g, 78 %).

¹H-NMR (CDCl₃): □□□-0.08 (s, 3 H, *Me*Si), -0.04 (s, 6 H, *Me*Si), 0.56 (m, 10 H, SiC*H*₂), 1.25 (m, 6 H, C*H*₂), 1.52 (d, J = 8.4 Hz, 8 H, C*H*₂CH=CH₂), 1.84 (m, 2 H, BrCH₂C*H*₂), 3.40 (t, J = 6.6 Hz, 2 H, BrC*H*₂), 4.84 (m, 8 H, CH=C*H*₂), 5.74 (m, 4 H, C*H*=CH₂); ¹³C-NMR{¹H} (CDCl₃): □□□-5.7 (*Me*Si), -5.1 (*Me*Si), 12.9 (Si*C*H₂), 17.9, 18.2 y 18.6 (*C*H₂), 21.4 (*C*H₂CH=CH₂), 22.5 (BrCH₂CH₂*C*H₂), 33.6 (Br*C*H₂), 36.3 (BrCH₂*C*H₂), 113.0 (CH=*C*H₂), 134.8 (*C*H=CH₂); ²⁹Si-NMR (CDCl₃): □□□0.1 (MeS*i*), 1.8 (MeS*i*), Anal. Calc.. C₂₅H₄₉BrSi₃ (513.82 g/mol): C, 58.44; H, 9.61; Exp.: C, 58.01; H, 9.53.

### Synthesis of BrG₃(SiCl₂)₄ (69).

This compound is prepared from BrG₂A₄ (2.50 g, 4.86 mmol), HSiMeCl₂ (3.80 g, 0.033 mol) and Kardsted's catalyst (3% mol) following the procedure described for the synthesis of BrG₁(SiCl₂), obtaining BrG₃(SiCl₂)₄ as a colorless oil (4.45 g, 94 %).

¹H-NMR (CDCl₃): □□□-0.07 (s, 3 H, *Me*Si), -0.04 (s, 6 H, *Me*Si), 0.58 (m, 18 H, SiC*H*₂), 0.75 (s,12 H, *Me*SiCl₂), 0.87 (m, 8 H, C*H*₂), 1.16 (m, 8 H, C*H*₂SiCl₂), 1.52 (m, 6 H, C*H*₂), 1.85 (m, 2 H, BrCH₂C*H*₂), 3.40 (t, J = 6.6 Hz, 2 H, BrC*H*₂); ¹³C-NMR{¹H} (CDCl₃): □□-5.2 (*Me*Si), 5.5 (*Me*SiCl₂), 12.9 (Si*C*H₂), 17.3 y 17.5 (CH₂), 18.4, 18.6 y 18.7 (CH₂), 22.6 (BrCH₂CH₂*C*H₂), 25.9 (CH₂SiCl₂), 33.6 Br*C*H₂), 36.3 (BrCH₂*C*H₂); ²⁹Si-NMR (CDCl₃): □□□1.4 (MeS*i*), 1.8 (MeS*i*), 32.1 (Me*Si*Cl₂).

### Synthesis of BrG₃A₈ (70).

This compound is prepared from BrG₃(SiCl₂)₄ (3.30 g, 3.39 mmol) and BrMg(C₃H₅) (0.031 mol) following the procedure described for the synthesis of BrG₁A₂, obtaining BrG₃A₈ as a colorless oil (2.62 g, 76 %).

¹H-NMR (CDCl₃): □□-0.10 (s, 3 H, *Me*Si), -0.08 (s, 6 H, *Me*Si), -0.04 (s, 12 H, *Me*Si), 055 (m, 26 H, SiC*H*₂), 1.31 (m, 16 H, C*H*₂), 1.52 (d, J = 8.4 Hz, 16 H, C*H*₂CH=CH₂), 1.84 (m, 2 H, BrCH₂C*H*₂), 3.40 (t, J = 6.6 Hz, 2 H, BrC*H*₂), 4.84 (m, 16 H, CH=C*H*₂), 5.73 (m, 8 H, C*H*=CH₂); ¹³C-NMR{¹H} (CDCl₃): □□□-5.7 (*Me*Si), -5.1 (*Me*Si), -4.9 (*Me*Si), 13.0 (Si*C*H₂), 17.9, 18.3, 18.5, 18.8 y 18.9 (*C*H₂), 21.5 (*C*H₂CH=CH₂), 22.6 (BrCH₂CH₂*C*H₂), 33.6 (Br*C*H₂), 36.4 (BrCH₂*C*H₂), 113.1 (CH=*C*H₂), 134.9 (*C*H=CH₂); ²⁹Si-NMR (CDCl₃): □□ 0.1 (MeS*i*), 0.9 (MeS*i*), 1.8(MeS*i*), Anal. Calc. C₅₃H₁₀₅BrSi₇ (1018.90 g/mol): C, 62.48; H, 10.39; Exp.: C, 61.92; H, 10.33.

### Synthesis of BrG₁H₂ (71).

A solution of BrG₁(SiCl)₂ (2.00 g, 4.44 mmol) in Et₂O (25 mL) is slowly added to a solution of LiAlH₄ (7.76 mmol) in Et₂O (25 mL) at 0 °C. Once the addition is completed the reaction is stirred at room temperature overnight. Afterward, the mixture is poured over an aqueous solution of NaCl and then a water/ether extraction (3 times) is carried out. The organic phase is dried with MgSO₄ and finally evaporated to dryness, obtaining compound **71** as a colorless liquid (1.20 g, 71%).

¹H-NMR (CDCl₃): □□□ -0.08 (s, 3 H, MeSi), 0.04 (d, J = 4.2 Hz, 12 H, *Me*₂SiH), 0.52 (m, 2 H, SiC*H*₂), 0.58 (m, 8 H, SiC*H*₂), 1.38 (m, 6 H, C*H*₂), 1.85 (m, 2 H, BrCH₂C*H*₂), 3.40 (t, J = 6.6 Hz, 2 H, BrC*H*₂), 3.82 (m, 2 H, Si*H*); ¹³C-NMR{¹H} (CDCl₃): □□□□-5.1 (*Me*Si), -4.3 (*Me*₂SiH), 12.9 (Si*C*H₂), 17.9 (*C*H₂), 18.8 y 19.0 (*C*H₂), 22.5 (BrCH₂CH₂*C*H₂), 33.7 (Br*C*H₂), 36.4 (BrCH₂*C*H₂); ²⁹Si-NMR (CDCl₃): □□□ -14.6 (Me₂S*i*H), 2.1 (MeS*i*). Anal. Calc. C₁₅H₃₇BrSi₃ (381.61 g/mol): C, 47.21; H, 9.77; Obt.: C, 47.89; H, 9.53.

### Synthesis of BrG₂H₄ (72).

This compound is prepared from BrG₂(SiCl)₄ (3.15 g, 3.53 mmol) and LiAlH₄ (10.60 mmol) following the procedure described for the synthesis of BrG₁H₂, obtaining BrG₂H₄ (**72**) as a colorless oil (1.76 g, 66 %).

¹H-NMR (CDCl₃): □□ -0.10 (s, 6 H, *Me*Si), -0.08 (s, 3 H, *Me*Si), 0.04 (d, J = 4.2 Hz, 12 H, *Me*₂SiH), 0.54 (m, 26 H, SiC*H*₂), 1.35 (m, 14 H, C*H*₂), 1.85 (m, 2 H, BrCH₂C*H*₂), 3.40 (t, J = 6.6 Hz, 2 H, BrC*H*₂), 3.82 (m, 4 H, Si*H*); ¹³C-NMR{¹H} (CDCl₃): □□ -5.0 (*Me*Si), -4.38 (*Me*₂SiH), 13.0 (Si*C*H₂), 18.2, 18.5, 18.7, 18.8, 19.0 (*C*H₂), 22.5 (BrCH₂CH₂*C*H₂), 33.6 (Br*C*H₂), 36.4 (BrCH₂*C*H₂); ²⁹Si-NMR (CDCl₃): □□□-14.1 (Me₂S*i*H), 1.0 (MeS*i*), 1.8 (MeS*i*). Anal. Calc. C₃₃H₈₁BrSi₇ (754.50 g/mol): C, 52.53; H, 10.82; Obt.: C, 53.00; H, 10.25.

### Synthesis of BrG₃H₈ (73).

This compound is prepared from BrG₃(SiCl)₈ (2.00 g, 1.13 mmol) and LiAlH₄ (6.75 mmol) following the procedure described for the synthesis of BrG₁H₂, obtaining BrG₃H₈, 73, as a colorless oil (1.11 g, 66 %).

¹H-NMR (CDCl₃): □-0.10 (s, 21 H, *Me*Si), 0.04 (d, J = 4.2 Hz, 48 H, *Me*₂SiH), 0.58 (m, 58 H, SiC*H*₂), 1.31 (m, 30 H, C*H*₂), 1.82 (m, 2 H, BrCH₂C*H*₂), 3.40 (t, J = 6.6 Hz, 2 H, BrC*H*₂), 3.83 (m, 8 H, Si*H*); ¹³C-NMR{¹H} (CDCl₃): □□□□ (*Me*Si), -4.3 (*Me*₂SiH), 13.0 (Si*C*H₂), 18.2-19.0 (*C*H₂), 22.6 (BrCH₂CH₂*C*H₂), 33.5 (Br*C*H₂), 36.5 (BrCH₂*C*H₂); ²⁹Si-NMR (CDCl₃): □□□-4.6 (Me₂S*i*H), 1.4 (MeS*i*). Anal. Calc. C₆₉H₁₆₉BrSi₁₅ (1500.27 g/mol): C, 55.24; H, 11.35; Obt.: C, 54.88; H, 10.73.

### Synthesis of BrG₁(COOMe)₄ (74).

A solution of BrG₁H₂ (1.00 g, 2.62 mmol), C₃H₅N(C₂H₄COOMe)₂ (1.21 g, 5.24 mmol) and Kardsted's catalyst (3% mol) in hexane (2 mL) is stirred overnight. Hexane (15 ml) is then added and the solution is filtered through active carbon. The solvent is removed under vacuum and BrG₁(COOMe)₄, 74, is obtained as a colorless oil (2.04 g, 92%).

¹H-NMR (CDCl₃): □□-0.10 (s, 3 H, *Me*Si), -0.08 (s, 12 H, *Me*₂Si), 0.33 (m, 4 H, SiC*H*₂), 0.52 (m, 10 H, SiC*H*₂), 1.27 (m, 4 H, C*H*₂), 1.35 (m, 6 H, C*H*₂), 1.83 (m, 2 H, BrCH₂C*H*₂), 2.33 (m, 4 H, C*H*₂N), 2.41 (t, J = 7.3 Hz, 8 H, C*H*₂N), 2.74 (t, J = 7.3 Hz, 8 H, C*H*₂CO), 3.39 (t, J = 6.6 Hz, 2 H, BrC*H*₂), 3.61 (s, 12 H, O*Me*). ¹³C-NMR{¹H} (CDCl₃): □□-5.1 (*Me*Si), -3.3 (*Me*₂Si), 12.8 (Si*C*H₂), 12.9 (Si*C*H₂), 18.4 (*C*H₂), 18.6 y 20.1 (*C*H₂), 21.5 (*C*H₂CH₂N), 22.5 (BrCH₂CH₂*C*H₂), 32.5 (*C*H₂CO), 33.7 (Br*C*H₂), 36.4 (BrCH₂*C*H₂), 49.2 (*C*H₂N), 51.6 (O*Me*), 57.5 (*C*H₂N), 173.1 (CO). Anal. Calc. C₃₇H₇₅BrN₂O₈Si₃ (840.16 g/mol): C, 52.89; H, 9.00; Obt.: C, 52.29; H, 8.63.

### Synthesis of BrG₂(COOMe)₈ (75).

This compound is prepared from BrG₂H₄ (0.30 g, 0.40 mmol) and C₃H₅N(C₂H₄CO₂Me)₂ (0.40 g, 1.75 mmol), following the procedure described for BrG₁(CO₂Me)₄, obtaining BrG₂(CO₂Me)₈ as a colorless oil (0.53 g, 80 %) after a gel permeation chromatography in toluene.

¹H-NMR (CDCl₃): □□-0.10 (s, 6 H, *Me*Si), -0.08 (s, 3 H, *Me*Si) , -0.07 (s, 24 H, *Me*₂Si), 0.37 (m, 8 H, SiC*H*₂), 0.52 (m, 26 H, SiC*H*₂), 1.33 (m, 22 H, C*H*₂), 1.83 (m, 2 H, BrCH₂C*H*₂), 2.33 (m, 8 H, C*H*₂N), 2.41 (t, J = 7.3 Hz, 16 H, C*H*₂N), 2.74 (t, J = 7.3 Hz, 16 H, C*H*₂CO), 3.39 (t, J = 6.6 Hz, 2 H, BrC*H*₂), 3.61 (s, 24 H, O*Me*).

### B.- Coupling of dendrons synthesized in the previous section with a polyphenolic core.

All the dendrons described in the preceding paragraph present in their focal point a -CH₂Br functionality, which can be employed to be anchored to a polyphenolic core, such as hydroquinone or 1,3,5-trihydroxibencene, to give rise to dendrimers with different functional groups on the surface. This coupling reaction of dendritic wedges with C-Br bonds at the focal point to phenolic compounds is generally carried out in acetone at 80° C in the presence of potassium carbonate (K₂CO₃) and crown ether 18-C-6, as described in Scheme 15: 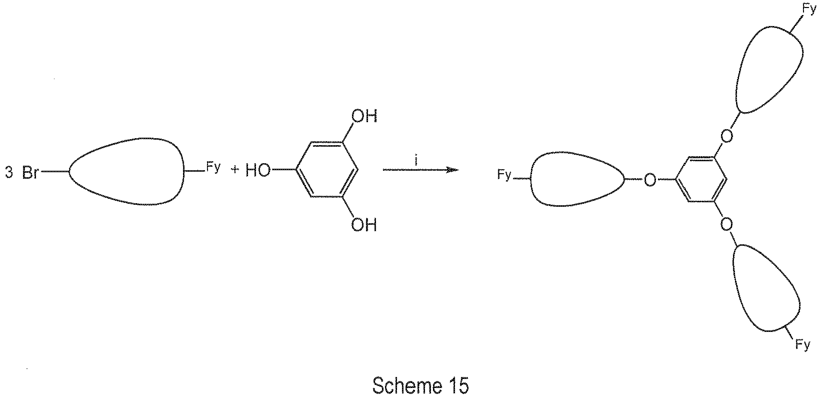 Where: i) K₂CO₃, C₁₈H₃₂O₆, Acetone, 80°C; F are functional groups; y number of functional groups.

In these dendrimers described below has been used a nomenclature of the type GnOxFy, where:
**Gn:** Means the dendrimer generation obtained by coupling of dendritic wedges with polyphenolic nuclei.
Ox: Means that the dendritic wedges have been coupled to the core of the dendrimer through an oxygen atom of the phenolic compound. The number x means the number of groups present in the initial polyphenol derivative. Thus 02, corresponds with coupling to a hydroquinone core and 03 corresponds with coupling to a 1,3,5-trihydroxibencene core, which presents three phenol groups.
**F:** represents one or more letters that indicate the nature of the functional groups present on the surface of the dendrimer. Thus **A** represents an allyl group, **Cl,** terminal Si-Cl bonds, **H** terminal Si-H bonds, **N** terminal amino groups, etc.
**y:** means the number of functional groups present on the surface of the dendrimer.

### Synthesis of dendrimers with terminal allyl groups

The coupling reaction of dendritic wedges with a C-Br bond at the focal point and allyl moieties on the surface, to a polyphenol core, leads to the formation of allyl terminated spherical dendrimers, as is schematised in the Scheme 16 for the first generation dendrimer. For example, the reaction of 1,3,5-trihydroxibencene with the dendritic wedge BrG₁A₂ (2) leads to formation of the dendrimer G₁O3A₆ (13) containing six allyl groups on the surface. If the second generation dendritic wedge BrG₂A₄ (4) is used the second generation dendrimer G₂O₃A₁₂ (14) is obtained and if the third generation wedge BrG₃A₈ **(6)** it is used, the third generation dendrimer G₃O₃A₂₄ **(15)** it is obtained. 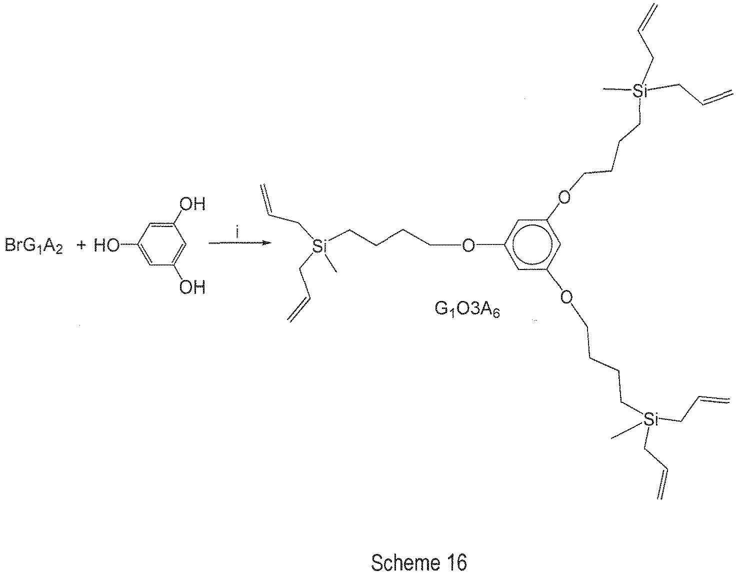 where: i) K₂CO₃, 18C6 (10%), Acetone, 80°C

### Synthesis of dendrimers with terminal Si-H groups

The coupling reaction of dendritic wedges containing a C-Br bond at the focal point and Si-H bonds on the surface, with a polyphenolic core, leads to the formation of spherical dendrimers functionalized with terminals Si-H groups (Scheme 17 for the first generation derivative). In this way, starting from BrG₁H₂ **(71)** the first generation dendrimer G₁O3H₆ **(80)** is obtained, from BrG₂H₄ the second generation dendrimer G₂O3H₁₂ **(81)** is isolated, and from BrG₃H₈ **(73)** dendrimer G₃O3H₂₄ **(82)** containing 24 Si-H branches is obtained.

These derivatives are also available from allyl terminated dendrimers **77-79,** through a two-step process, first hydrosilylation of the allyl dendrimers with HSiMe₂Cl and second substitution of chlorine atoms with hydrogen atoms with LiAlH₄, thus obtaining the dendrimers **80-82,** described above. 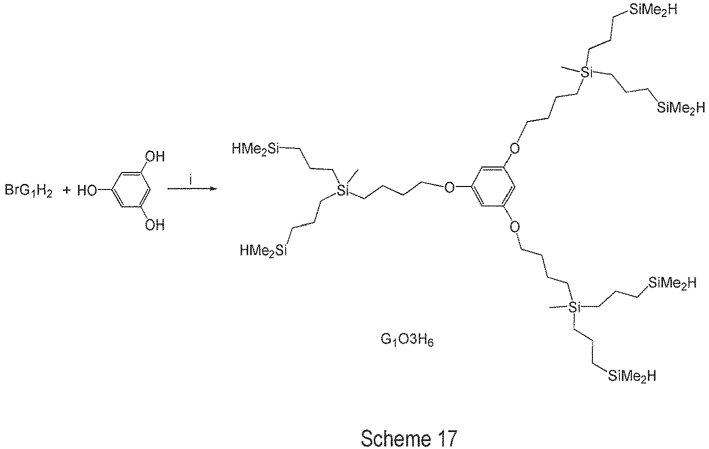 where: i) K₂CO₃, 18C6(10%), Acetone, 80°C

### Synthesis of dendrimers with terminal ester CO₂Me groups.

In a similar way as described in the two preceding paragraphs, the coupling reaction of dendritic wedges with a C-Br bond at the focal point and methyl ester groups on the surface, with a polyphenolic core leads to formation of spherical dendrimers functionalized with terminal ester groups (method A). The Scheme 18 shows the preparation of the first generation dendrimer. In this way, starting from BrG₁(COOMe)₄ **(74)** the first generation dendrimer G₁O3(COOMe)₁₂ **(83)** is obtained, starting from BrG₂(COOMe)₈ **(75)** the second generation dendrimer G₂O3(COOMe)₂₄ **(84)** is formed and starting from BrG₃(COOMe)₁₆ **(76),** the dendrimer G₃O3(COOMe)₄₈ **(85)** which contains 48 terminal ester groups is isolated. Also, these compounds can be prepared by hydrosilylation of allylamine **A** with terminal Si-H dendrimers (**80-82**) (method B), or by Michael-type addition of NH₂ functionalized dendrimers (**86-88**) to methyl acrylate (method C). The Scheme 19 shows the synthesis of these dendrimers with ester groups prepared by methods B and C. 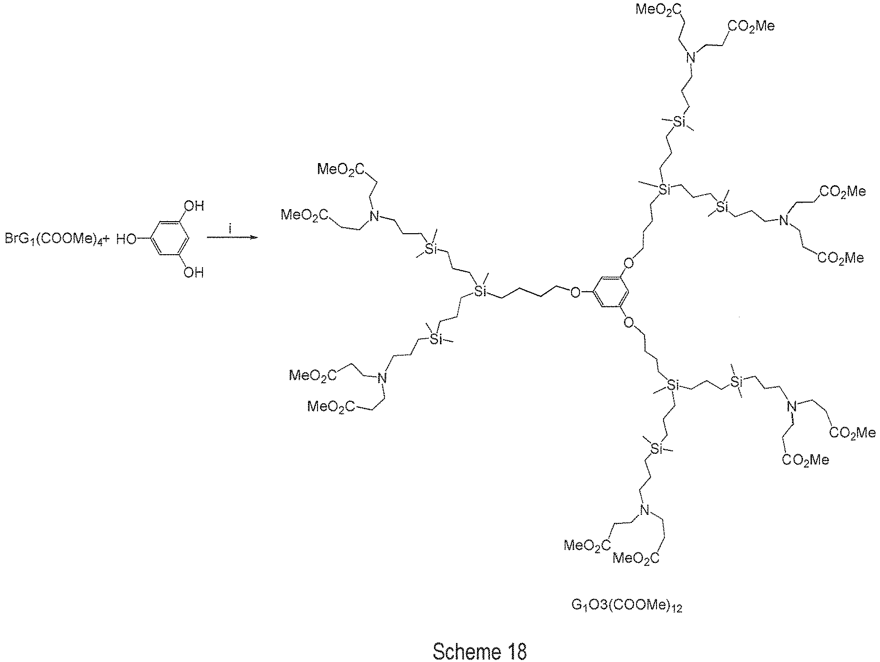 where: i) K₂CO₃,18C6(10%), Acetone, 80°C 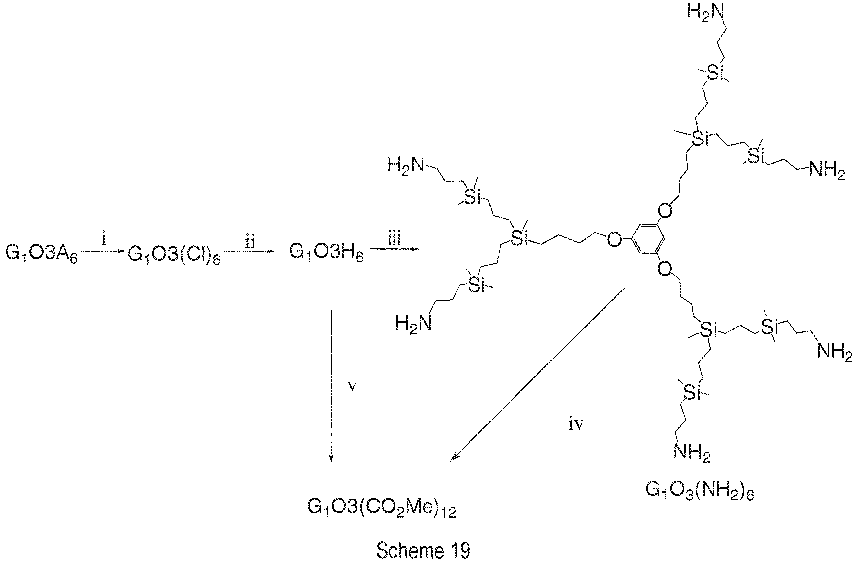 where: i) HSiMe₂Cl, Pt catalyst; ii) LiAlH₄; iii) C₃H₅NH₂, Pt catalyst; iv) C₂H₃CO₂Me; v) C₃H₅N(C₂H₄CO₂Me)₂, Pt cat.

The dendrimers **77-85** have been characterized by elemental analysis of carbon, hydrogen and nitrogen, ¹H, ¹³C and ²⁹Si NMR spectroscopies and mass spectrometry, which are detailed below:

### Synthesis of G₁O3A₆ (77)

A Teflon valve ampoule containing BrG1A₂ (1.50 g, 5.74 mmol), 1,3,5-(HO)₃C₆H₃ (0.24 g, 1.91 mmol), K₂CO₃ (1.59 g, 1148 mmol) and crown ether 18-C-6 (0.15 g, 0.57 mmol) in acetone (50 mL) is heated at 80 °C for 48 hours. After work up and extraction with Et₂O/H₂O (ammoniac, 12%), the organic phase is dried with MgSO₄ and then with SiO₂ for 30 minutes. The solution is filtered, the volatiles removed under vacuum and G₁O3A₆ is obtained as a pale yellow oil (1.01 g, 79%).

¹H-NMR (CDCl₃): □□-0.02 (s, 9 H, *Me*Si), 0.58 (m, 6 H, C*H*₂Si), 1.44 (m, 6 H, OCH₂CH₂C*H*₂), 1.54 (d, J = 8.5 Hz, 12 H, C*H*₂CH=CH₂), 1.76 (m, 6 H, OCH₂C*H*₂), 3.89 (t, J = 6.4 Hz, 6 H, OC*H*₂), 4.84 (m, 12 H, CH=C*H*₂), 5.78 (m, 6 H, C*H*=CH₂), 6.04 (s, 3 H, C₆*H*₃); ¹³C-NMR{¹H} (CDCl₃): □□□-5.9 (*Me*Si), 12.7 (Si*C*H₂), 20.1 (OCH₂CH₂*C*H₂), 21.3 (*C*H₂CH=CH₂), 32.9 (OCH₂*C*H₂), 67.4 (O*C*H₂), 93.7 (*C*₆H₃ (CH), 113.1 (CH=*C*H₂), 134.7 (*C*H=CH₂), 160.9 (*i*-*C*₆H₃); ²⁹Si-NMR (CDCl₃): □□□0.8 (MeS*i*). Anal. Calc. C₃₉H₆₆O₃Si₃ (667.20 g/mol): C 70.21; H 9.97; Obt.: C 69.83; H 9.22.

### Synthesis of G₂O3A₁₂ (78)

Method A) This compound is prepared from BrG₂A₄ (1.50 g, 2.92 mmol), 1,3,5-(HO)₃C₆H₃ (0.12 g, 0.97 mmol), K₂CO₃ (0.81 g, 5.84 mmol) and crown ether 18-C-6 (0.077 g, 0.29 mmol) following the procedure described for the synthesis of G₁O3A₆, obtaining G₂O3A₁₂, **78** as a yellowish oil (0.98 g, 71 %).

Method B) From G₂O3(SiCl₂)₆ (4.20 g, 3.09 mmol) and BrMg(C₃H₅) (0.044 mol), following the procedure described for the synthesis of BrG₁A₂, G₂O3A₁₂ **78** is obtained as a yellowish oil (3.35 g, 76%).

¹H-NMR (CDCl₃): □□□-0.08 (s, 9 H, *Me*Si), -0.04 (s, 18 H, *Me*Si), 0.57 (m, 30 H, SiC*H*₂), 1.30 (m, 12 H, C*H*₂), 1.41 (m, 6 H, OCH₂CH₂C*H*₂), 1.52 (d, J = 7.9 Hz, 24 H, C*H*₂CH=CH₂), 1.75 (m, 6 H, OCH₂C*H*₂), 3.88 (t, J = 6.3 Hz, 6 H, OC*H*₂), 4.84 (m, 24 H, CH=C*H*₂), 5.74 (m, 12 H, C*H*=CH₂), 6.04 (s, 3 H, C₆*H*₃); ¹³C-NMR{¹H} (CDCl₃): □□-5.7 (*Me*Si), -5.1 (*Me*Si), 13.8 (Si*C*H₂), 17.9, 18.2 y 18.6 (*C*H₂), 20.6 (OCH₂CH₂*C*H₂), 21.5 (*C*H₂CH=CH₂), 33.3 (OCH₂*C*H₂), 67.6 (O*C*H₂), 93.7 (*C*₆H₃ (CH), 113.1 (CH=*C*H₂), 134.9 (*C*H=CH₂), 160.9 (*i*-*C*₆H₃); ²⁹Si-NMR (CDCl₃): □□□0.1 (MeS*i*), 1.5 (MeS*i*). Anal. Calc. C₈₁H₁₅₀O₃Si₉ (1424.83 g/mol): C 68.28; H 10.61; Obt.: C 68.01; H 10.31.

### Synthesis of G₃O3A₂₄ (79)

Method A) This compound is prepared from BrG₃A₈ (1.50 g, 1.47 mmol), 1,3,5-(HO)₃C₆H₃ (0.061 g, 0.49 mmol), K₂CO₃ (0.41 g, 2.94 mmol) and crown ether 18-C-6 (0.039 g, 0.14 mmol) following the procedure described for the synthesis of G₁O3A₆, obtaining G₃O3A₂₄, **79,** as a yellowish oil (0.92 g, 62 %).

Method B) From G₃O3(SiCl₂)₁₂ (3.10 g, 1.10 mmol) and BrMg(C₃H₅) (0.027 mol), following the procedure described for the synthesis of BrG₁A₂, G₃O3A₂₄ as a yellowish oil obtained (2.50 g, 77 %).

¹H-NMR (CDCl₃): □□□-0.10 (s, 18 H, *Me*Si), -0.07 (s, 9 H, *Me*Si), -0.04 (s, 36 H, *Me*Si), 0.55 (m, 78 H, SiC*H*₂), 1.31 (m, 42 H, C*H*₂), 1.52 (d, J = 7.9 Hz, 48 H, C*H*₂CHCH₂), 1.75 (m, 6 H, OCH₂C*H*₂), 3.88 (t, J = 6.3 Hz, 6 H, OC*H*₂), 4.84 (m, 48 H, CH=C*H*₂), 5.74 (m, 24 H, C*H*=CH₂), 6.04 (s, 3 H, C₆*H*₃); ¹³C-NMR{¹H} (CDCl₃): □□-5.7 (*Me*Si), -5.0 (MeSi), 13.9 (*C*H₂Si), 17.9, 18.2, 18.5, 18.8, 18.9 (*C*H₂), 20.6 (OCH₂CH₂*C*H₂), 21.5 (*C*H₂CH=CH₂), 33.3 (OCH₂CH₂), 67.7 (O*C*H₂), 93.6 (*C*₆H₃ (*C*H), 113.0 (CH=*C*H₂), 134.9 (*C*H=CH₂), 160.9 (i-*C*₆H₃); ²⁹Si-NMR (CDCl₃): □□□0.1 (MeS*i*), 0.9 (MeS*i*), 1.7 (MeS*i*). Anal. Calc. C₁₆₅H₃₁₈O₃Si₂₁ (2940.08 g/mol): C 67.41; H 10.90; Obt.: C 66.56; H 10.87.

### Synthesis of G₁O3H₆ (80)

This compound can be prepared in a two stages process from G1O3A₆, **77.** The first step consists in hydrosilylation of **77** (1.01 g, 1.51 mmol) with HSiMe₂Cl (1.46 g, 0.015 mol) and Kardsted's catalyst (3% mol), obtaining the dendrimer with Si-Cl bonds G₁O3(SiCl)₆ as a pale yellow oil (1.77 g, 95%). The reaction of G₁O3(SiCl)₆ (1.77 g, 1.43 mmol) with LiAlH₄ (6.45 mmol) following the procedure described for the synthesis of BrG₁H₂, gives rise to G₁O3H₆, **80**, as a colorless oil compound (1.00 g, 68 %).

¹H-NMR (CDCl₃): □□□-0.08 (s, 9 H, *Me*Si), 0.04 (d, J = 4.2 Hz, 36 H, *Me₂*SiH), 0.55 (m, 18 H, SiC*H₂*), 0.63 (m, 12 H, C*H₂*SiH), 1.25 (m, 18 H, C*H₂*), 1.77 (m, 6 H, OCH₂C*H*₂), 3.82 (m, 6 H, Si*H*), 3.88 (t, J = 6.6 Hz, 6 H, OC*H₂*), 6.04 (s, 3 H, C₆*H*₃); ¹³C-NMR{¹H} (CDCl₃): □□-5.1 (*Me*Si), -4.4 (*Me₂*SiH), 13.7 (Si*C*H₂), 18.0, 18.8 y 18.9 (*C*H₂), 20.5 (OCH₂CH₂*C*H₂), 33.2 (OCH₂*C*H₂), 67.6 (OCH₂), 93.8 (*C*₆H₃ (*C*H), 161.0 (*i*-*C*₆H₃); ²⁹Si-NMR (CDCl₃):□□□ -14.0 (Me₂SiH), 1.9 (Me*Si*). Anal. Calc. C₅₁H₁₁₄O₃Si₉ (1028.22 g/mol): C 59.57; H 11.18; Obt.: C 59.89; H 11.53.

### Synthesis of G₂O3H₁₂ (81)

This compound can be prepared in a two stages process from G₂O3A₁₂, **78**. The first step consists in hydrosilylation of **78** (2.00 g, 1.40 mmol) with HSiMe₂Cl (2.71 g, 0.029 mol) and Kardsted's catalyst (3% mol), obtaining the dendrimer with Si-Cl bonds G₂O3(SiCl)₁₂ as a pale yellow oil (3.41 g, 95 %). The reaction of G₂O3(SiCl)₁₂ (1.45 g, 0.57 mmol) with LiAlH₄ (5.10 mmol) following the procedure described for the synthesis of BrG₁H₂, gives rise to G₂O3H₁₂, **81**, as a colorless oil compound (0.80 g, 66 %).

¹H-NMR (CDCl₃): □□□-0.10 (s, 18 H, *Me*Si), -0.08 (s, 9 H, *Me*Si), 0.03 (d, J = 4.2 Hz, 72 H, *Me*₂SiH), 0.55 (m, 54 H, SiC*H*₂), 0.63 (m, 24 H, C*H*₂SiH), 1.35 (m, 42 H, C*H*₂), 1.77 (m, 6 H, OCH₂C*H*₂), 3.82 (m, 12 H, Si*H*), 3.88 (t, J = 6.6 Hz, 6 M, (OC*H*₂), 6.04 (s, 3 H, C₆*H*₃); ¹³C-NMR{¹H} (CDCl₃): □□□-5.1 (*Me*Si), -5.0 (*Me*Si), -4.4 (*Me₂*SiH), 13.9 (Si*C*H₂), 18.2, 18.5, 18.8 y 19.0 (*C*H₂), 20.5 (OCH₂CH₂*C*H₂), 33.3 (OCH₂CH₂), 67.7 (OCH₂), 93.7 (*C*₆H₃ (*C*H), 160.9 (*i-C*₆H₃); ²⁹Si-NMR (CDCl₃): □□□-14.2 (Me₂*Si*H), 0.8 (Me*Si*), 1.9 (Me*Si*). Anal. Calc. C₁₀₅H₂₄₆O₃Si₂₁ (2146.87 g/mol): C 58.74; H 11.55; Obt.: C 57.89; H 11.53.

### Synthesis of G₃O3H₂₄ (82)

This compound can be prepared in a two stages process from G₃O3A₂₄, **82**. The first step consists in hydrosilylation of **79** (2.50 g, 0.85 mmol) with HSiMe₂Cl (3.28 g, 0.034 mol) and Kardsted's catalyst (3% mol), obtaining the dendrimer with Si-Cl bonds G₃O3(SiCl)₂₄ as a pale yellow oil (4.21 g, 95 %). The reaction of G₃O3(SiCl)₂₄ (3.00 g, 0.58 mmol) with LiAlH₄ (0.010 mol) following the procedure described for the synthesis of BrG₁H_{2,} gives rise to G₃O3H₂₄, **82**, as a colorless oil compound (1.64 g, 65 %).

¹H-NMR (CDCl₃): □□□-0.10 (s. a., 54 H, *Me*Si), 0.03 (d, J = 4.2 Hz, 144 H, *Me*₂SiH), 0.58 (m, 174 H, SiC*H*₂), 1.35 (m, 90 H, C*H₂*), 1.77 (m, 6 H, OCH₂C*H₂*), 3.82 (m, 30 H, Si*H* y OC*H*₂), 6.03 (s, 3 H, C₆*H₃*), ¹³C-NMR{¹H} (CDCl₃): □□ -5.0 (*Me*Si), -4.3 (*Me*₂SiH), 13.9 (*C*H₂Si), 18.2, 18.5, 18.8, 18.9 (*C*H₂), 20.6 (OCH₂CH₂CH₂), 33.2 (OCH₂CH₂), 67.7 (OCH₂), 94.5 (*C*₆H₃ (*C*H), 160.9 (i-C₆H₃); ²⁹Si-NMR (CDCl₃): □□□-14.2 (Me₂SiH), 1.0, 1.2 (Me*Si*). Anal. Calc. C₂₁₃H₅₁₀O₃Si₄₅ (4384.17 g/mol): C 58.35; H 11.73; Obt.: C 58.85; H 10.97.

### C. Functionalization of the periphery of the dendrimers obtained in the previous section with anionic groups of different nature, such as e.g. carboxylate, sulfonate, phosphonate.

Once the dendrimers described in the preceding paragraph were isolated, the synthesis of anionic dendrimers can be approached in different ways depending on first the functional groups present in the starting dendrimer and second on the nature of the wished anionic groups in the final dendrimer.

### Anionic carboxylate dendrimers.

Dendrimers with terminal ester groups prepared by the methods A, B and C above described can be transformed into anionic carboxylate dendrimers **89-91** by treatment with a base, e. g. NaOH, in a mixture of MeOH and water (Scheme 20).

Following the previous steps yields to the first generation dendrimer G₁O3(COONa)₁₂ (**89**) containing 12 anionic terminal groups, dendrimer G₂O3(COONa)₂₄ (90) with 24 anionic groups and dendrimer G₃O3(COONa)₄₈ (**91**) that presents 48 carboxylate terminal groups. Scheme 20 describes the synthesis of the first generation dendrimer. 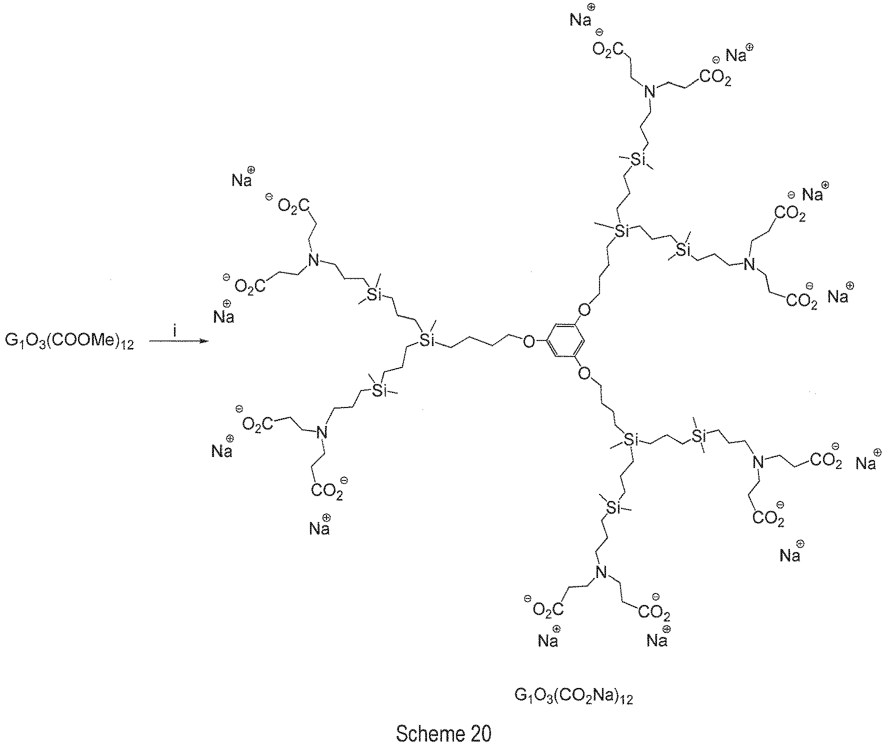 where: i) NaOH

### Anionic sulfonate dendrimers.

The synthesis of anionic dendrimers with terminal sulfonate groups is carried out through a Michael type addition of sodium vinyl sulfonate to NH₂ dendrimers (**86-88**) leading to anionic dendrimers with peripheral sulfonate groups.

In this way, the anionic dendrimers G₁O3(SO₃Na)₁₂ (**92**), G₂O3(SO₃Na)₂₄ (**93**) and G₃O3(SO₃Na)₄₈ (94) have been prepared, containing 12, 24 and 48 terminal sulfonate groups, respectively. The Scheme 21 describes the synthesis of the first generation dendrimer. 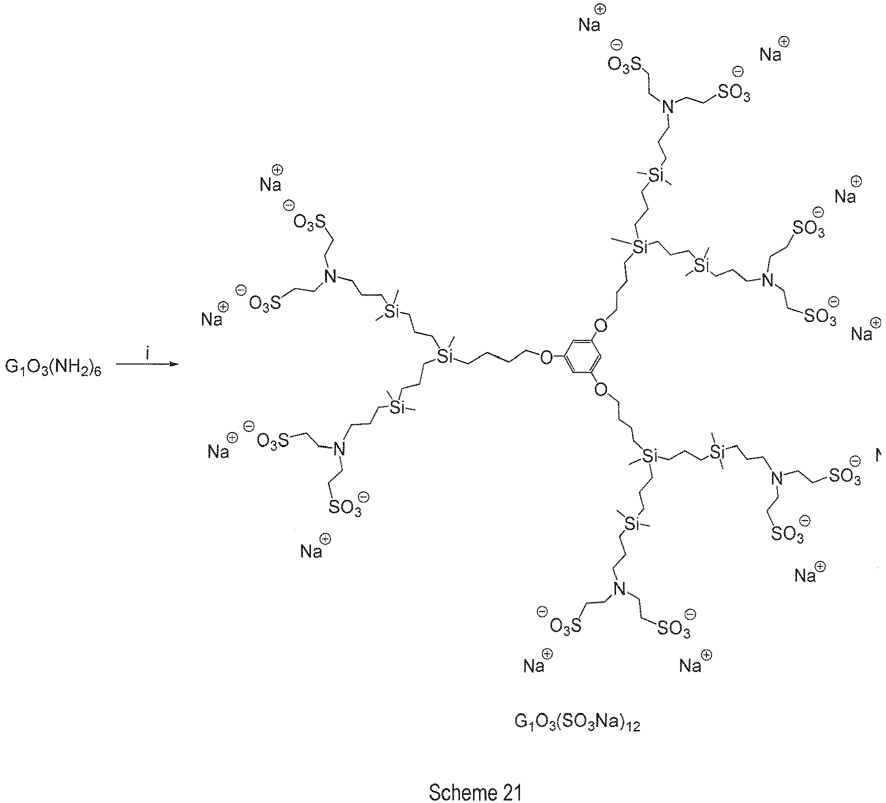 where: i) C₂H₃SO₃Na

### Synthesis of phosphonate anionic dendrimers.

The synthesis of anionic phosphonate dendrimers is carried out starting from NH₂ dendrimers (86-88) in two steps. First, reaction with formaldehyde in THF for 1 hour at room temperature and then is added "*in situ*" dimethyl phosphite and the mixture is heated at 40 °C for 3 hours, giving rise to dendrimers with terminal PO(OMe)₂ units. Finally, these dendrimers react with NaOH transforming the peripheral PO(OMe)₂ groups into anionic -PO(ONa)₂. groups

In this way, the anionic dendrimers G₁O3(PO(ONa)₂)₁₂ **(95)**, G₂O3(PO(ONa)₂)₂₄ **(96)** and G₃O3(PO(ONa)₂)₄₈ **(97)** have been prepared, containing 12, 24 and 48 terminal phosphonate groups, respectively. The Scheme 22 describes the synthesis of the first generation dendrimer. 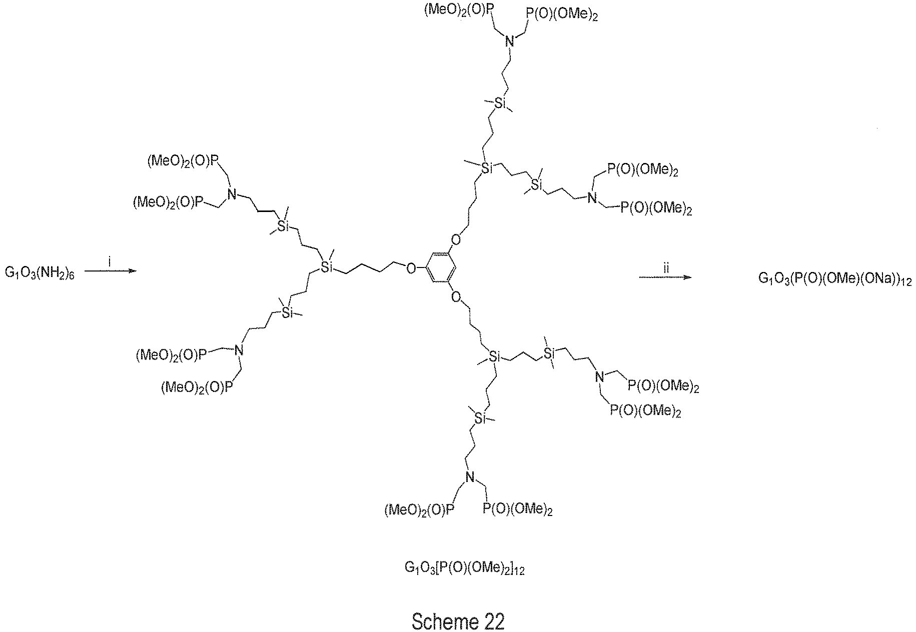 where: i) CH₂O, HP(O)(OMe)₂; ii) NaOH

The dendrimers **86-97** have been characterized by elemental analysis of carbon, hydrogen and nitrogen, ¹H, ¹³C, ²⁹Si and ³¹P NMR spectroscopies and mass spectrometry, as detailed below:

### Synthesis of G₁O3(NH₂)₆ (86).

A teflon valve ampoule containing G₁O3H₆ (0.50 g, 0.49 mmol), allylamine, C₃H₅NH₂ (0.37 g, 6.42 mmol) and Kardsted's catalyst (3% mol) in THF (3 mL) is heated at 120 °C for 48 hours. Next, volatiles are removed, CH₂Cl₂ (10 mL) is added and the solution is filtered through active carbon. The solvent is evaporated and G₁O3(NH₂)₆, **86** is obtained as a pale yellow oil (0.59 g, 89 %).

¹H-NMR (CDCl₃): □□-0.09 (s, 9 H, *Me*Si), -0.06 (s, 36 H, *Me*₂SiH), 0.48 (m, 18 H, SiC*H*₂), 0.54 (m, 36 H, SiC*H₂*), 1.29 (m, 18 H, C*H₂*), 1.40 (m, 24 H, C*H₂*), 1.74 (m, 6 H, OCH₂C*H₂*), 2.63 (t, J = 7.1 Hz, 12 H, C*H₂*N), 3.87 (t, J = 6.3 Hz, 6 H, OC*H₂*), 6.04 (s, 3 H, C₆*H₃*); ¹³C-NMR{¹H} (CDCl₃): □□-5.1 (*Me*Si), -3.3 (*Me*₂Si), 12.3 (NCH₂CH₂*C*H₂), 13.8 (Si*C*H₂), 18.4, 18.6 y 20.0 (*C*H₂), 20.5 (OCH₂CH₂*C*H₂), 33.2 (OCH₂*C*H₂), 45.6 (N*C*H₂), 67.6 (O*C*H₂), 93.7 (*C*₆H₃ (*C*H), 160.9 (*i*-*C*₆H₃); ²⁹Si-NMR (CDCl₃): □□ 1.8 (Me*Si*), 2.1 (Me₂*Si*). Anal. Calc. C₆₉H₁₅₆N₆O₃Si₉ (1370.78 g/mol): C 60.46, H 11.47, N 6.13; Obt.: C 61.11, H 11.26, N 5.86.

### Synthesis of G₂O3(NH₂)₁₂ (87).

The second generation dendrimer **87** is prepared by a procedure similar to that described for compound **86**, starting from G₂O3H₁₂ (0.50 g, 0.23 mmol), C₃H₅NH₂ (0.32 g, 5.59 mmol), obtaining G₂O3(NH₂)₁₂ as a yellowish oil (0.47 g, 72%).

¹H-NMR (CDCl₃): □□ -0.11 (s, 18 H, *Me*Si), -0.09 (s, 9 H, *Me*Si), -0.07 (s, 72 H, *Me*₂SiH), 0.43 (m, 26 H, C*H₂*), 0.54 (m, 72 H, C*H₂*), 1.21 (s, 24 H, N*H₂*), 1.28 (m, 36 H, C*H₂*), 1.38 (m, 30 H), 1.74 (m, 6 H, OCH₂C*H₂*), 2.63 (t, J = 7.1 Hz, 24 H, C*H*₂N), 3.85 (t, J = 6.3 Hz, 6 H, OC*H₂*), 6.04 (s, 3 H, C₆*H*₃); ¹³C-NMR{¹H} (CDCl₃): □□□-4.9(*Me*Si), -4.7 (*Me*Si), -3.3 (*Me*₂Si), 12.3 (Si*C*H₂), 13.8 (Si*C*H₂), 18.4, 18.6, 18.8, 20.0, 20.1 (*C*H₂), 20.6 (OCH₂CH₂*C*H₂), 28.3 (*C*H₂CH₂N), 33.3 (OCH₂*C*H₂), 45.7 (*C*H₂N), 67.6 (O*C*H₂), 93.7 (*C*₆H₃ (*C*H), 160.9 (i-*C*₆H₃); ²⁹Si-NMR (CDCl₃): □□□0.9 (Me*Si*), 1.7 (Me*Si*), 1.9 (Me₂*Si*). Anal. Calc. C₁₄₁H₃₃₀N₁₂O₃Si₂₁ (2832.00 g/mol): C 59.80; H 11.75; N 5.94; Obt.: C 60.92; H 10.03; N 5.44.

### Synthesis of G₃O3(NH₂)₂₄ (88).

The third generation dendrimer **88** is prepared by a procedure similar to that described for compound **86**, starting from G₃O3H₂₄ (1.00 g, 0.23 mmol), C₃H₅NH₂ (0.62 g, 0.011 mol), obtaining G₃O3(NH₂)₂₄ as a yellowish oil (0.48 g, 36 %).

¹H-NMR (CDCl₃): □□-0.11 (s.a., 54 H, *Me*Si), -0.07 (s, 144 H, *Me₂*Si), 0.52 (m, 222 H, SiC*H₂*), 1.21-1.50 (m, 186 H, N*H*₂, C*H₂*), 1.74 (m, 6 H, OCH₂C*H₂*), 2.63 (t, J = 7.1 Hz, 48 H, C*H₂*N), 3.85 (t, J 6.3 Hz, 6 H, OC*H₂*), 6.04 (s, 3 H, C₆*H₃)*; ¹³C-NMR{¹H} (CDCl₃): □□-4.9 (*Me*Si), -3.3 (*Me₂*Si), 12.8 (*C*H₂CH₂CH₂N), 14.1 (Si*C*H₂), 17.9-20.6 (*C*H₂), 27.1 (*C*H₂CH₂N), 33.4 (OCH₂*C*H₂), 45.1 (*C*H₂N), 67.8 (O*C*H₂), 93.7 (*C*₆H₃ (*C*H), 161.0 (i-*C₆*H₃). Anal. Calc. C₂₈₅H₆₇₈N₂₄O₃Si₄₅ (5754.44 g/mol): C 59.49; H 11.88; N 5.84; Obt.: C 58.50; H 11.67; N 5.34.

### Synthesis of G₁O3(COOMe)₁₂ (83).

Method A) This compound is prepared from BrG₁(CO₂Me)₄ (0.55 g, 0.65 mmol), 1,3,5-(HO)₃C₆H₃ (0.027 g, 0.21 mmol), K₂CO₃ (0.18 g, 1.30 mmol) y crown ether 18-C-6 (0.017 g, 0.06 mmol) following the procedure described for the synthesis of G₁O3A₆, obtaining G₁O3(CO₂Me)₁₂ as a yellowish oil (0.36 g, 68 %).

Method B) From G₁O3H₆ (0.20 g, 0.19 mmol) and C₃H₅N(C₂H₄CO₂Me)₂ (0.28 g, 1.18 mmol), following the procedure described for the synthesis of BrG₁(CO₂Me)₄, G₁O3(CO₂Me)₁₂ is obtained as a yellowish oil after wash with hexane (2 x 10 mL) (0.42 g, 89%).

Method C) A solution of G₁O3(NH₂)₆ (0.25 g, 0.18 mmol) and methyl acrylate C₂H₃CO₂Me (0.28 g, 3.28 mmol) in CH₃OH (5 mL) is stirred at room temperature for 16 hours. Then the solution is filtered, volatiles are evaporated and the remaining oil is washed with hexane (2 x 10 mL) to give G₁O3(CO₂Me)₁₂, **83** as a yellowish oil (0.39 g, 90%).

¹H-NMR (CDCl₃): □□□-0.09 (s, 9 H, *Me*Si), -0.07 (s, 36 H, *Me₂*Si), 0.40 (m, 12 H, SiC*H₂*), 0.53 (m, 30 H, SiC*H₂*), 1.27 (m, 12 H, C*H₂*), 1.36 (m, 18 H, C*H₂*), 1.75 (m, 6 H, OCH₂C*H₂*), 2.39 (m, 12 H, C*H₂*N), 2.41 (t, J = 7.3 Hz, 24 H, C*H₂*N), 2.74 (t, J = 7.3 Hz, 24 H, C*H₂*CO), 3.64 (s, 36 H, O*Me*), 3.87 (t, J = 6.6 Hz, 6 H, OC*H₂*), 6.03 (s, 3 H, C₆*H₃*); ¹³C-NMR{¹H} (CDCl₃): □□□-5.1 (*Me*Si), -3.3 (*Me*₂Si), 12.8 (Si*C*H₂), 13.9 (Si*C*H₂), 18.4, 18.6 y 20.1 (*C*H₂), 20.5 (OCH₂CH₂*C*H₂), 21.5 (*C*H₂CH₂N, 32.5 (*C*H₂CO), 33.7 (OCH₂*C*H₂), 49.2 (*C*H₂N), 51.6 (O*Me*), 57.5 (*C*H₂N), 67.7 (O*C*H₂), 93.6 (*C*₆H₃ (*C*H), 160.9 (*i*-*C*₆H₃), 173.1 (*C*O); ²⁹Si-NMR (CDCl₃): □□ 1.6 (Me*Si*), 1.9 (Me₂*Si*). Anal. Calc. C₁₁₇H₂₂₈N₆O₂₇Si₉ (2403.86 g/mol): C 58.46, H 9.56, N 3.50; Obt.: C 58.09, H 9.13, N 3.02.

### Synthesis of G₂O3(COOMe)₂₄ (84).

The synthesis of compound **84** can be carried out following similar procedures to those described for the derivative **83**. For example, from G₂O3H₁₂ (0.35 g, 0.16 mmol) and C₃H₅N(C₂H₄CO₂Me)₂ (0.46 g, 2.00 mmol), following the procedure described for the synthesis of BrG₁(CO₂Me)₄, G₂O3(CO₂Me)₂₄ is obtained as a yellowish oil (0.70 g, 88%).

¹H-NMR (CDCl₃): □□□-0.10 (s, 27 H, *Me*Si), -0.07 (s, 72 H, *Me*₂Si), 0.39 (m, 24 H, SiC*H₂*), 0.53 (m, 78 H, SiC*H₂*), 1.30 (m, 66 H, C*H*₂), 1.75 (m, 6 H, OCH₂C*H₂*), 2.39 (m, 24 H, C*H₂*N), 2.41 (t, J = 7.3 Hz, 48 H, C*H₂*N), 2.74 (t, J = 7.3 Hz, 48 H, C*H₂*CO), 3.64 (s, 72 H, O*Me*), 3.87 (t, J = 6.6 Hz, 6 H, OC*H₂*), 6.03 (s, 3 H, C₆*H*₃); ¹³C-NMR{¹H} (CDCl₃): □□-5.1 (*Me*Si), -3.3 (*Me₂*Si), 12.8 (Si*C*H₂), 13.9 (Si*C*H₂), 18.4, 18.8, 19.0 y 20.1, 20.5, 21.5 (*C*H₂), 32.5 (N*C*H₂), 33.7 (OCH₂*C*H₂), 49.2 (*C*H₂CO), 51.2 (O*Me*), 57.5 (*C*H₂N), 67.7 (OCH₂), 93.6 (*C*₆H₃ (CH), 160.9 (i-*C*₆H₃), 173.1 (CO); ²⁹Si-NMR (CDCl₃): □□□0.9 (Me*Si*), 1.7 (Me*Si*), 1.9 (Me₂*Si*).

### Synthesis of G₃O3(COOMe)₄₈ (85).

The synthesis of compound **85** can be carried out following similar procedures to those described for the derivative **83**. For example, from G₃O3H₂₄ (0.32 g, 0.075 mmol) and C₃H₅N(C₂H₄CO₂Me)₂ (0.41 g, 1.80 mmol), following the procedure described for the synthesis of BrG₁(CO₂Me)₄, G₃O3(CO₂Me)₄₈ is obtained as a yellowish oil (0.63 g, 88 %).

¹H-NMR (CDCl₃): □□-0.12 (s.a., 54 H, *Me*Si), -0.07 (s, 144 H, *Me₂*Si), 0.38 (m, 48 H, SiC*H*₂), 0.52 (m, 174 H, SiC*H*₂), 1.26 (m, 84 H, C*H*₂), 1.38 (m, 54 H, C*H*₂), 1.75 (m, 6 H, OCH₂C*H*₂), 2.45 (m, 144 H, C*H*₂N), 2.74 (s.a., 98 H, C*H*₂CO), 3.63 (s, 144 H, O*Me*), 3.87 (s.a., 6 H, OC*H₂*), 6.00 (s, 3 H, C₆*H₃*); ¹³C-NMR{¹H} (CDCl₃): □ -4.8 (*Me*Si), -3.3 (*Me₂*Si), 12.8 (Si*C*H₂), 14.1 (Si*C*H₂), 18.4-20.5 (*C*H₂), 32.3 (N*C*H₂), 33.6 (OCH₂*C*H₂), 49.2 (*C*H₂CO), 51.6 (O*Me*), 57.4 (*C*H₂N), 67.8 (O*C*H₂), 93.6 (*C*₆H₃ (*C*H), 160.9 (i-*C*₆H₃), 172.8 (CO); ²⁹Si-NMR (CDCl₃): □□□-0.9, 1.8 (Me*Si*), 1.9 (Me₂*Si*). Anal. Calc. C₄₇₇H₉₆₆N₂₄O₉₉Si₄₅ (9886.72 g/mol): C 57.95; H 9.85; N 3.40; Obt.: C 57.09; H 10.10; N 3.63.

### Synthesis of G₁O3(COONa)₁₂ (89).

A solution of NaOH (0.048 g, 1.19 mmol) in H₂O (1 mL) is added to a solution of G₁O3(COOMe)₁₂ (0.20 g, 0.083 mmol) in MeOH (3 mL), and the reaction is stirred for 24 hours. Then solvents are evaporate to dryness and the residue is washed with MeOH (2 x 10 mL) and with Et₂O (2 x 10 mL) and G₁O3(COONa)₁₂, **89** is obtained as a white solid (0.17 g, 82 %).

¹H-NMR (D₂O): □□□-0.26 (s, 45 H, *Me*Si), 0.19 (m, 18 H, SiC*H₂*), 0.32 (m, 24 H, SiC*H₂*), 1.19 (m, 30 H, C*H*₂), 1.45 (m, 6 H, OCH₂C*H₂*), 2.07 (m, 36 H, C*H₂*N), 2.47 (m, 24 H, C*H₂*CO), 3.50 (s, 6 H, OC*H₂*), 5.70 (s, 3 H, C₆*H*₃); ¹³C-NMR{¹H} (D₂O): □□□-4.8 (*Me*Si), -3.5 (*Me*₂Si), 12.8 (Si*C*H₂), 13.7 (Si*C*H₂), 18.4-20.2 (*C*H₂), 32.9 (OCH₂*C*H₂), 34.2 (*C*H₂N), 49.4 (*C*H₂CO), 57.0 (*C*H₂N), 67.1 (O*C*H₂), 93.7 (*C*₆H₃ (*C*H), 160.5 (*i*-*C*₆H₃), 181.1 (*C*O); ²⁹Si-NMR (D₂O): □□ 1.6 (Me*Si*).

### Synthesis of G₂O3(COONa)₂₄ (90).

From G₂O3(COOMe)₂₄ (0.35 g, 0.071 mmol) and NaOH (0.046 g, 1.16 mmol), following the procedure described for the synthesis of **89**, G₂O3(COONa)₂₄ (**90**) is obtained as a white solid (0.29 g, 82 %).

¹H-NMR (D₂O): □□□□-0.21 (s, 99 H, *Me*Si y *Me*₂Si), 0.22 (m, 30 H, Si*C*H₂), 0.38 (m, 72 H, SiC*H₂*), 1.17 (m, 66 H, C*H₂*), 1.58 (m, 6 H, OCH₂C*H₂*), 2.10 (m, 48 H, C*H₂*N), 2.19 (m, 24 H, C*H₂*N), 2.51 (m, 48 H, C*H*₂CO), 3.42 (s, 6 H, OC*H₂*), 5.64 (s, 3 H, C₆*H₃*); ¹³C-NMR{¹H} (D₂O) □□□-4.5 (*Me*Si), -3.4 (*Me₂*Si), 13.0 (SiCH₂), 13.7 (SiCH₂), 18.4-20.3 (*C*H₂), 32.9 (OCH₂*C*H₂), 34.3 (N*C*H₂), 49.6 (*C*H₂CO), 57.2 (N*C*H₂), 67.2 (O*C*H₂), 93.8 (*C*₆H₃ (*C*H), 168.4 (i-*C*₆H₃), 181.2 (*C*O); ²⁹Si-NMR (D₂O): □□□1.7 (Me*Si* y Me₂*Si*).

### Synthesis of G₃O3(COONa)₄₈ (91).

From G₃O3(COOMe)₄₈ (0.30 g, 0.030 mmol) and NaOH (0.069 g, 1.74 mmol), following the procedure described for the synthesis of **89**, G₃O3(COONa)₄₈ (**91**) is obtained as a white solid (0.24 g, 79 %).

¹H-NMR (D₂O): □□-0.21 (s. a., 207 H, *Me*Si y *Me₂*Si), 0.20-0.40 (m, 222 H, SiC*H₂*), 1.16 (m, 138 H, C*H*₂), 1.58 (m, 6 H, OCH₂C*H₂*), 2.09 (m, 144 H, C*H₂*N), 2.49 (m, 96 H, C*H₂*CO), 3.42 (s, 6 H, OC*H₂*), 5.64 (s, 3 H, C₆*H₃*); ¹³C-NMR{¹H} (D₂O): □□□-4.5 (*Me*Si), -3.4 (*Me*₂Si), 12.9 (Si*C*H₂), 18.0-21.0 (*C*H₂), 34.2 (N*C*H₂), 49.4 (*C*H₂CO), 57.2 (N*C*H₂), 181.1 (*C*O), no se observan los átomos de Carbono OCH₂CH₂CH₂CH₂ ni C₆H₃.

### Synthesis of G₁O3(SO₃Na)₁₂ (92).

A teflon valve ampoule containing G₁O3(NH₂)₆ (0.25 g, 0.18 mmol) and C₂H₃SO₃Na (0.28 g, 2.18 mmol) in an H₂O/EtOH mixture (5 mL / 10 mL) is heated at 120 °C for 48 hours. Then, volatiles are evaporated and the residue is washed first with MeOH (2 x 10 mL) and then with Et₂O (2 x 15 mL), obtaining G₁O3(SO₃Na)₁₂, **92**, as a white solid (0.30 g, 57 %).

¹H-NMR (D₂O): □□□-0.18 (s, 45 H, *Me*Si), 0.26 (m, 18 H, SiC*H₂*), 0.39 (m, 24 H, SiC*H₂*), 1.19 (m, 18 H, C*H₂*), 1.29 (m, 12 H, C*H₂*), 1.47 (m, 6 H, OCH₂C*H₂*), 2.26 (m, 12 H, C*H₂*N), 2.77 (m, 24 H, C*H₂*N), 2.86 (m, 24 H, C*H*₂S), 3.59 (s, 6 H, OC*H₂*), 5.78 (s, 3 H, C₆*H₃*); ¹³C-NMR{¹H} (D₂O): □□□-4.8 (*Me*Si), -3.5 (*Me₂*Si), 12.7 (SiCH₂), 13.7 (SiCH₂), 18.4, 19.9 y 20.2 (*C*H₂), 32.7 (OCH₂*C*H₂), 47.1 (*C*H₂S), 47.8 (*C*H₂N), 57.1 (*C*H₂N), 67.2 (O*C*H₂), 93.7 (*C*₆H₃ (*C*H), 160.6 (*i*-*C₆*H₃); ²⁹Si-NMR (D₂O): □□1.9 (Me*Si*).

### Synthesis of G₂O3(SO₃Na)₂₄ (93).

From G₂O3(NH₂)₁₂ (0.20 g, 0.071 mmol) and C₂H₃SO₃Na (0.13 g, 0.97 mmol), following the procedure described for the synthesis of **92**, G₂O3(SO₃Na)₂₄, **93**, is obtained as a white solid (0.25 g, 60 %).

¹H-NMR (D₂O): □□-0.18 (s, 99 H, *Me*Si y *Me₂*Si), 0.26 (m, 30 H, SiC*H₂*), 0.41 (m, 72 H, SiC*H₂*), 1.25 (m, 66 H, C*H₂*), 1.60 (m, 6 H, OCH₂C*H₂*), 2.27 (m, 24 H, C*H*₂N), 2.75 (m, 48 H, C*H₂*N), 2.86 (m, 48 H, C*H*₂S), 3.45 (s, 6 H, OC*H₂*), 5.78 (s, 3 H, C₆*H₃*); ¹³C-NMR{¹H} (D₂O): □□□-4.6 (*Me*Si), -3.4 (*Me₂*Si), 12.7 (Si*C*H₂), 13.7 (Si*C*H₂), 18.5-20.5 (*C*H₂), 33.0 (OCH₂*C*H₂), 47.0 (N*C*H₂), 47.9 (*C*H₂S), 57.2 (N*C*H₂), 66.8 (O*C*H₂), 93.6 (*C*₆H₃ (*C*H), 160.6 (i-*C₆*H₃); ²⁹Si-NMR (D₂O): □□1.9 (Me*Si* y Me₂*Si*).

### Synthesis of G₃O3(SO₃Na)₄₈ (94).

From G₃O3(NH₂)₂₄ (0.20 g, 0.035 mmol) and C₂H₃SO₃Na (0.12 g, 0.92 mmol), following the procedure described for the synthesis of **92**, G₃O3(SO₃Na)₄₈, **93**, is obtained as a white solid (0.26 g, 63%).

¹H-NMR (D₂O): □□-0.13 (s. a., 207 H, *Me*Si y *Me₂*Si), 0.41 (s. a., 222 H, SiC*H₂*), 1.15-1.60 (s. a., 144 H, C*H*₂), 2.40 (s. a., 48 H, C*H*₂N), 2.90 (s. a., 192 H, C*H₂*N y C*H*₂S), no se observan las señales correspondientes a OC*H₂* y C₆*H*₃); ¹³C-NMR{¹H} (D₂O): □□-4.4 (*Me*Si), -3.3 (*Me₂*Si), 12.5 (Si*C*H₂), 18.0-21.0 (*C*H₂), 46.7 (N*C*H₂), 48.5 (*C*H₂S), 57.2 (N*C*H₂), carbon atoms of OCH₂CH₂CH₂CH₂ or C₆H₃ are not observed.

### EXAMPLE 3: BIOLOGICAL ACTIVITY OF THE INVENTION DENDRIMERS

Below are an assessment of cytotoxicity, mitogenicity and microbicidal / microstatic of new anionic dendrimers synthesized according to the invention in different cell lines (vaginal and lymphoid) in different physiological populations (peripheral blood mononuclear cells, red blood cells. macrophages and dendritic cells) and bacterial cultures.

### Materials and methods

### 1. CELL

**Human endometrial carcinoma cells** (HEC-1A): human endometrial cell line derived from a human adenocarcinoma of the endometrium were obtained from American Type Culture Collection (ATCC). The HEC-1A were maintained in complete medium [RPMI 1640 (Gibco) supplemented with 10% fetal calf serum (SFT), 2 mM L-glutamine and antibiotics (1% cloxacillin, 1% 0.32% ampicillin and gentamicin)] plates 24 or 96-well or 12 well transwell plates with polycarbonate permeable support of 0.4 microns pore (Costar, Cambridge, MA) in culture conditions (37 ° C in an atmosphere of 5% CO₂ and 95% relative humidity.)

**Peripheral blood mononuclear cells (PBMC):** Peripheral blood was obtained from buffy coats from healthy donors from the Transfusion Centre of Madrid. This blood is diluted ½ with phosphate buffered saline 6.7 mM (PBS, Bio-Whittaker ®) and proceeds to density gradient centrifugation (Ficoll-Isopaque ®). Following this centrifugation was recovered halo containing the PBMC and proceeds to two cycles wash-centrifugation with PBS (10 minutes at 1500 rpm). The resulting PBMC were resuspended in complete medium and culture conditions.
**MT-2:** cell line transformed by human T-cell leukemia virus type 1 (HTLV-1). Were obtained from American Type Culture Collection (ATCC). Were cultured in complete medium in different plate formats (6, 24 or 96 wells) and culture conditions.
**Dendritic cells (DC):** We carried out a separation of PBMC by density gradient as described previously. Monocytes were obtained by immunomagnetic separation with anti-human monoclonal antibody CD14 (MicroBeads) by separation columns (MACS ®, Miltenyi Biotec, Germany). The purified monocytes were suspended in complete medium in flat bottom plate of 6 wells at a concentration of 2 * 10⁶ cells / well in a final volume of 3 mL. Were cultured the cells with 50 ng / ml rh GM-CSF and 20 ng / ml rh IL-4 (ImmunoTools, Germany) in culture conditions. Half pot of complte fresh medium was added with 50 ng / ml rh GM-CSF and 20 ng / ml rh IL-4 at 3 days to immature cells (CDi). Matured cells at 6 days were treated with 20 ng / ml LPS (Sigma) for 2 days (CDM). The CDI and CDM markers were analyzed using a Beckman-Coulter cytometer ® 4-color and used the EPICS ® software for Beckman-Coulter ®.
**Macrophages:** separation of PBMC was carried out by density gradient and immunomagnetic separation with anti-human monoclonal antibody CD14 (MicroBeads) as described previously The purified monocytes were resuspended in RPMI 1640 (Gibco) supplemented with 10% human serum AB, 2 mM L-glutamine and antibiotics (1% cloxaciclin, 1% ampicillin and 0.32% gentamicin) in flat bottom plate of 6 wells at a concentration of 2 * 10⁶ cells / well in a final volume of 3 mL. Were cultured the cells with 50 ng / ml rh M-CSF and fresh medium was added with 50 ng / ml rh M-CSF 3 days later. At 7 days, macrophage markers were analyzed using a Beckman-Coulter cytometer ® 4-color and used the EPICS ® software for Beckman-Coulter ®.

**T cell line Jurkat E6 clone** (ATCC; T lymphocyte cell line) was grown routinely in RPMI 1640 (Biochrom KG Seromed, Berlin, Germany) supplemented with 5% fetal calf serum heat inactivated (FCS), 1% penicillin/streptomycin, and 2mM L-glutamine (ICN Pharmaceuticals, Costa Mesa, CA, USA) at 37 ° C in an atmosphere of 5% CO₂.

### 2. DENDRIMERS

Synthesis of carbosilane dendrimers with sulfonate (G1SF8, G2SF16 and G3SF32) and carboxylate terminal groups (G1COO8, G2COO16, G3COO32) was described in the previous section (see pages 15 to 19). In addition to dendrimers and G2CKP32 G2CKCOO16 (see pages 21 to 23).

Dendrimers G1SF8, G2SF16, G3SF32 corresponding to the dendrimers G0-[Si(CH₂)₃N(CH₂CH₂SO₃Na)₂]₄ n = 0 and x = 4 **(18)**; G1-[Si(CH₂)₃N(CH₂CH₂SO₃Na)₂]₈ n =1 and x = 8 **(19)**; G2-[Si(CH₂)₃N(CH₂CH₂SO₃Na)₂]₁₆ n =2 and x = 16 **(20)**, respectively, whose synthesis and characterization has been described in previous sections. The same form, dendrimers G1COO8, G3COO32 G2COO16 corresponding to the dendrimers G0-[Si(CH₂)₃N(CH₂CH₂COONa)₂]₄ n=0 and x = 4 **(10)**; G1-[Si(CH₂)₃N(CH₂CH₂COONa)₂]₈ n =1 and x = 8 **(11)**; G2-[Si(CH₂)₃N(CH₂CH₂COONa)₂]₁₆ n =2 and x = 16 **(12)**, respectively. The same manner G2CKCOO16 and G2CKP32 dendrimers, corresponding to ck- *ck*-G1-[Si(CH₂)₄(N₃C₂H)CH₂N(CH₂CH₂COONa)₂]₈ **(43)** y *ck*-G1-[Si(CH₂)₄(N₃C₂H)CH₂N(CH₂PO(OMe)₂)₂]₈ **(51)**, respectively

Synthesis of carbosilane dendrimers with sulfonate terminal groups (G103SF12, G2O3SF24 and G203SF48) was described in the previous section. These dendrimers G1O3SF12, G203SF48 G2O3SF24 corresponding to G₁O3(SO₃Na)₁₂ **(92)**, G₂O3(SO₃Na)₂₄ **(93)** y G₃O3(SO₃Na)₄₈ **(94)** that have 12, 24 and 48 terminal sulfonate groups.

This new nomenclature for this part of the present invention has been taken to simplify the representation of these dendrimers in biomedical research and to facilitate follow up this discussion of results.

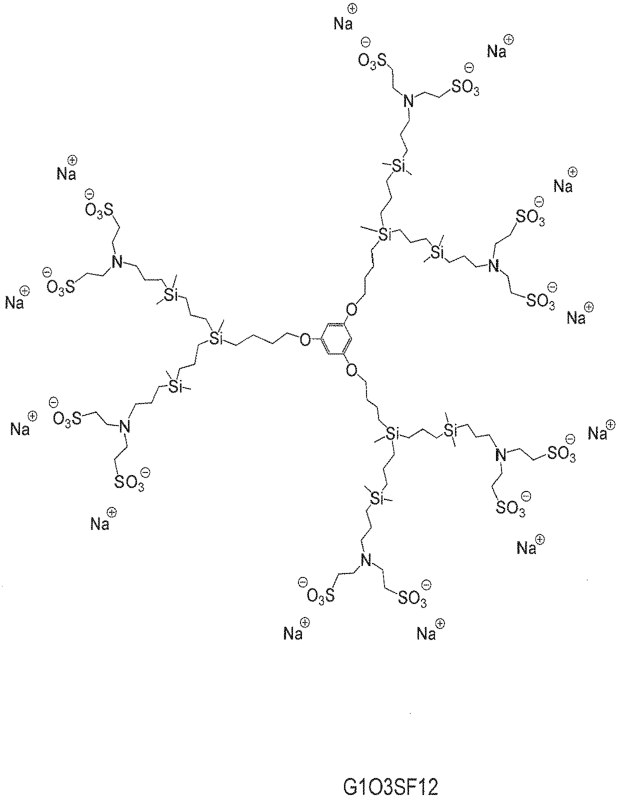

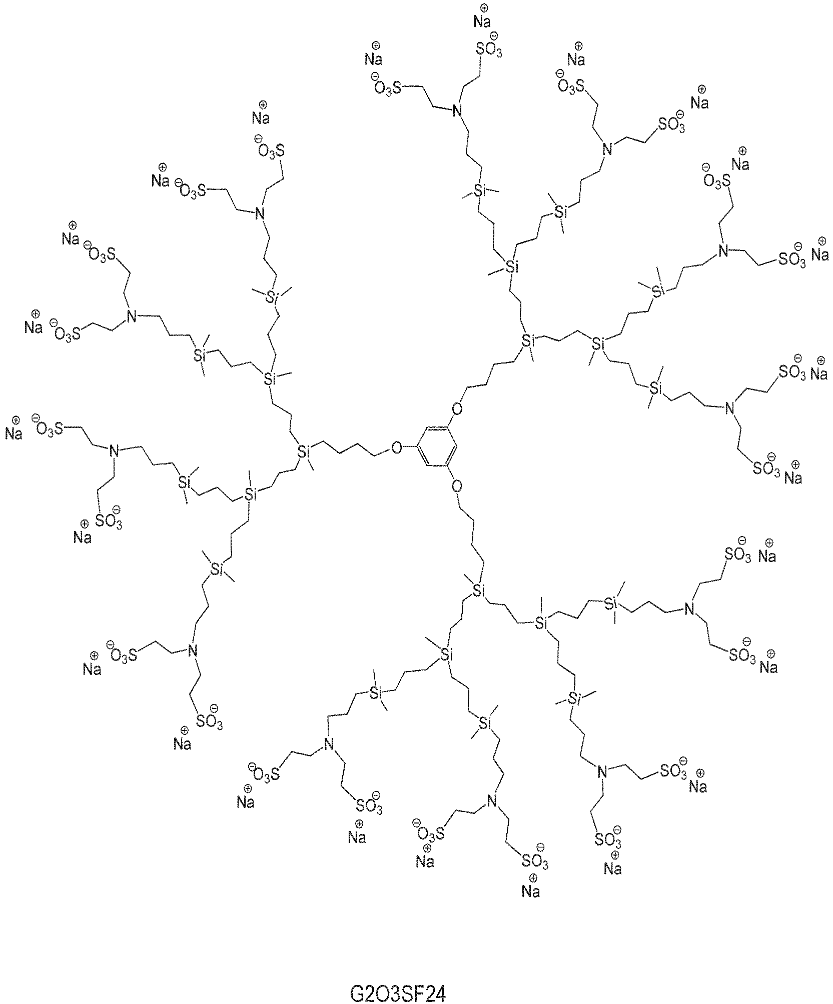

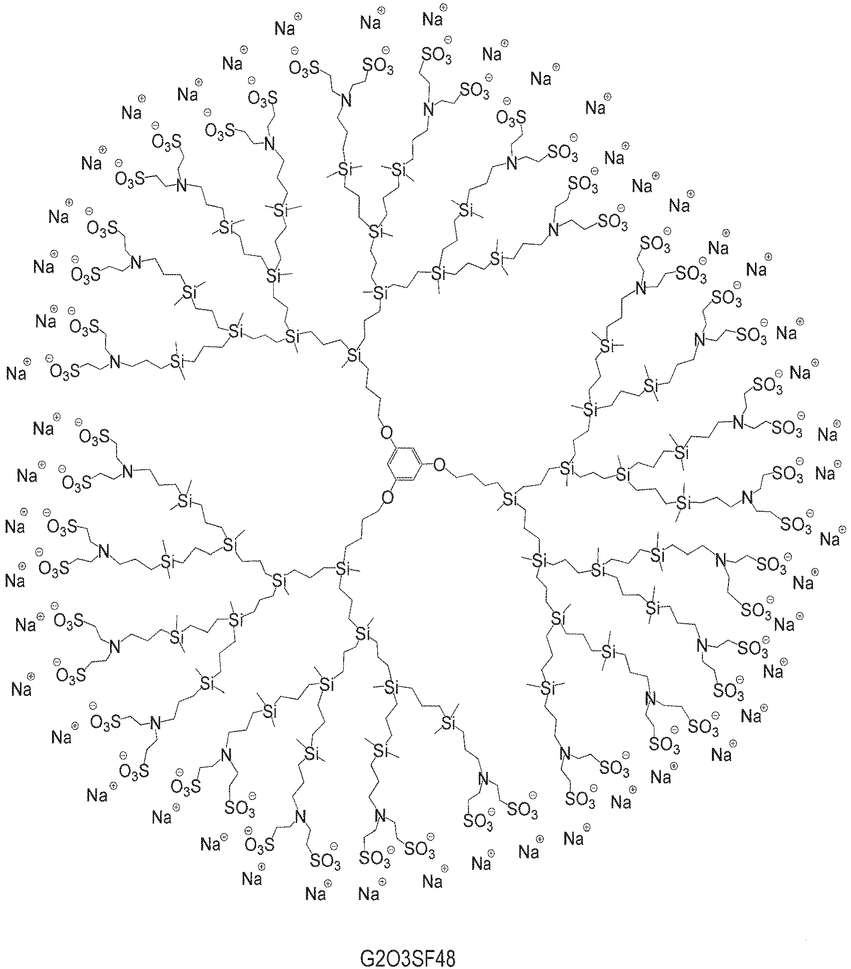

### 3. EVALUATION OF TOXICITY

Different techniques were used to test the viability in different aspects: test membrane rupture (trypan blue staining and LDH assay), mitochondrial activity (MTT reagent) and cell proliferation (BrdU incorporation). These techniques were applied to study the toxicity of the generations of dendrimers in HEC-1A and human PBMC. Additionally, the toxicity of dendrimers on human red blood cells has studied using a test for the presence of hemagglutination and hemolysis.

### 3.1 Staining with trypan blue

A solution with trypan blue (Sigma^{®}) to 0.8% was prepared. Cells treated with dendrimers were centrifuged and the supernatant was decanted, after resuspending the cell pellet was treated with trypan blue solution for 30 seconds, then the cell pellet was centrifuged and washed with PBS twice. The cells were observed under light microscopy and were counted positive for the presence of trypan blue (blue cells, dead) in relation to the percentage of negative cells (live cells). We selected a large field at least 100 cells each time.

### 3.2. MTT Assays.

This method was selected to analyze detrimental intracellular effects on mitochondria and metabolic activity. The colorimetric MTT test, based on the selective ability of viable cells to reduce MTT to purple formazan, relies on intact metabolic activity and is frequently used for cytotoxicity screening. After the desired time of incubation of the different cells with different concentrations of dendrimers in 96-well plate (100,000 cells / well respectively), and 3 wells as a positive control of cell inactivity [20% dimethyl sulfoxide (DMSO, Sigma^{®})], the supernatant that containing dendrimer was removed and replaced with 200 mL culture medium without serum or phenol red (Opti-MEM^{®}). In addition to the 200 µL of Opti-MEM^{®} , 20 mL of MTT previously filtered to achieve sterility (thiazolyl blue, Sigma^{®}) was added in PBS at a concentration of 5 mg / mL (final concentration of 0.5 well mg MTT / mL). After 4 hours incubation in culture conditions, we proceeded to centrifuge the plate at 2000 rpm 10 minutes and the subsequent removal of the supernatant with excess unreacted MTT. The concentration of formazan was then determined spectrophotometrically in a plate reader at a wavelength of 570 nm (test) and 690 nm (reference). The spectrophotometer was calibrated to zero absorbance by using Optimem medium without cells. The percentage cell viability relative to control wells (cells with no dendrimer) was calculated by ([A]test/[A]control)T100. Each dendrimer concentration was tested in triplicate, according to American Type Culture Collection (ATCC) directives.

### 3.3. LDH assays

This technique (CytoTox 96 ® Non-Radioactive Cytotoxicity Assay) was used to test the damage in the cell membrane surface due to the presence of anionic peripheral groups of dendrimers. The lactate dehydrogenase (LDH) is an enzyme that is released into the cytoplasm due to cell lysis. The LDH assay, therefore, is a measure of membrane integrity, which is based on the oxidation of LDH to pyruvate and reduction of tetrazolium salts to formazan crystals by pyruvate.
1 * 10⁵ cells / well were dispensed in 96-well plate round bottom in a volume of 100 □ L of complete medium and the dendrimer was added in a volume of 100 □ L, to obtain a final volume of 200 □ L in the well. Controls: 1. 200 □ L of complete medium without cells (background control), 2. 100 □ L of cells + 100 □ L of complete medium, 3. 100 □□L of cells + 100 □ L of Triton X-100 0.2% 4. 100 □ L of cells + 100 □ L with the dendrimer to the desired concentration. Incubated in culture conditions during the first 24h.
Plate was centrifuged at 1500 rpm 10 minutes and removed 100 □ L of supernatant, being careful not to take the cells. 100 □ L of reaction mixture prepared at the time on the 100 □ L was added of supernatant and incubated at room temperature for 30 minutes in darkness. Finally, measured at 490 nm and 690 nm reference. The cytotoxicity was obtained from the following formula: Cytotoxicity (%) = (4 - 2) / (3-2) * 100

### 3.4 HEMOLYSIS TEST

Red blood cells were separated from the PBMC by Ficoll gradient the same form that described previously. They were diluted appropriately in PBS until they can be viewed individually. Then the red blood cells were resuspended in 500 L of PBS and cultured in 24-well plate (300,000 / well). As positive control the cells were treated with Triton X-100 0.2%. Negative control: cells were treated only with PBS. RBCs were incubated with different concentrations of dendrimer. We evaluated the presence of hemagglutination, cell number and hemoglobin release at the time by collecting 100 µL of supernatant and measurement of absorbance by spectrophotometer using a plate reader

### 4.- TEST LYMPHOPROLIFERATIVE

A cell proliferation assay we performed in order to evaluate the mitogenicity of the dendrimers. Experiment was prepared in triplicate in 96-well flat-bottomed (100,000 cells per well in 200 µL of complete medium). The experiment included a negative control of proliferation (untreated cells), wells treated with different concentrations of dendrimer and a positive control of proliferation treated with 2 mg / mL of phytohemagglutinin (PHA). After 4 days of incubation in culture conditions, we proceeded to the removal of the supernatant and the sample was prepared for cell kit proliferation BrdU (Bromodeoxyuridine Cell Proliferation Assay, Chemicon International). It was performed as indicated by the kit protocol and obtained the incorporation of BrdU into newly synthesized DNA. A greater incorporation of BrdU increased cell proliferation.

### 5.- INHIBITION ASSAYS OF HIV

### 5.1 Preparation of X4 HIV-1NL4-3 strain.

The isolated viral X4 HIV-1_{NL4-3} is a laboratory viral standard strain and MT-2 cells were used for virus spread. 2 x 10⁶ MT-2 cells were washed and were transferred to a 15 mL conical tube with a concentration of 2 x 10⁶ cells/mL in complete medium. Next, HIV-1_{NL4-3} was added to a concentration of 1 particle per cell or what is the same, 1 M.O.I. (Multiplicity Of Infection). The MT-2 cells were cultivated with the virus for 2 hours in culture conditions, shaking it every 15-30 minutes. Finally, the cell culture (cells-virus) were washed twice to remove the virus not integrated into the cellular genome. The cells were transferred to a p6 well in a volume of 3-4 mL and were left cultivating during 2-3 days observing the presence of syncytium in the well. When the presence of syncytium reached 80-90% of production, 12 mL of complete medium containing 20 * 10⁶ MT-2 were added and they dispensed in petri plate. 2-3 days after, the volume is centrifugated and the supernatants were collected. Added 12 mL of complete medium with 20*10⁶ MT-2 cells to previous cells (infected MT-2) and they were dispensed in a petri plate. This process was repeated up to 3 times. The supernatants was removed and stored in a liquid nitrogen tank, being later titrated.

### 5.2. Preparation of R5 HIV-1Ba-L strain.

The isolated viral R5 HIV-1_{Ba-L} is a standard laboratory viral strain and the process of virus spread was carried out in a similar manner to that of HIV-1_{NL4-3}, but in this case stimulated PBMC was infected. The supernatant was removed and stored in a liquid nitrogen tank, being later titrated.

### 5.3. Virus titration.

The isolated virals HIV-1_{NL4-3} and HIV-2_{CBL23} were titrated in the MT-2 cell line. 2x10⁴ MT-2 cells were cultivated with complete medium in 96 well plates and 40 µL of viral preparations at different concentrations were added, using adequate dilutions. The dilutions were arranged by octuplicate and remained under culture conditions for a week. Afterward, titration value was obtained by visualization of the cytopathic effect. The titration was calculated applying Spearman-Karber equation. Also, titration was quantified by p24 protein enzymatic immunoassay (ELISA p24. INNOTEST™ HIV antigen mAB, Innogenetics®) and was established particles per mL and µg of virus by mL relationship. The HIV-1_{Ba-L} isolated viral was titrated by p24 protein quantification by ELISA test. To ensure virus purity, the thawed aliquots were filtered through 0.22 µm filters before quantification.

### 5.4. In vitro infection of different cell populations.

The PBMC were stimulated for 48 hours with 2 µg/mL of PHA and 50 UI of IL-2 to induce a polyclonal activation. Afterward, the cells were washed with PBS. The desired concentration of cells were incubated with the desired number of HIV particles for 2 hours in complete medium in culture conditions. After this time the cells of the cell culture were collected and washed three times with PBS to eliminate the virus not integrated into the cellular genome, after which were deposited in 24 wells or 96 wells plate (depending on the test) in complete medium with 50 IU/mL of IL-2. MT-2, HEC-1A, ILC, CDm and macrophages were collected the day of the experiment and were washed twice with PBS prior to be infected with HIV during 2 or 3 hours, depending on the experiment.

### 5.5. Adherence/internalization assays.

For these tests confluent HEC-1A were used in 24 well plates. 3*10⁵ cells were cultivated in 500 µL of well complete medium for 3 days. In the adherence assays, cells were pretreated with the dendrimers fo 2 h and 1 h and 30 prior to infection with HIV-1_{NL4-3}. Then, each well was infected with HIV-1_{NL4-3}3 for 3 hours. Afterward, the monolayer was washed three times with PBS and left in culture conditions. After 24 h and 72 h, the he supernatant was quantified by p24 antigen by ELISA. For the internalization assays, the initial procedure was previously specified. After 3 hours of infection, the monolayer was washed 3 times with PBS and cellular lysis was provoked by addition of Triton x-100 0.2% for 45 minutes at 37 ° C. The supernatant was collected to quantify p24 antigen by ELISA.

### 5.6. Action mechanism of carbososilane dendrimers.

It was used for this experiment a 96 well plate of flat bottom with Poli-L-lysine (PLL, Sigma^{®}) covering the bottom of the well. For this purpose, 50 uL of PLL were incubated at 37 ° C for one hour. After washing the wells three times with PBS, 4 ng of HIV were incubated overnight at 4 ° C, to ensure that the virus particles were adhered to the bottom of the well. Each well was washed three times with PBS to remove HIV not adhered to the plate and then each well was treated with different dendrimer concentratios for 1 h. Afterward, it was necessary to washed the product with PBS and PBMC previously stimulated were added. The supernatant of this cell culture was recovered after 4 days and p24 antigen was quantified by ELISA to assess the amount of HIV produced by the PBMC in different treatments. As positive control of lysis of the viral particles Triton x-100 (1%) was used.

### 5.7. HEC-1A monolayer.

To obtain a perfect monolayer of polarized epithelial cells, the HEC-1A were grown in complete medium on a permeable polycarbonate support of 0.4 µm pore (Costar, Cambridge, MA). Then, 2*10⁵ cells by well were cultivated for 7 days, with media changes every 2-3 days. The dendrimer were added one hour prior to the infection. After adding the viral particles to the upper chamber of the monolayer (100ng), the supernatant were collected from the lower chamber after 24/48 hours. The viral concentration was quantified by ELISA.

### 5.8. PBMC pre-treatment.

We used 2*10⁵ stimulated PBMC cells dispensed in 200 µL of complete medium in round bottom wells of p96 plate. For the pretreatment experiment, 50 ng of HIV-1_{NL4-3} were treated with dendrimer 1 hour prior to infection and then the cells were infected for 3 hours in culture conditions. After this time, the wells were washed twice with PBS and were incubated in culture conditions. The viral concentration after 3 days was quantified by ELISA of p24.

### 5.9. CDi, CDm and macrophages pre-treatment

Once the cells were differentiated, they were washed twice with PBS and 2*10⁵ CDi, 2*10⁵ CDm and 10⁵ macrophages were dispensed in 200 µL of complete medium in bottom p96 wells. The dendrimer were added one hour prior to the infection. After adding the viral particles (50 ng), they were incubated in culture conditions and after 3 hours the supernatant was retired and washed 4 times with PBS. The supernatant was collected after 4 days. The viral concentration was quantified by ELISA.

### 6. BACTERICIDAL/BACTERIOSTATIC EFFECT OF THE CARBOSILANO DENDRIMERS

### Staphylococcus aureus. Strain and culture conditions.

FDA209 (ATCC 6538) strain was used to study the ability of the dendrimers as bactericides. Bacteria were grown in Mueller Hinton agar plates (Sigma™) overnight at 37 °C prior to each experiment.

Experiment of biomass: the day of the experiment, a colony of *S. aureus* were grown in 15 mL of Mueller Hinton medium for 5 hours. After this time, the number of *S.aureus* were counted and 10⁵ *S.aureus* were seeded by point (100 µL of Mueller Hinton medium in 96 wells plate of U bottom). Serial dilutions were added of the dendrimers up to a total volume of 200 µL per point. Ampicillin (Gobemicina^{®}, Normon laboratories) were used as a positive bactericide control. The amount of biomass formed after 24 h of dendrimer treatment was quantified spectrophotometric with a plate reader (Biowhittakermicroplate reader 2001, Innogenetics®. λ=550nm).

Experiment of proliferation: To evaluate *S.aureus* proliferation in the presence of the dendrimer, the cell proliferation kit was used 24 h after treatment (Bromodeoxyuridine Cell Proliferation Assay, Chemicon International. Biowhittakermicroplate reader 2001, Innogenetics®. λ=450 and reference λ=550nm). The number of cells, dendrimer treatment and exposure time are described in the biomass experiment.

### Candida albicans. Strain and culture conditions.

The isolated clinical SC5314 (ATCC MYA-2876) was used to study the ability of dendrimers as fungicides. The yeasts were grown in YED agar plates (1% d-glucose, 1% Difco extract of yeast (BD Diagnostic Systems, USA) and 2% agar) overnight at 30°C prior to each experiment.

Experiment of biomass: the day of the experimentl, an isolated colony from yeast were grown in 15 mL of YED agar medium for 5 hours. After this time, the number of *C*. *albicans* was counted and 10⁵ were seeded by point (100 µL of medium YED agar plate of 96 wells of U bottom). Serial dilutions of the evaluated dendrimers were added for a total volume of 200 µL by point. Amphotericin B (Duchefa Biochemie) was used as positive control of fungicide. The amount of biomass formed 24 h after treatment with the dendrimer was spectrophotometric quantified with a plate reader (Biowhittakermicroplate reader 2001, Innogenetics ®. λ=550nm).

Experiment of proliferation: To evaluate proliferation of *C*. *albicans* in the presence of the dendrimer the cell proliferation kit was used 3 h before the 24 hours of treatment were completed (Bromodeoxyuridine Cell Proliferation Assay, Chemicon International. Biowhittakermicroplate reader 2001, Innogenetics®. λ=450 y referencia λ=550nm), studying in this way incorporation of BrdU to the DNA of new synthesis. The cell number, dendrimer treatment and exposure time are described in the experiment of biomass.

### 7. ANTIINFLAMATORY EFFECT OF CARBOSILANE DENDRIMERS.

Inflammation experiments: the experiments were conducted with transient transfection using plasmid indicators pTNF-luc and pNF-κB-luc containing 3 copies in tandem of site NF-κB of the promoter. The Jurkat cell line was nucleofected with the AMAXA nucleofector following the commercial house protocol "cell line nucleofector kit V" and the specific programme used in the X-005. Transfected cells were stimulated for 16 hours with the different stimuli that appear in the graphs (TNF-alpha, antibody monoclonal anti-TNF-alpha and different concentrations (5 and 10 micromolar) of the anionic sufonate dendrimer). After different treatments, the transfected cells were collected by centrifugation and lysed with commercial tampon lysis "Dual-luciferase Assay System Kit" (Promega), according to the manufacturer's recommendations. The amount of protein in 5 µl of lysed cell was measured by the method of BSA-BCA (Pierce, Rockford, USA). Once quantified, the values of all the samples were normalized to 20 micrograms to measure the lucifierase activity for about 10 seconds in a luminometer 1450 Microbeta Luminiscence Counter (Walax, Trilux). Luciferasa activity of firefly values are expressed as induction with respect to cells control.

7.1 Transfection experiment with TNF-alpha (proinflammatory cytokine) The Jurkat E6 clone cells transfected with the pTNF-alpha were treated with a monoclonal antibody anti-TNF as positive control of the TNF transcriptional activity inhibition. The sulfonate dendrimer was able to reduce the promoter activity of the pTNF-luc plasmid at 5 and 10 micromolar doses (two to three times lower with respect to the control of cells without stimulating in three independent experiments). The anionic dendrimer decreases the transcriptional activity of TNF-alpha.

7.2 Transfection experiment with plasmid pNF-kB-luc. Jurkat cells were transfected with the pNF-kB-luc plasmid, they were cultivated in the presence of sulfonate (10 micromolar) for 16 hours and the luciferasa activity was measured. TNF (20 ng/ml) was used as a positive control. The experiment clearly showed a decrease in the activity of the pNF-kB-luc plasmid in the presence of sulfonate, similar to that obtained when the cells are treated with 10 micrograms ml of an antibody anti-TNF-alpha. In addition, when the cells were treated with sulfonate+TNF-alpha a reversal of the induction of NF-kB-luc was observed. The results presented are expressed as standardized induction index with the values of cell controls without treatment control and it is a representative of three independent experiments.

### Results obtained with dendrimers of silicium atom core:

### Dendrimer citotoxicity

To perform a complete characterization of three generations of each new synthesized dendrimer, a screening system was drawn up to determine biocompatible concentrations requiring a more detailed study. First of all, the boundaries of solubility were set for each generation in water. Dendrimers were solved to 3 mM, 2 mM and 1 mM concentration, the last presented better solubility without the help of additional physical factors for its perfect solubilization (vortex, heat, etc). Once the starting concentration was set for all tested dendrimers, their cytotoxicity were studied in the target cells, the vaginal epithelial cells (HEC-1A) and the PBMC, at increasing concentrations of dendrimers. The number of dead cells were counted after 24 hours with trypan blue technique.

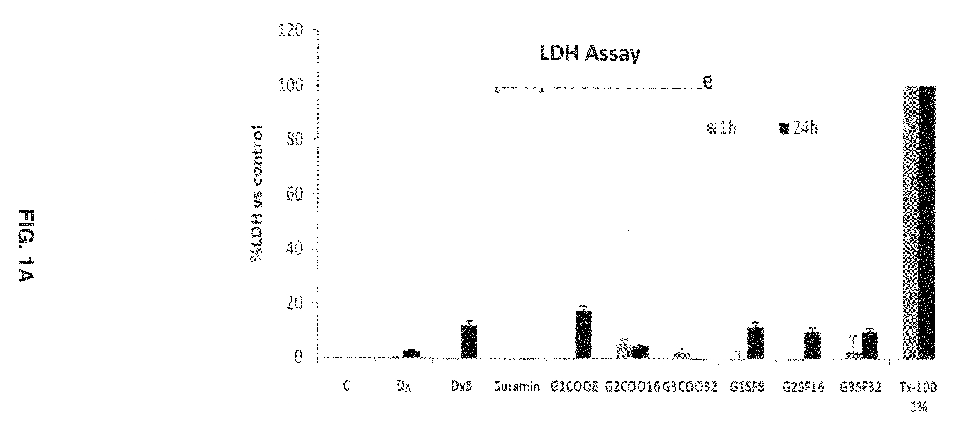

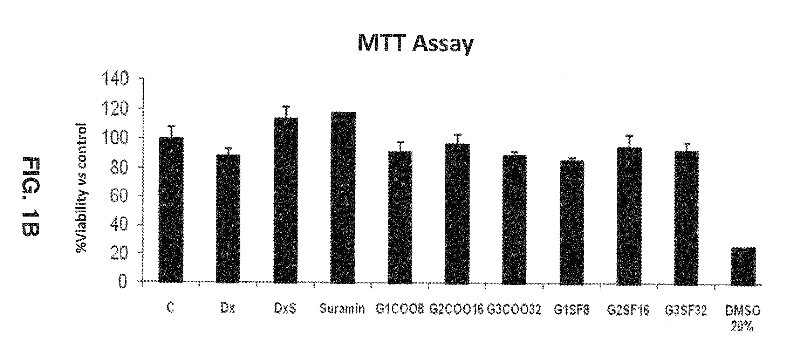

The cells showed blue dye exceeding the control from 20 µM of dendrimer, so it was decided to focus feasibility studies on a certain concentration, 10 µM. To assess the damage to the cell membrane that can cause the periphery anionic charges (forming pores due to interaction with various cell receptors or by electrostatic interaction with positive areas of the membrane), liberation test of lactate dehydrogenase in the supernatant were carried out. These studies were supplemented with assays of cell activity of the mitochondria, where the reduction of tetrazolium to formazan salt crystals is an indirect reflection of the correct metabolism of cells. See the figure 1, where we evaluated initially 3 generations of the two types of dendrimers with silicon core, obtaining acceptable levels of biocompatibility and always within the margins of acceptability for both tests (not more than 10% of LDH in supernatant and no more than a reduction of 20% in cellular activity).

This experiment was carried out in cells where the dendrimers will be longer exposed, in the epithelial cells of the vagine endometrium (HEC-1A). A good viability of the cell membrane to short time can be stablished by the LDH test, since no dendrimer produces a release of more than 10% LDH after an hour of contact with the cells. Suramin, a molecule made up of 6 naphthalene disulfonic acid groups and a urea core, did not show toxicity despite its high concentration. At the 24 hours of treatment, control of cells treated with an inert molecule such as dextran (Dx), showed nearly 10% concentration of LDH in the supernatant with regard to the control. Dextran with sulfonate groups in the periphery (DxS) showed similar toxicity. In this way, 10% of LDH was considered acceptable at 24 hours because the presence of a foreign molecule seems to be causing a slight disturbance in the cells. Similar results with the carboxylate and sulfonate dendrimers were obtained, although it was noted that the second and third generation of carboxylate showed no significant values of LDH.

The cell viability was established at the same time quantifying the reduction of tetrazolium salts (MTT) by the mitochondria of the HEC-1A treated 24 h with the dendrimers. Production levels of formazan crystals were obtained above 80% and it was determined that the concentration of 10 µM of dendrimer is biocompatible with cellular metabolism of the HEC-1A.

To extend this study, an experiment was designed to evaluate the cell damage that these dendrimers could cause in a therapeutic model of infection treatment, as well as the possibility to prevent the infection of the PBMC (CD4 + cells) which are among the stratified squamous epithelium of the vaginal epithelium and under it.

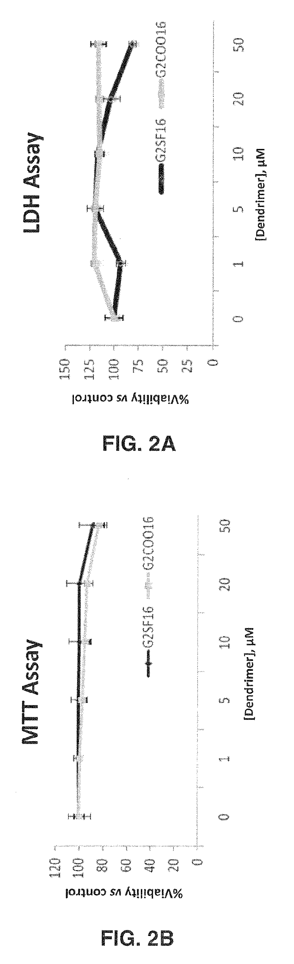

To reproduce toxicity more physiologically, toxicity in PBMC were evaluated. The feasibility here is reduced in all cases and the most visible point of tipping is more visible from 10 µM. Please refer to the figure 2.

A good integrity of the membrane after 24 hours was observed with respect to the control. Other factors can change the reading by spectrophotometry, and this slight increase in viability was accepted owing to the presence of the dendrimer. The LDH release positive control, the Tx-100 at 0.2%, worked in both experiments and represented 0% viability vs. control. The toxicity was presented from 10 µM, except for the dendrimer with termination carboxylate. Mitochondrial activity was stable in the concentration of 10 µM. The low damage that produced to the membrane of physiological cell as the PBMC, together with the optimal mitochondrial activity of cells in the presence of a concentration of 10 µM, determined that the G2SF16 were selected to develop inhibition test of the virus-cell adherence, internalization and infection of the HIV in epithelial cells and PBMC. To see the damage that this dendrimer produces at working concentration, red blood cells were faced with different concentrations of dendrimer.

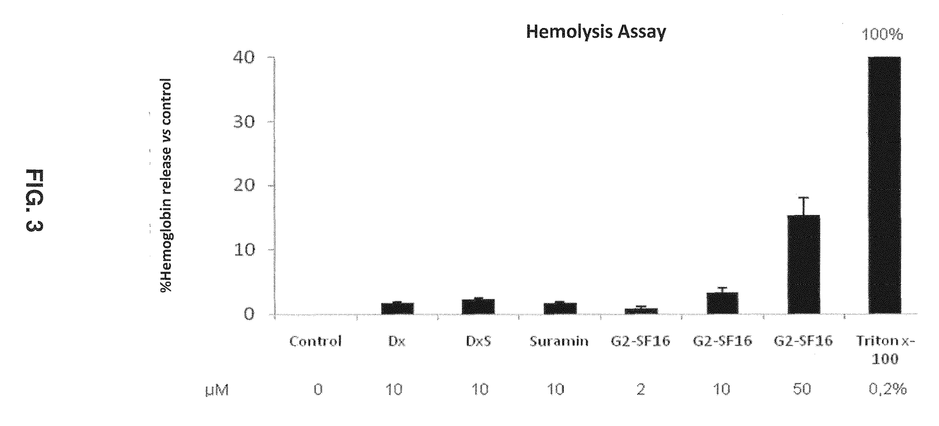

There was 100% hemolysis in red blood cells treated with Tritón X-100 0.2% (cells were all killed and broken) and 5% of hemolysis in the negative control of cells treated only with PBS. Each well is expressed as the percentage with respect to D.O (optical density) of Triton X-100 at 0.2%, which is considered to be 100%. Furthermore to that percentage will be subtracted 5% of hemolysis of negative control of not treated cells, finding a good biocompatibility to 10 µM, as well as a lack of hemagglutination in the optical microscope. See the figure 3.

### Lymphoproliferative

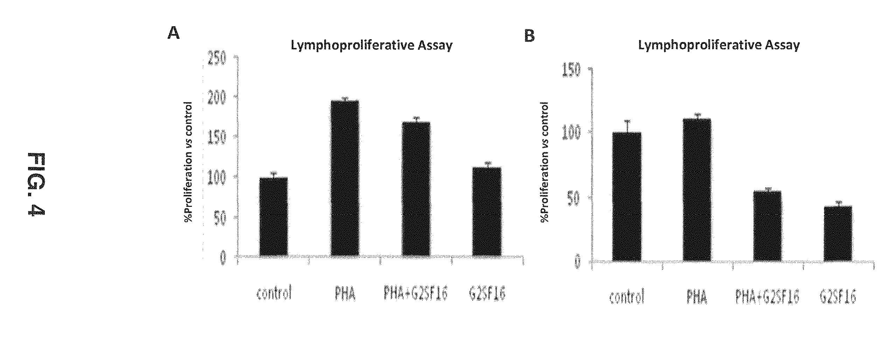

It is very important when you want to know the biocompatibility profile of a new molecule, whether it can be a nonspecific antigenic stimulus. This would be a drawback, since the cells of the immune system may recognize the molecule as a foreign element which trigger a response that will most likely be deleterious for the organism. Therefore, a study of lymphocyte proliferation in the presence of different dendrimers was performed, in order to compare the ability of lymphocyte stimulation of such systems in contrast to a classical mitogenic stimulus such as the fitohematoglutinin (PHA). PBMC were cultured in p96, and treated with different stimuli for 4 days. We used the dendrimer concentration of 10 □M, selected for all experiments with HIV infection.
The results are expressed as percentage of BrdU incorporated into cells that have proliferated compared to control. In FIG. 4A, there was an increase of BrdU incorporation in the new DNA synthesized by the dividing cells, due to the presence of PHA. The dendrimer alone at concentration of 10 mM has no mitogenic effect on PBMC. Dendrimer treatment at the same time that PHA, slightly decreases the mitogenic effect. To check whether the dendrimer G2SF16 delayed cell proliferation in the presence of a mitogen, in FIG. 4B were used PBMC previously stimulated with PHA for 3 days. On the fourth day, cells were treated with equal amounts of PHA, PHA and G2SF16 and G2SF16 alone, founding a decrease in BrdU incorporation after 4 days of treatment. This indicates that the dendrimer is inhibiting mitogenic stimulation, an interesting finding in the study of local inflammation at the time of using this type of dendrimers in vivo. See FIG. 4.

### Testing of adhesion / internalization.

Dendrimers that were exposed on the monolayer of epithelial cells were shown to be no toxic or nor mitogenic at a concentration of 10 □M. For a possible use as a microbicide, it was decided to evaluate the ability to interact with the virus in the process of its accession to the surface of the cell membrane. The dendrimer molecule would act as a physical barrier in preventing HIV infection of the endometrial cells and its passage into the cavity of the uterus.

To evaluate the effectiveness of impairment of cell surface adhesion and the ability to slow down the internalization of the virus in HEC-1A, we designed an experiment with the second generation dendrimers with carboxylate and sulfonate groups as terminal units. The idea of a topical application of this molecule makes necessary to evaluate whether treatment times prior to infection (pretreatment) are limiting for efficacy.

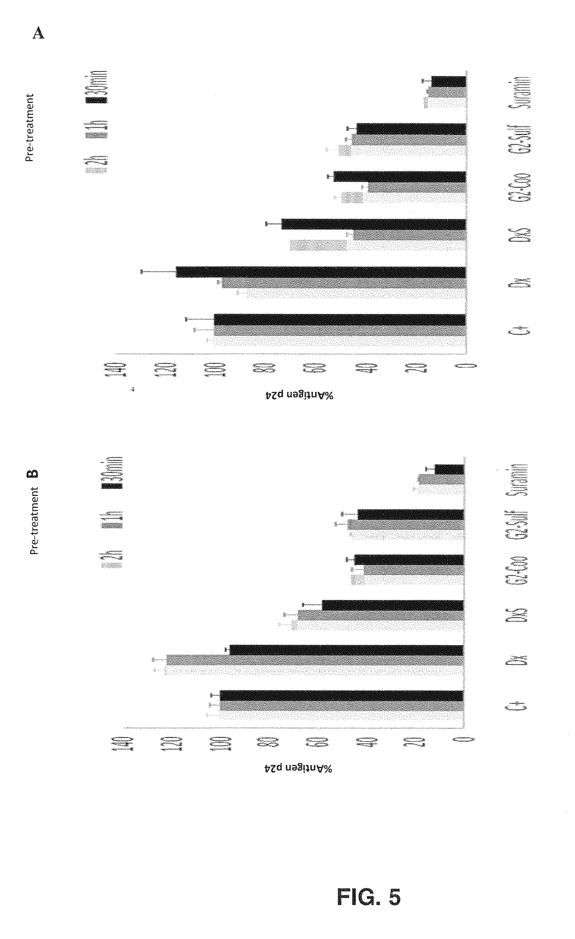

The graph below shows how the pre-treatment of HEC-1A at different times does not significantly alter the effectiveness of 60% inhibition of adherence for HIV-1 NL4.3. This data was verified by collecting the culture supernatants at two different times after infection, pretreatment is for 30 min, 1 hour and 2 hours, then infected with HIV and cultivation remains for 24 hours (Fig. 5A), supernatant collected and measured and infected Agp24 for 72 hours (Fig. 5B), the supernatant is collected and the Agp24 quantified. Suramin was selected as the positive inhibition control of adhesion as it has demonstrated its electrostatic binding to the gp120 V3 region of HIV, preventing that the infection machine works properly. p24 represents the p24 antigen (viral genetic material) that is detected in the supernatant of cell cultures using an ELISA kit (INNOTESTTM HIV antigen mAb, Innogenetics ®). See FIG. 5.

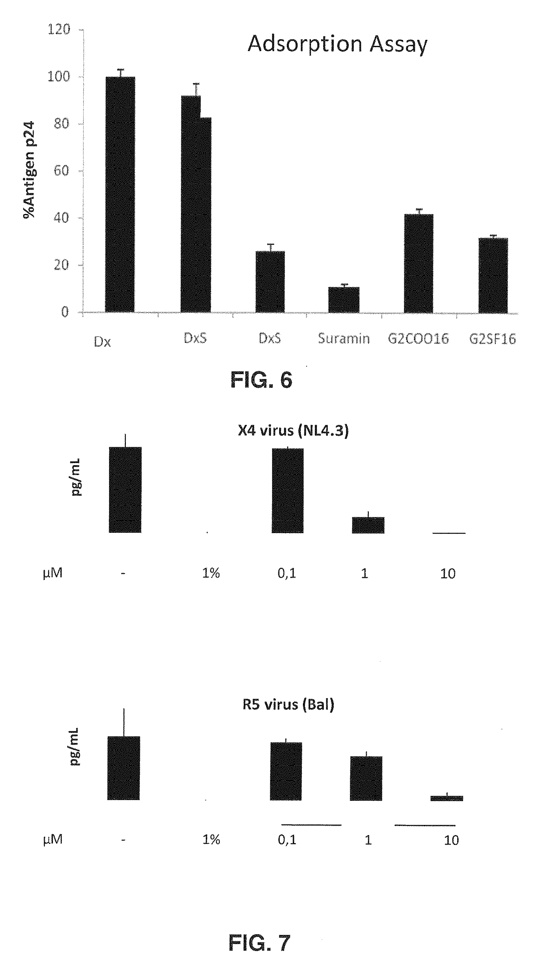

The following experiment was performed to quantify the number of virus particles that were inside the cells previously treated with dendrimer. It was found that during the 3 h of infection, the amount of virus detected by ELISA was 70% lower than in control cells, confirming the previous data and strengthens the theory that the polyanionic dendrimer is acting as a barrier on the cells before viral attack. A concentration of 10 □M was used for all reagents. See FIG. 6 .

### Mechanisms of action.

After verifying the effectiveness of the dendrimer to block adherence and internalization of virus particles, we proceeded to try to understand the mechanism of action. To this end, we designed an experiment that consisted of exposing the viral particles to the presence of dendrimer, to then use this combination in stimulated PBMC cultures (prepared for the viral infection). We used HIV-1 NL4.3 (X4 tropic) and HIV-1 BaL (R5 tropic), to analyze the effect that would have the polyanionic dendrimer with the V3 of gp120 of both viral types, and whether the use of a different co-receptor may vary the efficiency of inhibition. The results of ELISA quantification of p24 at 4 days of culture, show a low prevalence of HIV in the supernatant of PBMC treated with 10 □M G2SF16 dendrimer. This translates into an encapsulating or interfering ability of the dendrimer with the normal functioning of HIV to infect the PBMCs. See FIG. 7.

### Inhibition assays.

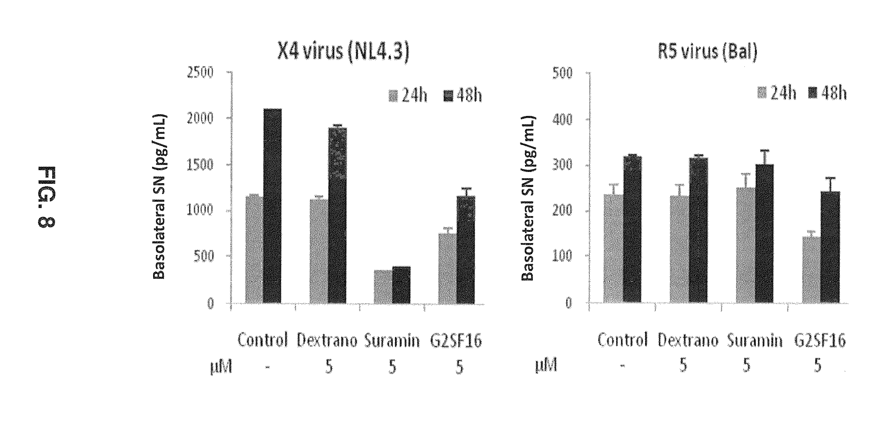

To reproduce in the best possible way the role that the anionic dendrimer develop on the surface of the endometrium and to study the passage of virions through the mucosal stratified vaginal epithelium, we used transwell devices that recreate the phenomenon of transcytosis (transport of macromolecules from the extracellular space to another through the cytoplasm of a cell via an endocytic vesicle) due to the possibility of forming a perfect monolayer of adherent cells on the inside and collect information from the supernatants of the apical and basolateral side of the monolayer. The following graphic shows the effect of dendrimer on the passage of viral particles through the monolayer.
This effect was reduced by 40% in both cases, but as a relevant data, to indicate that in the case of R5-tropic virus, our positive control (suramin) has no inhibitory effect. Therefore, the internal structure of the dendrimer is involved in the antiviral capacity of the molecule (see Fig. 8). On the monolayer of HEC-1A, the concentration was reduced from 10 □M to 5 □M of G2SF16 to avoid any damage to the monolayer perfectly formed in the membranes of transwall. This graph shows the effect of dendrimer on the passage of HIV particles through the monolayer. Supernatant was collected from the basolateral area at 24 and 48 hours after infection.
Second generation of sulfonate dendrimers was evaluated for possible prophylactic and therapeutic effect in PBMC, showing a 80% inhibition in trials of pre-treatment of cells with the dendrimer and 60% in post-treatment tests, confirming the two applications of this dendrimer in a more physiological as it is a primary lymphoid line. We used T-20 (Fuzeon) and AZT as controls for inhibition of viral fusion and reverse transcriptase respectively. See FIG. 9. The G2SF16 showed an 80% inhibition in pre-treatment tests of cells with the dendrimer prior to infection by HIV-1 NL4.3 and 60% in the post-treatment tests, confirming the two applications of this dendrimer in a more physiological as it is a primary lymphoid culture. Prevention of the HIV infection is favored as well as the inhibition of viral replication in cells already infected by HIV.

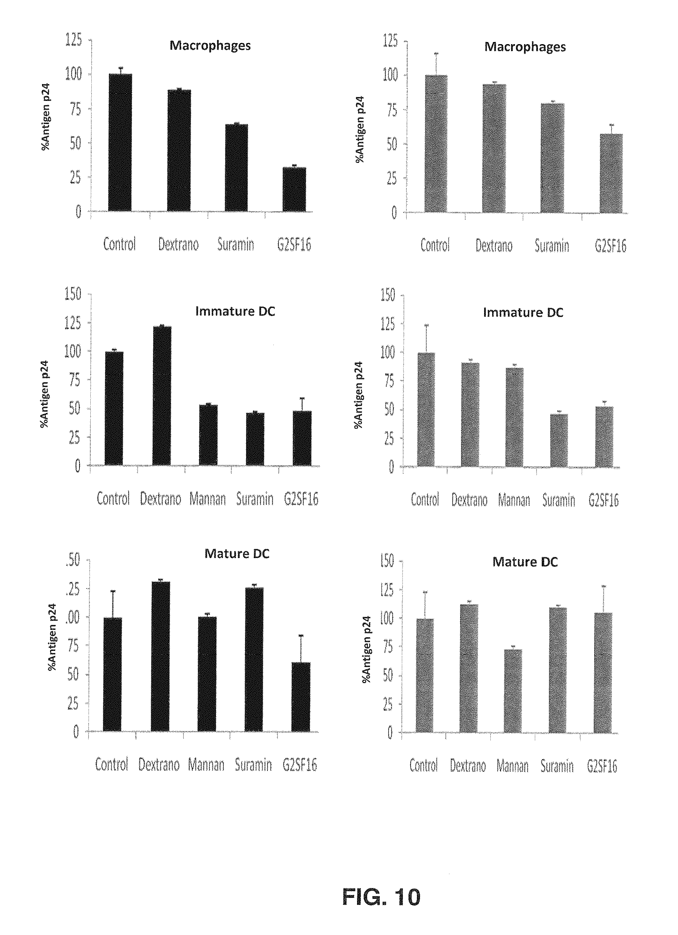

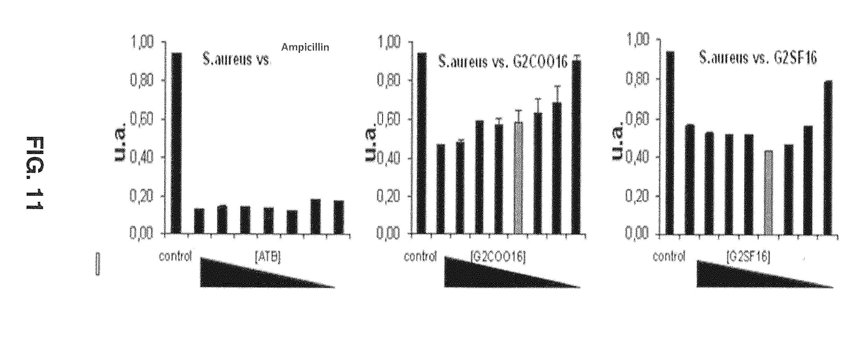

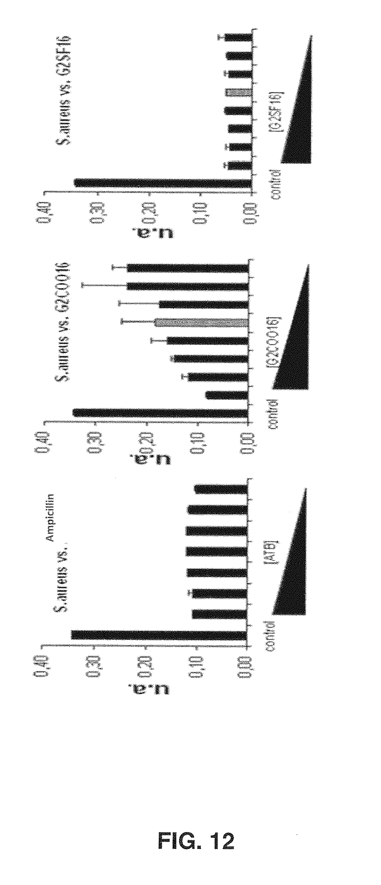

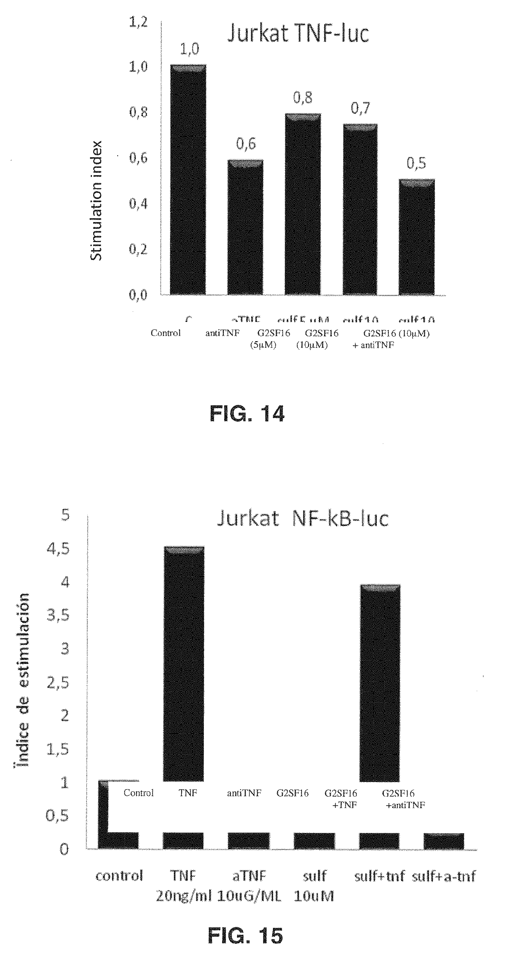

The release of viral particles by dendritic cells and macrophages was assessed. Controls were tested as dextran, suramin and mannan in dendritic cells. Bal and NL4.3 were used for a internalization test of the virus in the presence of nanoparticles inhibitors. There was a clear decrease of p24 in the supernatant of macrophages in the presence of G2SF16. In immature dendritic cells, decreased p24 antigen was significant for suramin, mannan and G2SF16. See FIG. 10, where, as it was mentioned above, we evaluated the ability to release viral particles in infected cells and was infected with 50 ng for good quantification. Controls as dextran, suramin and mannan were tested in dendritic cells.
NL4.3 was used (black columns) and Bal (white columns) for a test of the virus internalization in the presence of nanoparticles inhibitors. There was a clear decrease of p24 in the supernatant of macrophages in the presence of G2SF16. In immature dendritic cells, decreased p24 antigen was significant for suramin, mannan and G2SF16. We also determined the antibacterial effect with these dendrimers in the presence of bacteria. *Staphylococcus aureus* was chosen as an experimental model. In these experiments, we observed that the used concentration of 10 □m, dendrimers have approximately 50% of a bactericidal (Fig. 11) and 85% of bacteriostatic effect (Fig. 12). We have also observed that the sulfonate-terminated dendrimers are more potent bactericidal agents than the carboxylate-terminated dendrimers analogues.
On the other hand, it is important to note that preliminary experiments carried out, show that the dendrimers described in this invention exhibit an antiinflammatory effect. The data obtained in the experiments of Jurkat cells transfected with TNF-luc plasmids (Fig. 14) and PNF-kB-luc (Fig. 15) in the presence of anionic dendrimer G2SF16 clearly indicate that the anionic dendrimer decreases the transcriptional activity of NF-kappa and therefore the inflammation process.

### Biocompatibility and inhibitor effect of the entrance of anionic dendrimers obtained by click coupling.

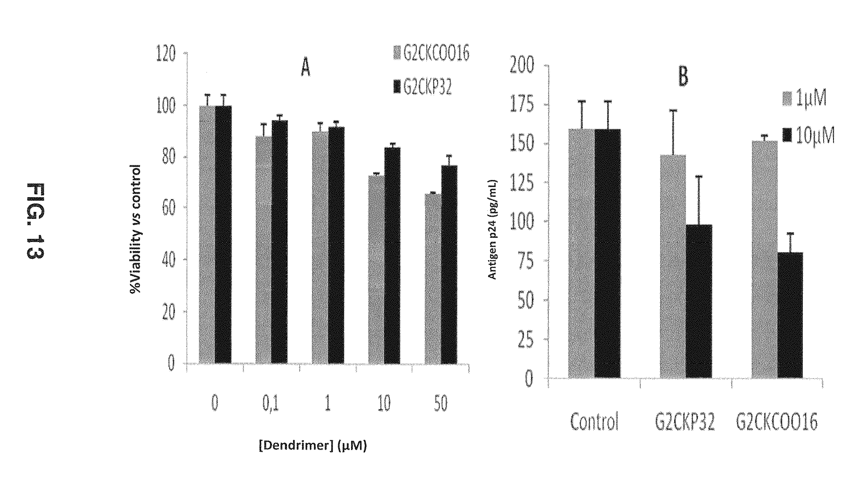

The new click dendrimers with carboxylate and phosphonate ending groups (G2CKCOO16 and G2CKP32) were also evaluated for biocompatibility in the cell population most exposed to its action, endometrial epithelial cells. In FIG. 13A, one can appreciate good cell viability up to 10 □M, whereas in FIG. 13B shows the inhibition of entry into the same cell line pretreating these with dendrimers one hour before infection with VIHNL4-3. This inhibition is concentration dependent of dendrimer and up to 50% was obtained in the case of 10 □M concentration.
These results show that anionic carbosilane dendrimers obtained by synthetic routes based on the so-called "click-chemistry", also behave as potent microbicide, similar to the anionic sulfonate and carboxylate dendrimers described above.
Dendrimers previously described in the present invention have been shown to inhibit the infection caused by viruses, interfering with both virus entry into cells and in subsequent steps of viral replication. This is the case for example of the Herpes Simplex, whose infection is inhibited in vitro by the effect of modified polylysine dendrimers. It has also been able to inhibit HIV replication, both at the cellular entry and in subsequent steps, in this case through the use of covalently modified PAMAM dendrimers, which were capable of interfering with reverse transcriptase and integrase of the virus. Using these properties, vaginal gels have been developed for the prevention of sexually transmitted diseases with formulations based on dendrimers, as is the case VivaGel™ (Starpharma), whose active ingredient is a polylysine dendrimer functionalized with naftalendisulfonate units that appears to be effective in prevention of HIV infection through their ability to bind to the glycoprotein gp120 of the virus surface. Although the design of antiviral dendrimers have a preference for those who come in groups that mimic the surface which are present on the cell surface and therefore are able to compete with cells for binding to the virus, also it has been designed a dendrimer with amide groups at the surface functioning as inhibitor of respiratory syncytial virus. It is believed that due to the formation of hydrogen bonds between the peripheral groups of the dendrimer with the fusion protein of the virus, it is expected that dendrimers functionalized with groups capable of forming hydrogen bonds with viral proteins involved in the interaction with the cell surface are also able to interact with different viruses, inhibiting the infection provoked by them.

In other cases, the dendrimers have been used as antibacterial, or to disrupt the cell walls of some fungi. When designed for this purpose, it has a preference for cationic dendrimers with surface groups such as amine or tetraalkylammonium groups, which facilitate the adhesion of the dendrimers to the bacterial membrane, causing lysis of the bacteria. Such is the case of Poly (Propylene Imine) (PPI) dendrimers with tertiary alkyl groups on their surface, which have demonstrated a broad antibacterial activity against both Gram positive and Gram negative bacteria [Chen, CZ and SL Cooper, Biomaterials, 2002, 23, 3359-68, CZ Chen et al, biomacromolecules, 2000, 1, 473-80]. These dendrimers have a greater antibacterial activity than other hyperbranched polymers. In the case of dendrimers functionalized with anionic groups, the bacteriostatic and fungistatic effect that such as dendrimers can be produced at the cellular level is being studied. The ability to add various pathogens and to prevent their growth, may favor the elimination of microorganisms by the polymorphonuclear cells of the immune system.
The dendrimers of the present invention, functionalized with anionic residues containing carboxylate and sulfonate groups, are one option to be used as microstatics.

The dendrimers of the present invention represent interesting alternatives for these areas of biomedicine and describe here the chemical nature of dendrimers, the studies on the biocompatibility (performed on cell lines, in peripheral blood mononuclear cells (PBMC) and erythrocytes), mitogenicity studies, studies of impairment of adhesion and internalization of HIV particles to the cell surface and transcytosis inhibition studies through vaginal epithelial monolayers. In addition to the prophylactic application, also the therapeutic effect is evaluated, since in infected patients, dendrimers may prevent the progression of infection to healthy cells.

### Results with phenolic core dendrimers

### Evaluation of cytotoxicity of dendrimers

To perform a complete characterization of the three generations of the new dendrimer synthesized, a screening system was developed to determine the biocompatible concentrations required for a more detailed study.

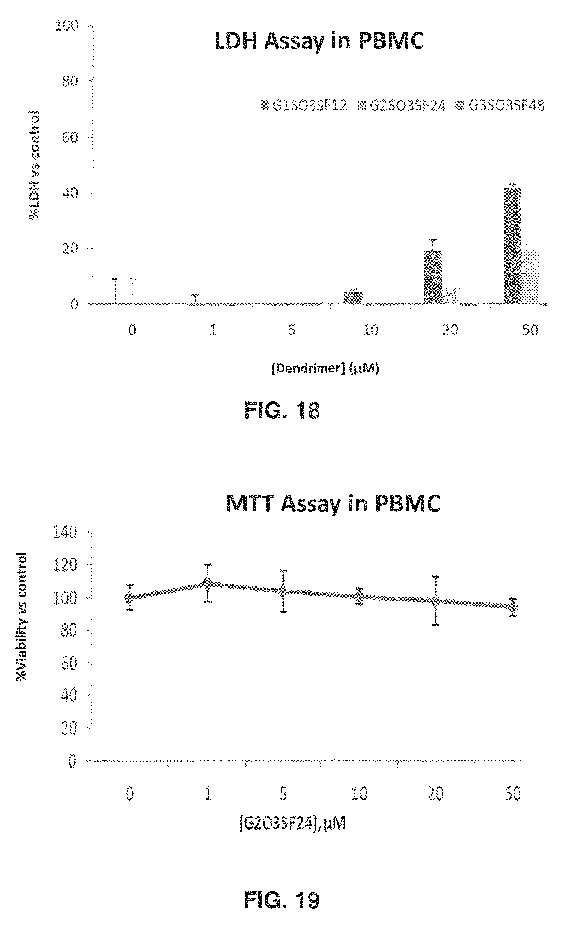

First of all, the limits of solubility of the dendrimers of each generation in water were fixed. Dendrimers were dissolved at concentrations of 3 mM, 2 mM and 1 mM, the latter presented the best solubility without the aid of additional physical factors for a perfect solubilization (vortex, heat, etc.). Once seated the starting concentration for all dendrimers tested, we proceeded to evaluate their cytotoxicity on target cells in our study, vaginal epithelial cells (HEC-1A) and the PBMC. A study of dendrimers at different concentrations was made, evaluating the biocompatibility of the 3 generations by trypan blue staining. To evaluate the damage to the cell membrane that can cause the peripheral anionic charges (forming pores due to interaction with various cell receptors or electrostatic interaction with positive areas of the membrane), test release of lactate dehydrogenase to the supernatant were made. It was found that for values below 10 □M, the dendrimers were not toxic in a lymphoid cell line (Fig. 17) or in PBMC (Fig. 18).

These studies were complemented by testing cellular activity of the mitochondria, where the reduction of tetrazolium salts to formazan crystals is an indirect reflection of the correct cell metabolism.

The three generations of dendrimers with phenolic core were evaluated, obtaining acceptable levels of biocompatibility and always within of acceptability indicated for both trials (no more than 10% of LDH in supernatants and no more than a 20% decrease in cell activity). The results show that even at a concentration of 50 µM, there is no significant membrane damage in a cell line such as the MT-2.

A detailed study to assess the effect of a dendrimer in the metabolism of the PBMC was performed. We tested the second generation dendrimer and observed that no mitochondrial activity was reduced noticeably. FIG. 19).

Given these results, it was determined that the G203SF24 would be chosen to tune the inhibition assays of adherence, internalization and infection of HIV to epithelial cells and PBMC.

### Assays of adhesion / internalization

The dendrimer that was exposed on the monolayer of epithelial cells and showed not to be toxic or mitogenic at a concentration of 10 □M. For its possible use as a microbicide, it was decided to evaluate the ability to interact with the virus in the process of its adhesion to the surface of the cell membrane. The dendrimer molecule would act as a physical barrier in preventing HIV infection of the endometrial cells and their passage into the cavity of the uterus.

To evaluate the effectiveness of impairment of cell surface adhesion and the ability to slow down the internalization of the virus in HEC-1A, we designed an experiment with G2O3SF24. The idea of a topical application of this molecule is necessary to evaluate whether the treatment times prior to infection (pretreatment) are limiting to check the efficacy. Trials were conducted at different times and established a 1h pretreatment in all experiments. Suramin, a polysulfonate of naftilurea, is a molecule with identical terminal sulfonate groups that G203SF24 but different internal structure. This molecule was chosen as positive control for inhibition of adhesion as it has been demonstrated its electrostatic binding to the gp120 V3 region of HIV, which prevents the right performance of the infection machinery in vitro.

### Inhibition assays

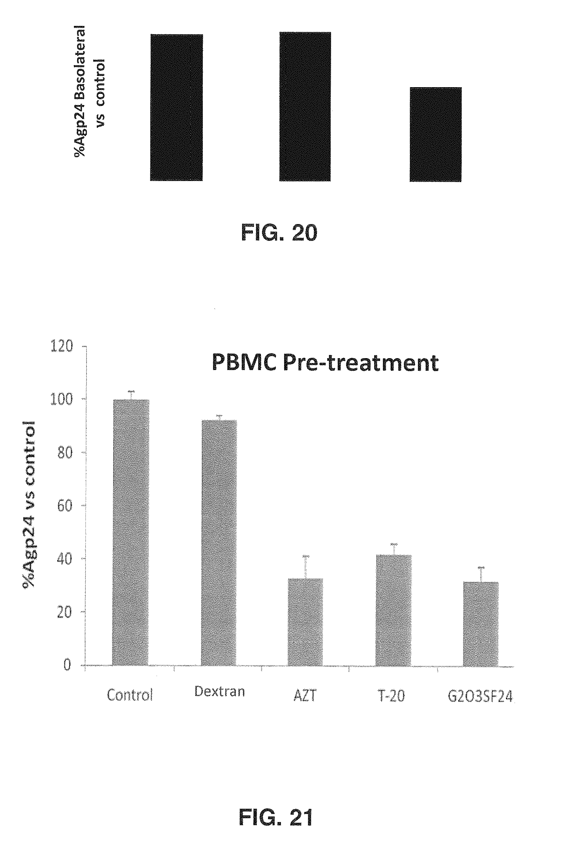

To play the best possible manner the role that the anionic dendrimer develop on the surface of the endometrium and to study the passage of virions through the mucosal stratified vaginal epithelium, we used transwell devices that recreate the phenomenon of transcytosis (transport of macromolecules from the extracellular space to another through the cytoplasm of a cell through an endocytic vesicle) due to the possibility of forming a perfect monolayer of adherent cells on the inside and collect information from the supernatants of the apical and basolateral side of the monolayer. In the graph of FIG. 20, shows the effect of dendrimer on the passage of viral particles through the monolayer. This effect was reduced by 40%, whereas our positive control lacks the inhibitory effect. Therefore, the internal structure of the dendrimer is involved in the antiviral capacity of the molecule.

The second generation of sulfonates was evaluated for its possible prophylactic and therapeutic effect in PBMCs, showing a 60% inhibition in the pre-treatment test of cells with the dendrimer, which confirms the application of this dendrimer in a more physiological system as a primary lymphoid line, FIG. 21.

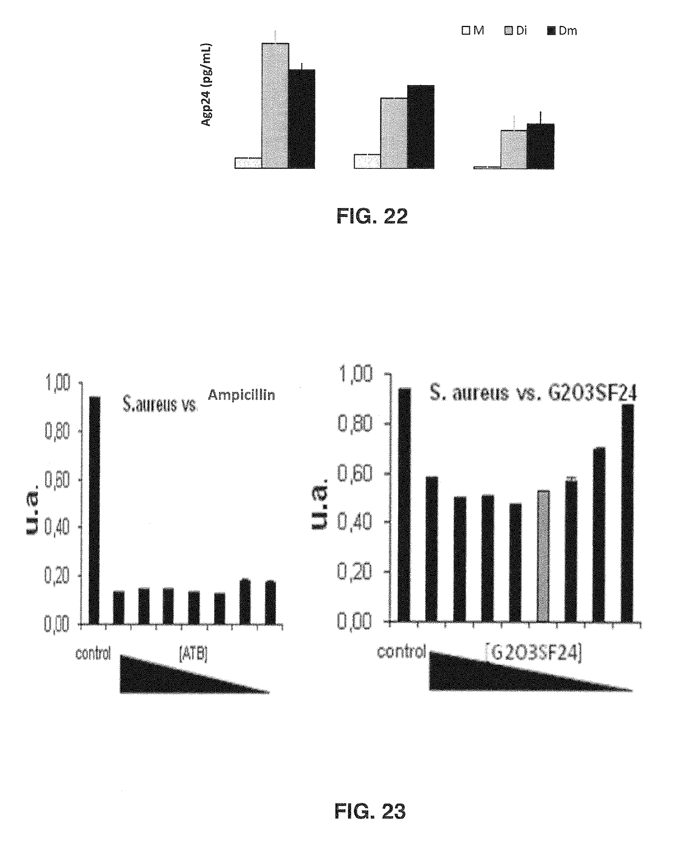

It is currently evaluating its action on dendritic cells and macrophages, because these cell lines were less infected than in the experiments with lymphocytes, the decrease by 50 to 70% in different cell populations is linked to the availability of virus in contact with them. FIG. 22.

### Application Assays antibacterial and antifungal

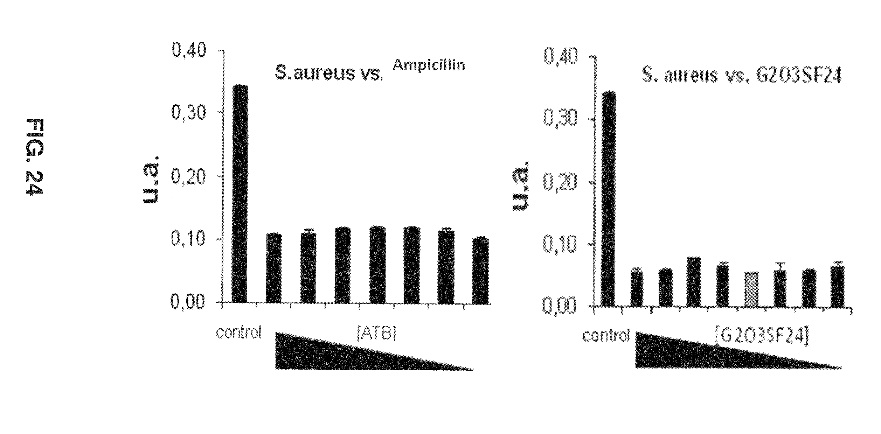

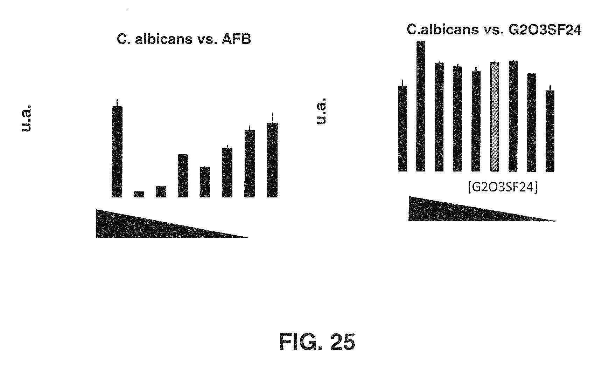

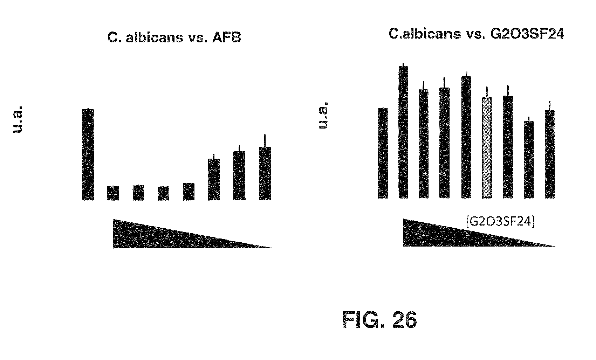

The antibacterial and antifungal effect of these dendrimers was evaluated in the presence of bacteria. Staphylococcus aureus and candida albicans were choosen as models of bacterial and fungal testing, respectively. In these experiments it was observed that the used concentration of dendrimer, 10 □M, has a 40% bactericidal and 90% bacteriostatic effect FIG. 23-24. There were no fungicides or fungistatic effects at the concentrations used, FIG. 25-26.

## Claims

1. Carbosilane dendrimer comprising:
- a polyfunctional core and
- an outer layer, which consists, wholly or partly in the same or different units of the group (I) with formula: 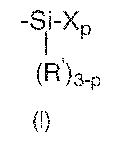
wherein: R' is an alkyl group (C₁-C₄),
p varies between 1 and 3, and
X is the next group of formula (II): 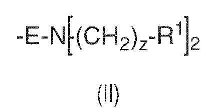
wherein: E is a bonding group between silicon and the amine group,
z varies between 1 and 4,
R¹ is selected from a group comprising the list -COOR², -SO₃R² or -PO₃(R²)₂, and
R² is hydrogen or an alkyl group (C₁-C₄).

2. Dendrimer according to claim 1, wherein the core is silicon or polyphenols.

3. Dendrimer according to claim 2, wherein the polyphenolic nucleus is 1,3,5-trihydroxybenzene.

4. Dendrimer according to any of claims 1 to 3, wherein p is 1.

5. Dendrimer according to any of claims 1 to 4, wherein R' is methyl.

6. Dendrimer according to any of claims 1 to 5, wherein E is selected from an alkyl (C₁-C₁₀) or a group -(CH₂)ₓ-R³, wherein R³ is a triazole or phenoxide group and x varies between 1 and 6.

7. Dendrimer according to claim 6, wherein the alkyl group of E is a propyl.

8. Dendrimer according to any of claims 1 to 7, wherein R² is hydrogen or methyl.

9. Dendrimer according to any of claims 1 to 8, wherein E is the group -(CH₂)ₓ-R³ and R³ is triazole, x is defined in claim 6.

10. Dendrimer according to claim 9, wherein x is 4.

11. Dendrimer according to any of claims 1 to 10, wherein E is the group -(CH₂)ₓ-R³ and R³ is phenoxide, x is defined in claim 6.

12. Dendrimer according to claim 11, wherein x is 1.

13. Dendrimer according to any of claims 1 to 12, wherein R¹ is the group -COOR².

14. Dendrimer according to any of claims 1 to 12, wherein R¹ is the group -SO₃R².

15. Dendrimer according to any of claims 1 to 12, wherein R¹ is the group -PO₃(R²)₂.

16. Dendrimer according to any of claims 1 to 14, wherein z is 2.

17. Dendrimer according to claim 15, wherein z is 1.

18. Dendrimer according to any of claims 1 to 17, wherein said dendrimer is an anionic salt.

19. Dendrimer according to claim 18, wherein the salt is Na⁺.

20. Dendrimer according to any of claims 1 to 19, wherein said dendrimer is of formula (III): 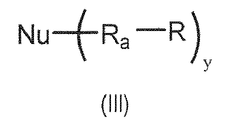 wherein: Nu represents a polyfunctional core, Rₐ is an alkyl group (C₁-C₆); and has a value between 2 and 6, and R is the terminal group of general formula (I) as described in any of Claims 1 to 19.

21. Dendrimer according to any of claims 1 to 19, wherein said dendrimer is at least of first generation with the following formula (IV): 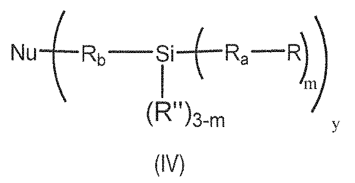 wherein: Nu represents a polyfunctional core, Rₐ R_{b} and R", are the same or different and represent an alkyl group (C₁-C₆); m has a value between 1 and 3; y has a value of between 2 and 6, and R is the terminal group of general formula (I) as described in any of claims 1 to 19.

22. Dendrimer according to any of claims 1 to 19, wherein said dendrimer is at least of second generation with the following formula (V): 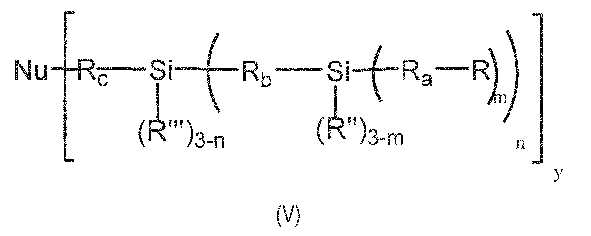 wherein: Nu represents a polyfunctional core, Rₐ, R_{b}, R_{c}, R^{II} and R^{III}, are the same or different and represent an alkyl group (C₁-C₆), m and n are the same or different and have a value between 1 and 3; y has a value between 2 and 6, and R is the terminal group of general formula (I) as described in any of claims 1 to 19.

23. Dendrimer according to any of claims 1 to 19, wherein said dendrimer is at least of third generation with the following formula (VI): 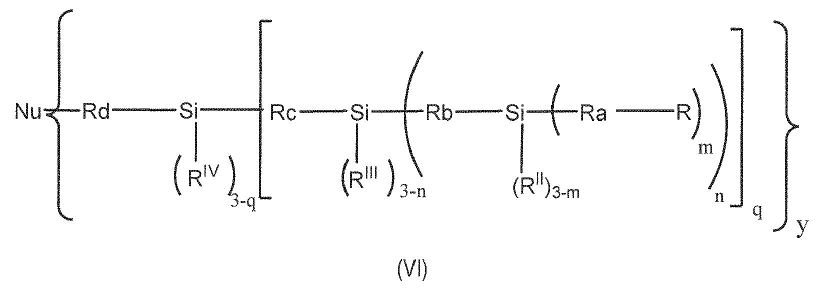 wherein: Nu represents a polyfunctional core, Rₐ, R_{b}, R_{c}, R_{d}, R^{II}, R^{III} and R^{IV} are the same or different and represent an alkyl group (C₁-C₆), m, n and q are the same or different and has a value between 1 and 3, y has a value between 2 and 6, and R is the terminal group of general formula (I) as described in any of claims 1 to 19.

24. Dendrimer according to any one of claims 20 to 23, wherein Rₐ, R_{b}, R_{c} and R_{d} are the same or different and represent an alkyl group (C₂-C₄).

25. Dendrimer according to any one of claims 20 to 24, wherein R", R"' or R^{IV}, are a methyl group.

26. Compound of formula:
Et₃-R
wherein: Et is an ethyl group and
R is the terminal group of formula (I) described in any of claims 1 to 19, a group -Si(R')₃₋ₚ[(CH₂)₄-N₃]ₚ, or the group -Si(R')₃₋ₚ[E-NH₂]ₚ wherein R' is an alkyl group (C₁-C₄), p has a value between 1 and 3 and E is a bonding group described in the previous claims.

27. carbosilane dendrimer comprising:
A polyfunctional core and
an outer layer, which consists, wholly or partly in the same or different units of the group -Si(R')₃₋ₚ[(CH₂)₄-N₃]ₚ, or the group -Si(R')₃₋ₚ[E-NH₂]ₚ; wherein R' is an alkyl group (C₁-C₄), p varies between 1 and 3 and E is a bonding group described in the previous claims.

28. Compound according to claim 24 or dendrimer according to claim 25, wherein p is 1.

29. Compound according to claim 24 or 26 or dendrimer according to claim 25 or 26, wherein R' is a methyl group.

30. Compound according to claim 24 or 26 to 27 or dendrimer according to any one of claims 25 to 27, wherein E is the group -(CH₂)ₓ-R³ and R³ is triazole, x is defined in claim 6, preferably x is 4.

31. Process for obtaining dendrimers according to any of claims 1 to 25, comprising:
Michael type addition of a dendrimer containing a polivalent core and terminal -NH₂ groups to an alkene containing sulfonates or carboxylic groups or the two-step reaction of a dendrimer containing a polyvalent core and terminal -NH₂ groups with formaldehyde and dimethyl phosphonate.

32. Process for obtaining dendrimers according to any of claims 1 to 25, comprising:
the hydrosilylation of a dendrimer containing terminal -Si-H groups with an allylamine derivative functionalized with carboxylic, phosphonic or sulphonic groups.

33. Use of the dendrimers according to any of claims 1 to 25, to the preparation of a medicament.

34. Use of the dendrimers according to any of claims 1 to 25, for the preparation of a medicament in the prevention and/or treatment of diseases caused by viruses, bacteria or fungi.

35. Use according to claim 34, wherein the disease is caused by HIV.

36. Pharmaceutical composition comprising at least one dendrimer according to any of claims 1 to 25 and a pharmaceutically acceptable vehicle.

37. Composition according to claim 34, further comprising another agent (active principle).

38. Use of the dendrimers according to any of claims 1 to 25 as transport vehicles for molecules.
